# EUROPEAN PATENT APPLICATION

(11) **EP 4 257 604 A1**
(43) Date of publication of application: **11.10.2023**
(21) Application number: 21899892.0
(22) Date of filing: 22.11.2021
(51) Int. Cl.: C07K 16/24, C12N 15/13, A61K 39/395

(54) **ANTI-TSLP ANTIBODY PHARMACEUTICAL COMPOSITION AND USE THEREOF**

(30) Priority: 03.12.2020 CN 202011405207
(71) Applicant: Jiangsu Hengrui Pharmaceuticals Co., Ltd., Lianyungang, Jiangsu 222047 (CN); Shanghai Hengrui Pharmaceutical Co., Ltd, Shanghai 200245 (CN)
(72) Inventor: WANG, Zhiwan, Shanghai 200245 (CN); WU, Tingting, Shanghai 200245 (CN); LIU, Xun, Shanghai 200245 (CN)
(74) Representative: dompatent von Kreisler Selting Werner - Partnerschaft von Patent- und Rechtsanwälten mbB
(86) International application number: PCT/CN2021/132037
(87) International publication number: WO 2022/116858

(57) **Abstract**

Provided are a pharmaceutical composition comprising an anti-TSLP antibody and the use thereof. The pharmaceutical composition comprises an anti-TSLP antibody and a buffer.

## Description

The present application claims priority to Chinese Patent Application No. 202011405207.9 filed on December 3, 2020, which is incorporated herein by reference in its entirety.

### TECHNICAL FIELD

The present disclosure belongs to the field of pharmaceutical formulations, and in particular relates to a pharmaceutical composition comprising an anti-TSLP antibody, and use thereof as a medicament for treating a disease or disorder.

### BACKGROUND

The statements herein merely provide background information related to the present disclosure and may not necessarily constitute the prior art.

Asthma is a severe chronic inflammatory disease of the airways, and there are about 3.34 hundred million asthmatics all over the world. As the environment deteriorates and the air pollution increases, more people are likely to suffer from this disease, which seriously endangers the life and health of human beings.

Thymic stromal lymphopoietin (TSLP) is a cytokine produced by epithelial cells in response to pro-inflammatory stimuli and promotes allergic inflammatory responses primarily through its activity on dendritic cells and mast cells. TSLP is an interleukin-7 (IL-7)-like cytokine, originally found in thymus stromal cell conditioned medium of mice. TSLP is expressed primarily in lung, skin and intestinal epithelial cells. TSLP consists of 4 α-helices and two loops AB and CD. It contains three pairs of disulfide bonds consisting of six cysteine residues in the molecule and two N-glycosylation sites, and has a molecular weight of about 15-20 kD. The receptor for TSLP is a complex comprising two portions, one for TSLPR and the other for IL-7Rα. TSLP binds to TSLPR with relatively low affinity, then recruits IL-7Rα binding with high affinity, and finally leads to DC maturation and T cell differentiation through the activation of signaling pathways such as stat5.

Myeloid dendritic cells (mDCs) are the most prominent effector cells of TSLP, and TSLP acts on immature mDCs. mDCs secrete cytokines IL-8, eotaxin-2, TARC, and MDC, and highly express OX40L. In the absence of IL-12, OX40L binds to native CD4⁺ T cells, causing them to be differentiated into Th2 cells, which in turn secrete Th2 cytokines such as IL-5, IL-4, IL-9, IL-13, and TNF to induce the Th2 inflammatory response in the body. In addition, TSLP can also induce DC cells to produce the cytokine IL-8, which in turn recruits neutrophils, resulting in neutrophil-induced innate immune inflammation. TSLP can also induce DCs to produce eotaxin-2, which recruits eosinophils to act with IL-5, causing the body to rapidly enter the eosinophil-infiltrated inflammatory state. TSLP also acts on mast cells and natural killer cells, and mediates natural inflammation by inducing production of IL-4, IL-6, IgE, and the like. In conclusion, TSLP can cause both innate inflammation and Th2 inflammation, thereby causing increased tissue mucus, airway remodeling leading to tracheal stenosis, and severe cellular fibrosis, which gradually evolve into three allergic diseases such as asthma, allergic dermatitis and allergic rhinitis. Therefore, blocking TSLP is a potentially effective strategy for treating diseases such as asthma and allergic dermatitis.

Currently, there is no TSLP monoclonal antibody available on the market worldwide. To keep an antibody effective, the antibody must maintain its biological activity during production, purification, transportation, and storage. However, antibodies are susceptible to denaturation, aggregation, contamination, and other changes during formulation and storage, which are great obstacles to the preparation of antibody formulations. Due to the diversity of antibodies, there is no universal formula or condition suitable for storage of all antibodies, and the optimal formulation of one antibody is generally specific to that antibody. The formulation of antibodies is generally an important part of the commercial antibody research and development process, and therefore, there is a need to develop stable antibody formulations.

### SUMMARY

The present disclosure provides a pharmaceutical composition comprising an anti-TSLP antibody and use thereof.

In some embodiments, provided is the pharmaceutical composition described above, which comprises an anti-TSLP antibody and a buffer, wherein the buffer is a histidine salt buffer or a succinate buffer.

In some embodiments, provided is the pharmaceutical composition described above, which comprises an anti-TSLP antibody and a buffer, wherein the buffer is a histidine salt buffer.

In some embodiments, provided is the pharmaceutical composition, wherein the buffer is a histidine-acetate buffer or a histidine-hydrochloride buffer.

In some embodiments, provided is the pharmaceutical composition described above, wherein the buffer is a succinic acid-sodium succinate buffer.

In some embodiments, provided is the pharmaceutical composition described above, wherein the anti-TSLP antibody comprises a heavy chain variable region and a light chain variable region, wherein:
i) the heavy chain variable region comprises a HCDR1, a HCDR2, and a HCDR3 set forth in SEQ ID NO: 26, SEQ ID NO: 94, and SEQ ID NO: 28, respectively; and
   the light chain variable region comprises a LCDR1, a LCDR2, and a LCDR3 set forth in SEQ ID NO: 29, SEQ ID NO: 113, and SEQ ID NO: 31, respectively;
ii) the heavy chain variable region comprises a HCDR1, a HCDR2, and a HCDR3 set forth in SEQ ID NO: 20, SEQ ID NO: 21, and SEQ ID NO: 22, respectively; and the light chain variable region comprises a LCDR1, a LCDR2, and a LCDR3 set forth in SEQ ID NO: 70, SEQ ID NO: 24, and SEQ ID NO: 25, respectively;
iii) the heavy chain variable region comprises a HCDR1, a HCDR2, and a HCDR3 set forth in SEQ ID NO: 14, SEQ ID NO: 15, and SEQ ID NO: 45, respectively; and the light chain variable region comprises a LCDR1, a LCDR2, and a LCDR3 set forth in SEQ ID NO: 17, SEQ ID NO: 18, and SEQ ID NO: 54, respectively; or
iv) the heavy chain variable region comprises a HCDR1, a HCDR2, and a HCDR3 set forth in SEQ ID NO: 32, SEQ ID NO: 33, and SEQ ID NO: 34, respectively; and the light chain variable region comprises a LCDR1, a LCDR2, and a LCDR3 set forth in SEQ ID NO: 35, SEQ ID NO: 36, and SEQ ID NO: 37, respectively.

In some embodiments, provided is the pharmaceutical composition described above, wherein the anti-TSLP antibody comprises a heavy chain variable region and a light chain variable region as shown in any one of the following:
a) a heavy chain variable region sequence set forth in SEQ ID NO: 97 and a light chain variable region set forth in SEQ ID NO: 119;
b) a heavy chain variable region sequence set forth in SEQ ID NO: 69 and a light chain variable region set forth in SEQ ID NO: 74;
c) a heavy chain variable region sequence set forth in SEQ ID NO: 50 and a light chain variable region set forth in SEQ ID NO: 52; or
d) a heavy chain variable region sequence set forth in SEQ ID NO: 132 and a light chain variable region set forth in SEQ ID NO: 125.

In some embodiments, provided is the pharmaceutical composition described above, wherein the anti-TSLP antibody comprises:
a) a heavy chain set forth in SEQ ID NO: 139 and a light chain set forth in SEQ ID NO: 140;
b) a heavy chain set forth in SEQ ID NO: 137 and a light chain set forth in SEQ ID NO: 138;
c) a heavy chain set forth in SEQ ID NO: 135 and a light chain set forth in SEQ ID NO: 136; or
d) a heavy chain set forth in SEQ ID NO: 141 and a light chain set forth in SEQ ID NO: 142.

In some embodiments, provided is the pharmaceutical composition described above, wherein the buffer is at a concentration of 5-50 mM, including but not limited to 5-10 mM, 10-15 mM, 15-25 mM, 25-35 mM, or 35-45 mM.

In some embodiments, provided is the pharmaceutical composition described above, wherein the buffer is at a concentration of 10-30 mM.

In some embodiments, provided is the pharmaceutical composition described above, wherein the buffer is at a concentration of 5-50 mM, including but not limited to 5 mM, 15 mM, 20 mM, 25 mM, 30 mM, 35 mM, 40 mM, 45 mM, 50 mM, and any range therebetween.

In some embodiments, provided is the pharmaceutical composition described above, wherein the buffer is at a concentration of 10-20 mM.

In some embodiments, provided is the pharmaceutical composition described above, wherein the buffer is at a concentration of about 20 mM.

In some embodiments, provided is the pharmaceutical composition described above, wherein the buffer has a pH of 5.0-6.5, including but not limited to 5.0-5.5, 5.5-6.0, or 6.0-6.5.

In some embodiments, provided is the pharmaceutical composition described above, wherein the buffer has a pH of 5.0-6.5, including but not limited to 5.0, 5.1, 5.2, 5.3, 5.4, 5.5, 5.6, 5.7, 5.8, 5.9, 6.0, 6.1, 6.2, 6.3, 6.4, 6.5, and any range therebetween.

In some embodiments, provided is the pharmaceutical composition described above, wherein the buffer has a pH of 5.5-6.5.

In some embodiments, provided is the pharmaceutical composition described above, wherein the buffer has a pH of about 6.0.

In some embodiments, provided is the pharmaceutical composition described above, wherein the buffer has a pH of about 6.2.

In some embodiments, there may be a shift in the pH of the pharmaceutical composition described above, with the degree of shift being about ± 0.3.

In some embodiments, provided is the pharmaceutical composition described above, wherein the pharmaceutical combination has a pH of 5.0-6.5.

In some embodiments, provided is the pharmaceutical composition described above, wherein the pharmaceutical combination has a pH of 5.5-6.5.

In some embodiments, provided is the pharmaceutical composition described above, wherein the pharmaceutical combination has a pH of about 5.8-6.3.

In some embodiments, provided is the pharmaceutical composition described above, wherein the pharmaceutical combination has a pH of about 6.0-6.3.

In some embodiments, provided is the pharmaceutical composition described above, wherein the pharmaceutical combination has a pH of about 6.3.

In some embodiments, provided is the pharmaceutical composition described above, wherein the anti-TSLP antibody is at a concentration of 1-150 mg/mL, including but not limited to 80-150 mg/mL, 80-120 mg/mL, 80-100 mg/mL, 100-150 mg/mL, or 100-120 mg/mL.

In some embodiments, provided is the pharmaceutical composition described above, wherein the anti-TSLP antibody is at a concentration of 1-150 mg/mL, including but not limited to 1 mg/mL, 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, and any range therebetween.

In some embodiments, provided is the pharmaceutical composition described above, wherein the anti-TSLP antibody is at a concentration of 100-150 mg/mL.

In some embodiments, provided is the pharmaceutical composition described above, wherein the anti-TSLP antibody is at a concentration of about 100 mg/mL.

In some embodiments, provided is the pharmaceutical composition described above, wherein the pharmaceutical composition further comprises a surfactant.

In some embodiments, provided is the pharmaceutical composition described above, wherein the surfactant is a polysorbate.

In some embodiments, provided is the pharmaceutical composition described above, wherein the surfactant is polysorbate 80 or polysorbate 20.

In some embodiments, provided is the pharmaceutical composition described above, wherein the surfactant is polysorbate 80.

In some embodiments, provided is the pharmaceutical composition described above, wherein the surfactant is at a concentration of 0.01-1.0 mg/mL, including but not limited to 0.05-1.0 mg/mL, 0.05-0.8 mg/mL, 0.1-1.0 mg/mL, 0.1-0.8 mg/mL, 0.1-0.6 mg/mL, 0.1-0.4 mg/mL, 0.2-1.0 mg/mL, 0.2-0.8 mg/mL, 0.2-0.6 mg/mL, or 0.2-0.4 mg/mL.

In some embodiments, provided is the pharmaceutical composition described above, wherein the surfactant is at a concentration of 0.1-0.8 mg/mL.

In some embodiments, provided is the pharmaceutical composition described above, wherein the surfactant is at a concentration of 0.01-1.0 mg/mL, including but not limited to 0.01 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.55 mg/mL, 0.6 mg/mL, 0.65 mg/mL, 0.7 mg/mL, 0.75 mg/mL, 0.8 mg/mL, 0.85 mg/mL, 0.9 mg/mL, 0.95 mg/mL, 1.0 mg/mL, and any range therebetween.

In some embodiments, provided is the pharmaceutical composition described above, wherein the surfactant is at a concentration of about 0.8 mg/mL.

In some embodiments, provided is the pharmaceutical composition described above, wherein the composition further comprises a stabilizer, wherein the stabilizer is selected from the group consisting of one or more of a sugar, an amino acid, and EDTA.

In some embodiments, provided is the pharmaceutical composition described above, wherein the sugar is selected from the group consisting of one or more of trehalose, sucrose, sorbitol, and mannitol.

In some embodiments, provided is the pharmaceutical composition described above, wherein the amino acid is selected from the group consisting of one or more of histidine, tryptophan, and methionine.

In some embodiments, provided is the pharmaceutical composition described above, wherein the stabilizer is sucrose.

In some embodiments, provided is the pharmaceutical composition described above, wherein the stabilizer is a sugar at a concentration of 20-100 mg/mL, including but not limited to 30-90 mg/mL, 30-70 mg/mL, 50-90 mg/mL, or 50 mg/mL-70 mg/mL.

In some embodiments, provided is the pharmaceutical composition described above, wherein the stabilizer is a sugar at a concentration of 30-100 mg/mL, including but not limited to 40-90 mg/mL, 40-80 mg/mL, 50-80 mg/mL, 60-90 mg/mL, or 60-80 mg/mL.

In some embodiments, provided is the pharmaceutical composition described above, wherein the stabilizer is a sugar at a concentration of 20-100 mg/mL, including but not limited to 20 mg/mL, 30 mg/mL, 40 mg/mL, 50 mg/mL, 60 mg/mL, 70 mg/mL, 80 mg/mL, 90 mg/mL, 100 mg/mL, and any range therebetween.

In some embodiments, provided is the pharmaceutical composition described above, wherein the stabilizer is a sugar at a concentration of 50-70 mg/mL.

In some embodiments, provided is the pharmaceutical composition described above, wherein the stabilizer is a sugar at a concentration of 50-60 mg/mL.

In some embodiments, provided is the pharmaceutical composition described above, wherein the stabilizer is a sugar at a concentration of about 70 mg/mL.

In some embodiments, provided is the pharmaceutical composition described above, wherein the stabilizer is a sugar at a concentration of 30-70 mg/mL, wherein the sugar is trehalose, sucrose, sorbitol, or mannitol.

In some embodiments, provided is the pharmaceutical composition described above, wherein the stabilizer is a sugar, wherein the sugar is trehalose at a concentration of about 60-70 mg/mL.

In some embodiments, provided is the pharmaceutical composition described above, wherein the stabilizer is sucrose at a concentration of 70 mg/mL.

In some embodiments, provided is the pharmaceutical composition described above, wherein the stabilizer is sorbitol or mannitol at a concentration of 50 mg/mL.

In some embodiments, provided is the pharmaceutical composition described above, wherein the stabilizer further comprises an amino acid at a concentration of 5-50 mM, including but not limited to 10-50 mM, 10-40 mM, 10-30 mM, or 10-20 mM.

In some embodiments, provided is the pharmaceutical composition described above, wherein the stabilizer further comprises an amino acid at a concentration of 5-50 mM, including but not limited to 5-40 mM, 5-30 mM, 5-20 mM, 20-30 mM, 20-40 mM, or 30-40 mM.

In some embodiments, provided is the pharmaceutical composition described above, wherein the stabilizer further comprises an amino acid at a concentration of 10-30 mM.

In some embodiments, provided is the pharmaceutical composition described above, wherein the stabilizer further comprises an amino acid at a concentration of about 30 mM.

In some embodiments, provided is the pharmaceutical composition described above, wherein the stabilizer further comprises an amino acid at a concentration of 5-50 mM, including but not limited to 5 mM, 10 mM, 15 mM, 20 mM, 25 mM, 30 mM, 35 mM, 40 mM, 45 mM, 50 mM, and any range therebetween.

In some embodiments, provided is the pharmaceutical composition described above, wherein the stabilizer further comprises an amino acid at a concentration of 5-50 mM, wherein the amino acid is selected from the group consisting of histidine, tryptophan, and methionine.

In some embodiments, provided is the pharmaceutical composition described above, wherein the stabilizer further comprises an amino acid at a concentration of 10-30 mM, wherein the amino acid is selected from the group consisting of histidine, tryptophan, and methionine.

In some embodiments, provided is the pharmaceutical composition described above, wherein the stabilizer further comprises an amino acid at a concentration of 30 mM, wherein the amino acid is selected from the group consisting of histidine, tryptophan, and methionine.

In some embodiments, provided is the pharmaceutical composition described above, wherein the stabilizer further comprises EDTA at a concentration of 0.1-10 mM, including but not limited to 0.1 mM, 0.2 mM, 0.3 mM, 0.4 mM, 0.5 mM, 1 mM, 2 mM, 3 mM, 4 mM, 5 mM, 6 mM, 7 mM, 8 mM, 9 mM, 10 mM, and any range therebetween.

In some embodiments, provided is the pharmaceutical composition described above, wherein the stabilizer further comprises EDTA at a concentration of 0.1-10 mM, including but not limited to 0.1-8 mM, 0.1-6 mM, 0.1-4 mM, or 0.1-2 mM.

In some embodiments, provided is the pharmaceutical composition described above, wherein the stabilizer further comprises EDTA at a concentration of 0.37-10 mM, including but not limited to 0.37-9 mM, 0.37-7 mM, 0.37-5 mM, or 0.37-3 mM.

In some embodiments, provided is the pharmaceutical composition described above, wherein the stabilizer is 30-70 mg/mL sugar and 10-30 mM amino acid; wherein the sugar is selected from the group consisting of one or more of trehalose, sucrose, sorbitol, and mannitol, and the amino acid is selected from the group consisting of one or more of histidine, tryptophan, and methionine.

In some embodiments, provided is the pharmaceutical composition described above, wherein the stabilizer is 30-70 mg/mL sugar and 0.37-10 mM EDTA; wherein the sugar is selected from the group consisting of one or more of trehalose, sucrose, sorbitol, and mannitol.

In some embodiments, provided is the pharmaceutical composition described above, wherein the stabilizer is 30-70 mg/mL sugar, 10-30 mM amino acid, and 0.37-10 mM EDTA; wherein the sugar is selected from the group consisting of one or more of trehalose, sucrose, sorbitol, and mannitol, and the amino acid is selected from the group consisting of one or more of histidine, tryptophan, and methionine.

In some embodiments, provided is the pharmaceutical composition described above, wherein the stabilizer is:
i) 30 mg/mL sugar and 30 mM amino acid;
ii) 70 mg/mL sugar and 0.37-10 mM EDTA; or
iii) 70 mg/mL sugar, 20 mM amino acid, and 0.37-10 mM EDTA; wherein the sugar is selected from the group consisting of trehalose, sucrose, sorbitol, and mannitol, and the amino acid is selected from the group consisting of histidine, tryptophan, and methionine.

In some embodiments, provided is the pharmaceutical composition described above, wherein the stabilizer is:
i) 30 mg/mL sucrose and 30 mM histidine, tryptophan or methionine;
ii) 70 mg/mL sucrose and 0.37-10 mM EDTA; or
iii) 70 mg/mL sucrose, 20 mM methionine, and 0.37-10 mM EDTA.

In some embodiments, provided is the pharmaceutical composition described above, wherein the stabilizer is:
i) 30 mg/mL sucrose and 30 mM histidine, tryptophan or methionine;
ii) 70 mg/mL sucrose and 10 mM EDTA; or
iii) 70 mg/mL sucrose, 20 mM methionine and 10 mM EDTA.

In some embodiments, provided is the pharmaceutical composition described above, wherein the pharmaceutical composition has a pH of 5.0-6.5. In some embodiments, provided is the pharmaceutical composition described above, wherein the pharmaceutical composition has a pH of 5.5-6.5. In some embodiments, provided is the pharmaceutical composition described above, wherein the pharmaceutical composition has a pH of about 6.0-6.3.

In some embodiments, provided is the pharmaceutical composition described above, which comprises the following components:
(a) 100-150 mg/mL anti-TSLP antibody; (b) 0.1-0.8 mg/mL polysorbate 80; and (c) 10-30 mM histidine salt buffer; wherein the pharmaceutical composition has a pH of 5.5-6.5.

In some embodiments, provided is the pharmaceutical composition described above, which comprises the following components:
(a) 100-150 mg/mL anti-TSLP antibody; (b) 0.1-0.8 mg/mL polysorbate 80; and (c) 10-30 mM histidine-hydrochloride buffer; wherein the pharmaceutical composition has a pH of 5.5-6.5.

In some embodiments, provided is the pharmaceutical composition described above, which comprises the following components:
(a) 100-150 mg/mL anti-TSLP antibody; (b) 0.1-0.8 mg/mL polysorbate 80; and (c) 10-30 mM histidine-hydrochloride buffer; wherein the pharmaceutical composition has a pH of 6.0-6.3.

In some embodiments, provided is the pharmaceutical composition described above, which comprises the following components:
(a) 100-150 mg/mL anti-TSLP antibody; (b) 0.1-0.8 mg/mL polysorbate; (c) 10-30 mM histidine salt buffer; and (d) 50-70 mg/mL sugar; wherein the pharmaceutical composition has a pH of 5.5-6.5.

In some embodiments, provided is the pharmaceutical composition described above, which comprises the following components:
(a) 100-150 mg/mL anti-TSLP antibody; (b) 0.1-0.8 mg/mL polysorbate; (c) 10-30 mM histidine salt buffer; and (d) 50-70 mg/mL sugar; wherein the sugar is selected from the group consisting of trehalose, sucrose, sorbitol, and mannitol, and the polysorbate is selected from the group consisting of polysorbate 20 and polysorbate 80; the pharmaceutical composition has a pH of 5.5-6.5.

In some embodiments, provided is the pharmaceutical composition described above, which comprises the following components:
(a) 100-150 mg/mL anti-TSLP antibody; (b) 0.1-0.8 mg/mL polysorbate; (c) 10-30 mM histidine salt buffer; and (d) 30-70 mg/mL sucrose and 10-30 mM amino acid; wherein the pharmaceutical composition has a pH of 5.5-6.5.

In some embodiments, provided is the pharmaceutical composition described above, which comprises the following components:
(a) 100-150 mg/mL anti-TSLP antibody; (b) 0.1-0.8 mg/mL polysorbate; (c) 10-30 mM histidine salt buffer; and (d) 30-70 mg/mL sucrose and 0.37-10 mM EDTA; wherein the pharmaceutical composition has a pH of 5.5-6.5.

In some embodiments, provided is the pharmaceutical composition described above, which comprises the following components:
(a) 100-150 mg/mL anti-TSLP antibody; (b) 0.1-0.8 mg/mL polysorbate; (c) 10-30 mM histidine salt buffer; and (d) 30-70 mg/mL sucrose, 10-30 mM amino acid, and 0.37-10 mM EDTA; wherein the pharmaceutical composition has a pH of 5.5-6.5.

In some embodiments, provided is the pharmaceutical composition described above, wherein the sugar is selected from the group consisting of trehalose, sucrose, sorbitol, and mannitol; and/or the polysorbate is selected from the group consisting of polysorbate 20 and polysorbate 80; and/or the amino acid is selected from the group consisting of histidine, tryptophan, and methionine; and/or the histidine salt buffer is selected from the group consisting of a histidine-hydrochloride buffer and a histidine-acetate buffer.

In some embodiments, provided is the pharmaceutical composition described above, which comprises the following components:
(a) 100-150 mg/mL anti-TSLP antibody, wherein the anti-TSLP antibody comprises a heavy chain set forth in SEQ ID NO: 139 and a light chain set forth in SEQ ID NO: 140; (b) 0.1-0.8 mg/mL polysorbate; (c) 10-30 mM histidine-acetate buffer; and (d) 30-100 mg/mL trehalose, sucrose, sorbitol, or mannitol; wherein the pharmaceutical composition has a pH of 5.5-6.5.
(a) 100-150 mg/mL anti-TSLP antibody, wherein the anti-TSLP antibody comprises a heavy chain set forth in SEQ ID NO: 139 and a light chain set forth in SEQ ID NO: 140; (b) 0.1-0.8 mg/mL polysorbate; (c) 10-30 mM histidine-acetate buffer; and (d) 50-70 mg/mL trehalose, sucrose, sorbitol, or mannitol; wherein the pharmaceutical composition has a pH of 5.5-6.5.

In some embodiments, provided is the pharmaceutical composition described above, which comprises the following components:
(a) about 100 mg/mL anti-TSLP antibody, wherein the anti-TSLP antibody comprises a heavy chain set forth in SEQ ID NO: 139 and a light chain set forth in SEQ ID NO: 140; (b) about 0.8 mg/mL polysorbate 80; (c) about 20 mM histidine-acetate buffer; and (d) about 70 mg/mL sucrose; wherein the pharmaceutical composition has a pH of about 6.0-6.3.

In some embodiments, provided is the pharmaceutical composition described above, which comprises the following components:
(a) 100-150 mg/mL anti-TSLP antibody, wherein the anti-TSLP antibody comprises a heavy chain set forth in SEQ ID NO: 139 and a light chain set forth in SEQ ID NO: 140;
(b) 0.1-0.8 mg/mL polysorbate;
(c) 10-30 mM histidine-acetate buffer; and
(d) 30-70 mg/mL sugar and 10-30 mM amino acid; or
the pharmaceutical composition comprises:
(a) 100-150 mg/mL anti-TSLP antibody, wherein the anti-TSLP antibody comprises a heavy chain set forth in SEQ ID NO: 139 and a light chain set forth in SEQ ID NO: 140;
(b) 0.1-0.8 mg/mL polysorbate;
(c) 10-30 mM histidine-acetate buffer; and
(d) 30-70 mg/mL sugar and 0.37-10 mM EDTA; or
the pharmaceutical composition comprises:
(a) 100-150 mg/mL anti-TSLP antibody, wherein the anti-TSLP antibody comprises a heavy chain set forth in SEQ ID NO: 139 and a light chain set forth in SEQ ID NO: 140;
(b) 0.1-0.8 mg/mL polysorbate;
(c) 10-30 mM histidine-acetate buffer; and
(d) 30-70 mg/mL sugar, 0.37-10 mM EDTA, and 10-30 mM amino acid;
wherein:
the sugar is selected from the group consisting of trehalose, sucrose, sorbitol, and mannitol;
the amino acid is selected from the group consisting of histidine, tryptophan, and methionine;
the polysorbate is selected from the group consisting of polysorbate 80 and polysorbate 20; and
the pharmaceutical composition has a pH of 5.5-6.5; preferably, the pharmaceutical composition has a pH of about 6.0-6.3.

In some embodiments, provided is the pharmaceutical composition described above, which comprises the following components:
(a) 100-150 mg/mL anti-TSLP antibody; (b) 0.1-0.8 mg/mL polysorbate; and (c) 10-30 mM acetate buffer; wherein the pharmaceutical composition has a pH of 4.5-6.5; preferably, the pharmaceutical composition has a pH of 4.5-5.5.

In some embodiments, provided is the pharmaceutical composition described above, which comprises the following components:
(a) 100-150 mg/mL anti-TSLP antibody; (b) 0.1-0.8 mg/mL polysorbate 80; and (c) 10-30 mM acetate buffer; wherein the pharmaceutical composition has a pH of 4.5-6.5; preferably, the pharmaceutical composition has a pH of 4.5-5.5.

In some embodiments, provided is the pharmaceutical composition described above, which comprises the following components:
(a) 100-150 mg/mL anti-TSLP antibody; (b) 0.1-0.8 mg/mL polysorbate; (c) 10-30 mM acetate buffer; and (d) 30-100 mg/mL sugar; wherein the pharmaceutical composition has a pH of 4.5-6.5; preferably, the pharmaceutical composition has a pH of 4.5-5.5.

In some embodiments, provided is the pharmaceutical composition described above, which comprises the following components:
(a) 100-150 mg/mL anti-TSLP antibody; (b) 0.1-0.8 mg/mL polysorbate; (c) 10-30 mM acetate buffer; and (d) 50-70 mg/mL sugar; wherein the pharmaceutical composition has a pH of 4.5-6.5; preferably, the pharmaceutical composition has a pH of 4.5-5.5.

In some embodiments, provided is the pharmaceutical composition described above, which comprises the following components:
(a) 100-150 mg/mL anti-TSLP antibody; (b) 0.1-0.8 mg/mL polysorbate 80; (c) 10-30 mM acetate buffer; and (d) 30-70 mg/mL sucrose; wherein the pharmaceutical composition has a pH of 4.5-6.5; preferably, the pharmaceutical composition has a pH of 4.5-5.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM succinic acid-sodium succinate buffer, wherein the pharmaceutical composition has a pH of about 5.0-5.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM succinic acid-sodium succinate buffer, wherein the pharmaceutical composition has a pH of about 5.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM succinic acid-sodium succinate buffer, wherein the pharmaceutical composition has a pH of about 5.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM citric acid-sodium citrate buffer, wherein the pharmaceutical composition has a pH of about 5.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM histidine-hydrochloride buffer, wherein the pharmaceutical composition has a pH of about 5.5-6.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM histidine-hydrochloride buffer, wherein the pharmaceutical composition has a pH of about 5.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM histidine-hydrochloride buffer, wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM histidine-hydrochloride buffer, wherein the pharmaceutical composition has a pH of about 6.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM histidine-acetate buffer, wherein the pharmaceutical composition has a pH of about 5.5-6.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM histidine-acetate buffer, wherein the pharmaceutical composition has a pH of about 5.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM histidine-acetate buffer, wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM histidine-acetate buffer, wherein the pharmaceutical composition has a pH of about 6.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM phosphate buffer, wherein the pharmaceutical composition has a pH of about 6.0-7.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM phosphate buffer, wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM phosphate buffer, wherein the pharmaceutical composition has a pH of about 7.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM acetic acid-sodium acetate buffer, wherein the pharmaceutical composition has a pH of about 4.5-5.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM acetic acid-sodium acetate buffer, wherein the pharmaceutical composition has a pH of about 4.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM acetic acid-sodium acetate buffer, wherein the pharmaceutical composition has a pH of about 5.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.1 mg/mL polysorbate 80; and (c) 20 mM acetic acid-sodium acetate buffer, wherein the pharmaceutical composition has a pH of about 5.5.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.2-0.6 mg/mL polysorbate 80; (c) 20 mM histidine-hydrochloride buffer; and (d) 70 mg/mL sucrose; wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.2-0.6 mg/mL polysorbate 20; (c) 20 mM histidine-hydrochloride buffer; and (d) 70 mg/mL sucrose; wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.4 mg/mL polysorbate 80; (c) 20 mM histidine-hydrochloride buffer; and (d) 50-70 mg/mL sucrose; wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.4 mg/mL polysorbate 80; (c) 20 mM histidine-hydrochloride buffer; and (d) 50-70 mg/mL trehalose; wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.4 mg/mL polysorbate 80; (c) 20 mM histidine-hydrochloride buffer; and (d) 50-70 mg/mL sorbitol; wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.4 mg/mL polysorbate 80; (c) 20 mM histidine-hydrochloride buffer; and (d) 50-70 mg/mL mannitol; wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.8 mg/mL polysorbate 80; (c) 20 mM histidine-hydrochloride buffer; (d) 70 mg/mL sucrose; and (e) 30 mM amino acid; wherein the amino acid is selected from the group consisting of histidine, tryptophan, and methionine; and the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.8 mg/mL polysorbate 80; (c) 20 mM histidine-hydrochloride buffer; (d) 70 mg/mL sucrose; and (e) 10 mM EDTA; wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.8 mg/mL polysorbate 80; (c) 20 mM histidine-hydrochloride buffer; (d) 70 mg/mL sucrose; and (e) 10 mM EDTA and 20 mM amino acid; wherein the amino acid is selected from the group consisting of histidine, tryptophan, and methionine; and the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.8 mg/mL polysorbate 80; (c) 20 mM histidine-hydrochloride buffer; (d) 70 mg/mL sucrose; and (e) 10 mM EDTA and 20 mM methionine; wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.8 mg/mL polysorbate 80; (c) 20 mM histidine-hydrochloride buffer; (d) 70 mg/mL sucrose; and (e) 0.37-10 mM EDTA; wherein the pharmaceutical composition has a pH of about 6.0.

In some embodiments, provided is the pharmaceutical composition described above, which comprises the following components:
(a) 100 mg/mL anti-TSLP antibody, wherein the anti-TSLP antibody comprises a heavy chain set forth in SEQ ID NO: 139 and a light chain set forth in SEQ ID NO: 140; (b) 0.8 mg/mL polysorbate 80; (c) 20 mM histidine-hydrochloride buffer; (d) 70 mg/mL sucrose; and (e) 0.37-5 mM EDTA; wherein the pharmaceutical composition has a pH of about 6.0.

In some embodiments, provided is the pharmaceutical composition described above, which comprises the following components:
(a) 150 mg/mL anti-TSLP antibody, wherein the anti-TSLP antibody comprises a heavy chain set forth in SEQ ID NO: 139 and a light chain set forth in SEQ ID NO: 140; (b) 0.4 mg/mL polysorbate 80; (c) 20 mM histidine-hydrochloride buffer; and (d) 70 mg/mL sucrose; wherein the pharmaceutical composition has a pH of about 6.0.

The present disclosure further provides a lyophilized formulation comprising an anti-TSLP antibody, wherein the lyophilized formulation is obtained by lyophilizing the pharmaceutical composition as described in any one of the above.

The present disclosure further provides a method for preparing a pharmaceutical composition as described in any one of the above, which comprises the step of buffer-exchanging an anti-TSLP antibody stock solution.

The present disclosure further provides an article of manufacture, which comprises a container containing the pharmaceutical composition as described in any one of the above or the lyophilized formulation as described above.

The present disclosure further provides a method for treating a disease or disorder, which comprises administering to a subject a therapeutically effective amount of the pharmaceutical composition as described in any one of the above or the lyophilized formulation as described above.

The present disclosure further provides use of the pharmaceutical composition as described in any one of the above or the lyophilized formulation as described above in the preparation of a medicament for treating a disease or disorder.

The pharmaceutical composition as described in any one of the above or the lyophilized formulation as described above of the present disclosure can be used as a medicament for treating a disease or disorder.

In some embodiments, the disease or disorder is selected from the group consisting of an allergic disease, cancer, and an immune disease; wherein the allergic disease is selected from the group consisting of: asthma, idiopathic pulmonary fibrosis, atopic dermatitis, allergic conjunctivitis, allergic rhinitis, allergic sinusitis, urticaria, Netherton syndrome, eosinophilic esophagitis, food allergy, allergic diarrhea, eosinophilic gastroenteritis, allergic bronchopulmonary aspergillosis, allergic fungal sinusitis, and chronic pruritus; the cancer is selected from the group consisting of: breast cancer, colon cancer, lung cancer, ovarian cancer, and prostate cancer; and the immune disease is selected from the group consisting of: rheumatoid arthritis, chronic obstructive pulmonary disease, systemic sclerosis, multiple sclerosis, keloid, ulcerative colitis, nasal polyposis, chronic eosinophilic pneumonia, eosinophilic bronchitis, celiac disease, Churg-Strauss syndrome, eosinophilic myalgia syndrome, hypereosinophilic syndrome, eosinophilic granulomatosis with polyangiitis, inflammatory bowel disease, scleroderma, interstitial lung disease, fibrosis induced by chronic hepatitis B or C, radiation-induced fibrosis, and fibrosis induced by wound healing.

In some embodiments, the disease or disorder described above is related to TSLP.

### BRIEF DESCRIPTION OF THE DRAWINGS

FIG. 1: results for the blocking of the binding activity of TSLP against the TSLP receptor by the antibodies.
FIG. 2: results for the blocking of the binding activity of TSLP against the TSLP receptor on the cell surface by the antibodies.
FIG. 3: inhibition of TSLP-induced proliferative activity of BaF3 cells by the antibodies.
FIG. 4A: inhibition of TSLP-induced production activity of the chemokine TARC by the antibodies.
FIG. 4B: inhibition of TSLP-induced production activity of the chemokine OPG by the antibodies.
FIG. 5A: inhibition of the production activity of the Th2 cytokine IL-13 by the antibodies.
FIG. 5B: inhibition of the production activity of the Th2 cytokine IL-4 by the antibodies.
FIG. 5C: inhibition of the production activity of the Th2 cytokine TNF-α by the antibodies.
FIG. 5D: inhibition of the production activity of the Th2 cytokine IL-5 by the antibodies.

### DETAILED DESCRIPTION

### Terms

In order to facilitate the understanding of the present disclosure, some technical and scientific terms are specifically defined below. Unless otherwise explicitly defined herein, all other technical and scientific terms used herein have the meanings generally understood by those of ordinary skill in the art to which the present disclosure belongs.

As used in the description and in the claims, the singular forms of "a", "an", and "the" include plural referents unless otherwise clearly indicated in the context.

Unless otherwise clearly stated in the context, throughout the description and the claims, the words "comprise", "have", "include", and the like, should be understood in an inclusive sense as opposed to an exclusive or exhaustive sense; that is, the meaning of "including but not limited to".

The term "and/or", such as "X and/or Y" should be understood to refer to "X and Y" or "X or Y", and should be used to provide explicit support for both or either meaning.

The term "thymic stromal lymphopoietin (TSLP)" is a four-α-helical bundle type I cytokine, also an epithelial cell-derived cytokine produced in response to pro-inflammatory stimuli, closely related to interleukin-7 (IL-7), which initiates allergic reactions by stimulating dendritic cells (DCs), and is an important factor in regulating the human immune response. The term "TSLP" includes variants, isotypes, homologs, orthologs, and paralogs of TSLP.

"Buffer" refers to a buffer that resists changes in pH by the action of its acid-base conjugate components. Examples of buffers that control the pH in an appropriate range include acetate, succinate, gluconate, histidine salt, oxalate, lactate, phosphate, citrate, tartrate, fumarate, glycylglycine and other organic acid buffers.

"Histidine salt buffer" is a buffer comprising histidine ions. Examples of histidine salt buffers include histidine-hydrochloride buffer, histidine-acetate buffer, histidine-phosphate buffer, histidine-sulfate buffer, and the like, and the histidine-acetate buffer or the histidine-hydrochloride buffer is preferred. The histidine-acetate buffer is prepared from histidine and acetic acid, and the histidine-hydrochloride buffer is prepared from histidine and hydrochloric acid.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate. "Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from succinic acid and sodium hydroxide, or from succinic acid and sodium succinate.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Acetate buffer" is a buffer comprising acetate ions. Examples of acetate buffers include acetic acid-sodium acetate, acetic acid-histidine salt, acetic acid-potassium acetate, acetic acid-calcium acetate, acetic acid-magnesium acetate, and the like. The preferred acetate buffer is acetic acid-sodium acetate.

"Surfactant" refers to a surface-active agent, preferably a nonionic surface-active agent. The use of surfactants can reduce aggregation of proteins in the formulation and/or reduce particle formation. The amount of surfactant added is an amount such that it can reduce aggregation of proteins in the formulation and minimize particle formation. The surfactant of the present disclosure may be selected from the group consisting of polysorbate (including but not limited to polysorbate 20 or polysorbate 80), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymer of ethylene and propylene glycol, and the like. The preferred surfactant is polysorbate 80 or polysorbate 20, and the more preferred surfactant is polysorbate 80.

"Stabilizer" refers to a component that helps maintain the structural integrity of a biopharmaceutical drug, particularly during freezing and/or lyophilization and/or storage (particularly when exposed to stress). The stabilization may occur for a variety of reasons, and generally the stabilizer may act as an osmotic agent to reduce protein denaturation. As used herein, the stabilizer contains a sugar, an amino acid, and EDTA. The amino acid in the stabilizer described herein is an amino acid added in addition to the amino acid in the buffer.

"Sugar" of the present disclosure includes the general composition (CH₂O)ₙ and derivatives thereof, including monosaccharides, disaccharides, trisaccharides, polysaccharides, sugar alcohols, reducing sugars, non-reducing sugars, and the like. The sugar of the present disclosure may be selected from the group consisting of glucose, sucrose, trehalose, lactose, fructose, maltose, dextran, glycerin, erythritol, glycerol, arabitol, xylitol, sorbitol, mannitol, mellibiose, melezitose, raffinose, mannotriose, stachyose, maltose, lactulose, maltulose, glucitol, maltitol, lactitol, iso-maltulose, and the like. The preferred sugars are sucrose, trehalose, sorbitol, and mannitol, the more preferred sugar is trehalose or sucrose, and the most preferred sugar is sucrose.

"Exchange" refers to the exchange of a solvent system that solubilizes an antibody protein. For example, a high-salt or hypertonic solvent system comprising the antibody protein is exchanged, by physical operations, with a buffer system of a stable formulation, such that the antibody protein is present in the stable formulation. The physical operations include, but are not limited to, ultrafiltration, dialysis or reconstitution following centrifugation.

The terms "about" and "approximately" as used herein mean that a numerical value is within an acceptable error range for the specific value determined by those of ordinary skill in the art, and the numerical value depends in part on how the value is measured or determined (i.e., the limits of the measurement system). For example, "about" may mean a standard deviation within 1 or more than 1 in each practice in the art. Alternatively, "about" or "substantially comprising" may mean a range of ±10% of a specific numerical value indicated thereafter. Furthermore, particularly for biological systems or processes, the term may mean up to an order of magnitude or up to 5-fold of a numerical value. Unless otherwise stated, when a specific value is provided in the present application and claims, the meaning of "about" or "substantially comprising" should be assumed to be within an acceptable error range for that specific value.

While the present disclosure provides content ranges or values, those of ordinary skill in the art will understand that the content ranges or values encompass acceptable error ranges for the specific value determined.

"Pharmaceutical composition" refers to a mixture containing one or more of the antibody drug conjugates described herein or physiologically/pharmaceutically acceptable salts or prodrugs thereof, and other chemical components, wherein the other components are, for example, physiologically/pharmaceutically acceptable carriers and excipients. The purpose of the pharmaceutical composition is to maintain the stability of the active ingredient of the antibody and promote the administration to an organism, which facilitates the absorption of the active ingredient, thereby exerting biological activity.

As used herein, "pharmaceutical composition" and "formulation" are not mutually exclusive. Unless otherwise stated, the solvent in the pharmaceutical composition described herein in solution form is water.

The pharmaceutical composition described herein can achieve a stable effect: a pharmaceutical composition in which the antibody substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable formulation is one in which no significant change is observed under the following conditions: storage at refrigeration temperature (2-8 °C) for at least 3 months, preferably 6 months, more preferably 1 year, and even more preferably up to 2 years. In addition, a stable liquid formulation includes a liquid formulation that exhibits desirable characteristics after storage at temperatures including 25 °C for periods including 1 month, 3 months, and 6 months. Typical examples for stability are as follows: generally, no more than about 10%, preferably no more than about 5%, of antibody monomers aggregate or are degraded as measured by SEC-HPLC. The formulation is a liquid that is colorless to yellowish, clear and transparent to slightly opalescent by visual analysis. The concentration, pH, and osmolality of the formulation have a change of no more than ±10%. Generally, a decrease of no more than about 10%, preferably no more than about 5% is observed. Generally, aggregation of no more than about 10%, preferably no more than about 5% is formed.

An antibody drug conjugate "retains its physical stability" in a pharmaceutical formulation if it shows no significant increase in aggregation, precipitation, and/or denaturation upon visual inspection of color and/or clarity, or as determined by ultra-violet (UV) light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed forms of the protein. Degradation processes that often change the chemical structure of proteins include hydrolysis or clipping (evaluated by methods such as size exclusion chromatography and capillary electrophoresis sodiu dodecyl sulfate (CE-SDS)), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, and the like).

An antibody "retains its biological activity" in a pharmaceutical formulation if the biological activity of the antibody at a given time is within a predetermined range of the biological activity exhibited during the preparation of the pharmaceutical formulation.

"Lyophilized formulation" refers to a formulation or a pharmaceutical composition obtained by lyophilizing a pharmaceutical composition or a formulation in liquid or solution form in vacuum.

The three-letter and single-letter codes for amino acids used in the present disclosure are as described in J. Biol. Chem., 243, p3558 (1968).

The term "antibody" herein is used in the broadest sense and encompasses a variety of antibody structures, including but not limited to monoclonal antibodies, polyclonal antibodies, multispecific antibodies (e.g., bispecific antibodies), full-length antibodies or antigen-binding fragments thereof (also known as antigen-binding moieties) so long as they exhibit the desired antigen-binding activity. A full-length antibody is an immunoglobulin (Ig) that comprises at least two heavy chains and two light chains interconnected by disulfide bonds. According to differences in the amino acid composition and the order of arrangement of the heavy chain constant regions of immunoglobulins, immunoglobulins can be divided into five classes, otherwise called isotypes of immunoglobulins, namely IgM, IgD, IgG, IgA and IgE, with their corresponding heavy chains being µ chain, δ chain, γ chain, α chain and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3 and IgG4. Light chains are divided into κ or λ chains according to differences in the constant regions. Each of the five classes of Ig may have a κ chain or λ chain.

The term "variable region" or "variable domain" refers to a domain in an antibody heavy or light chain that is involved in binding of the antibody to an antigen. VH and VL each comprise four conserved framework regions (FRs) and three complementarity determining regions (CDRs). The term "complementarity determining region" or "CDR" refers to a region in the variable domain that primarily contributes to antigen binding. "framework" or "FR" refers to variable domain residues other than CDR residues. VH comprises 3 CDR regions: HCDR1, HCDR2, and HCDR3; and VL comprises 3 CDR regions: LCDR1, LCDR2, and LCDR3. Each VH and VL is composed of three CDRs and four FRs arranged from the amino terminus to the carboxyl terminus in the following order: FR1, CDR1, FR2, CDR2, FR3, CDR3, and FR4. A single VH or VL may be sufficient to provide antigen-binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGen Tics (IMGT) numbering scheme (Lefranc, M.P. et al., Dev. Comp. Immunol., 27, 55-77 (2003); Front Immunol. 2018 Oct 16; 9: 2278), and the like. The relationships between the numbering systems are well known to those skilled in the art and are shown in Table 1 below.

**Table 1 Relationships between CDR numbering systems**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the sequences of the variable regions and CDRs in examples of the present disclosure.

The term "antigen-binding fragment" or "functional fragment" or "antigen-binding moiety" refers to one or more fragments of an intact antibody that retain the ability to specifically bind to an antigen. It is shown that a fragment of a full-length antibody can be used to perform the antigen-binding function of the antibody. Illustratively, examples of the binding fragment encompassed in the term "antigen-binding fragment" include (i) a Fab fragment, a monovalent fragment consisting of VL, VH, CL and CH1 domains; (ii) an F(ab')₂ fragment, a bivalent fragment comprising two Fab fragments linked by a disulfide bridge in the hinge region; (iii) an Fd fragment, consisting of VH and CH1 domains; (iv) an Fv fragment, consisting of VH and VL domains of a single arm of an antibody; (V) a dsFv, a stable antigen-binding fragment formed by VH and VL via interchain disulfide bonds; (vi) an scFv; (vii) a diabody, a bispecific antibody, and a multispecific antibody, comprising fragments such as an scFv, a dsFv, and a Fab.

The term "single chain antibody", "single chain Fv" or "scFv" refers to a molecule comprising an antibody heavy chain variable domain (or region; VH) and an antibody light chain variable domain (or region; VL) linked by a linker. Such scFv molecules may have a general structure: NH₂-VL-linker-VH-COOH or NH₂-VH-linker-VL-COOH. Numerous linkers suitable for linking VH and VL of an antibody have been disclosed in the prior art, for example, consisting of repeated GGGGS amino acid sequences or variants thereof, for example using variants of 1-4 repeats (Holliger et al (1993), Proc. Natl. Acad. Sci. USA 90: 6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061; Kipriyanov et al. (1999), J. Mol. Biol. 293:41-56; and Roovers et al. (2001), Cancer Immunol*.*

A diabody is an antibody fragment in which an scFv or Fab is dimerized, and is an antibody fragment with bivalent antigen-binding activity. In the bivalent antigen-binding activity, the two antigens may be identical or different.

Bispecific antibodies and multispecific antibodies refer to antibodies that can simultaneously bind to two or more antigens or antigenic determinants and comprise scFv or Fab fragments that can bind to TSLP.

The "conventional variant" of the human antibody heavy chain constant region and the human antibody light chain constant region described herein refers to a variant of heavy chain constant region or light chain constant region derived from human that has been disclosed in the prior art and does not change the structure and function of the antibody variable region. Exemplary variants include IgG1, IgG2, IgG3 or IgG4 heavy chain constant region variants with site-directed modifications and amino acid substitutions in the heavy chain constant region. Specific substitutions are, for example, YTE mutation, L234A and/or L235A mutation, or S228P mutation, and/or mutations to obtain a knob-into-hole structure (so that the antibody heavy chain has a combination of knob-Fc and hole-Fc) known in the art. Those mutations have been confirmed to confer new properties on the antibody, but do not change the function of the antibody variable regions.

The terms "full-length antibody", "intact antibody", "complete antibody" and "whole antibody" are used interchangeably herein to refer to an antibody in its substantially intact form, as distinguished from an antigen-binding fragment defined below. The term especially refers to antibodies in which the light and heavy chains comprise constant regions.

The terms "specific binding", "selective binding", "selectively bind to" and "specifically bind to" refer to the binding of an antibody to an epitope on a predetermined antigen. Generally, the antibody binds with an affinity (KD) of less than about 10⁻⁸ M, e.g., less than about 10⁻⁹ M, 10⁻¹⁰ M, 10⁻¹¹ M, 10⁻¹² M, or less.

The term "KD" refers to the dissociation equilibrium constant for specific antibody-antigen interaction. Generally, the antibody of the present disclosure binds to TSLP with a dissociation equilibrium constant (KD) of less than about 10⁻⁷ M, e.g., less than about 10⁻⁸ M or 10⁻⁹ M. For example, the KD value for the affinity of an antibody to an antigen on the cell surface is determined by the FACS or Biacore method in the present disclosure.

The term "nucleic acid molecule" used herein refers to a DNA molecule and an RNA molecule. The nucleic acid molecule may be single-stranded or double-stranded, and is preferably a double-stranded DNA, a single-stranded mRNA or a modified mRNA. A nucleic acid is "operably linked" when it is placed into a functional relationship with another nucleic acid sequence. For example, a promoter or enhancer is operably linked to a coding sequence if it affects the transcription of the coding sequence.

The amino acid sequence "identity" refers to the percentage of amino acid residues shared by a first sequence and a second sequence, wherein in aligning the amino acid sequences and when necessary, gaps are introduced to achieve maximum percent sequence identity, and any conservative substitution is not considered as part of the sequence identity. For the purpose of determining percent amino acid sequence identity, alignments can be achieved in a variety of ways that are within the skill in the art, for example, using publicly available computer software such as BLAST, BLAST-2, ALIGN, ALIGN-2 or Megalign (DNASTAR) software. Those skilled in the art can determine parameters suitable for measuring alignment, including any algorithms required to achieve maximum alignment of the full length of the aligned sequences.

The term "expression vector" refers to a nucleic acid molecule capable of transporting another nucleic acid to which it has been linked. In one embodiment, the vector is a "plasmid" that refers to a circular double-stranded DNA loop into which additional DNA segments may be ligated. In another embodiment, the vector is a viral vector, wherein additional DNA segments may be ligated into the viral genome. The vectors disclosed herein are capable of autonomous replication in a host cell into which they have been introduced (e.g., bacterial vectors having a bacterial origin of replication and episomal mammalian vectors) or being integrated into the genome of a host cell upon introduction into the host cell and thereby replicated along with the host genome (e.g., non-episomal mammalian vectors).

Methods for producing and purifying antibodies and antigen-binding fragments are well known in the art, for example, those described in chapters 5-8 and 15 of "Antibodies: A Laboratory Manual", Cold Spring Harbor Press. For example, mice can be immunized with human TSLP or a fragment thereof, and the obtained antibodies can be renatured and purified, and amino acid sequencing can be performed by conventional methods. Likewise, antigen-binding fragments can be prepared by conventional methods. The antibody or antigen-binding fragment described herein is genetically engineered to contain one or more additional human FRs in the non-human CDRs. Human FR species sequences can be obtained at the website http://imgt.cines.fr of ImMunoGeneTics (IMGT) or from the immunoglobulin journal, 2001ISBN012441351, by alignment with the IMGT human antibody variable region germline gene database with the MOE software.

The term "host cell" refers to a cell into which an expression vector has been introduced. Host cells may include bacterial, microbial, plant or animal cells. Bacteria susceptible to transformation include members of the *Enterobacteriaceae* family, such as strains of *Escherichia coli* or *Salmonella*; members of the *Bacillaceae* family, such as *Bacillus subtilis*; *Pneumococcus*; *Streptococcus* and *Haemophilus influenzae.* Suitable microorganisms include *Saccharomyces cerevisiae* and *Pichia pastoris.* Suitable animal host cell lines include CHO (Chinese hamster ovary cell lines), 293, and NS0 cells.

The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of antibodies, particularly at the highly conserved N-terminal site of the Fc region. Stable clones are obtained by expression of the antibody that specifically binds to human TSLP. Positive clones are expanded in a serum-free medium of a bioreactor to produce antibodies. The culture medium with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized. "Administering", "giving" and "treating", when applied to animals, humans, experimental subjects, cells, tissues, organs or biological fluid, refer to contacting an exogenous drug, a therapeutic agent, a diagnostic agent or composition with the animals, humans, subjects, cells, tissues, organs or biological fluid. "Administering", "giving" and "treating" can refer to, for example, therapeutic, pharmacokinetic, diagnostic, research and experimental methods. The treatment of cells comprises making the reagent in contact with the cells and making the reagent in contact with fluid, where the fluid is in contact with the cells. "Administering", "giving" and "treating" also refer to treating, e.g., cells by reagents, diagnosis, binding compositions or by another cell *in vitro* and *ex vivo.* "Treating", when applied to humans, veterinary or research subjects, refers to therapeutic treatment, preventive or prophylactic measures, and research and diagnostic applications.

"Treatment" refers to administering a therapeutic agent, such as a composition comprising any one of the conjugation compounds of the present disclosure, either internally or externally to a patient with one or more symptoms of a disease on which the therapeutic agent is known to have a therapeutic effect. Generally, the therapeutic agent is administered in an amount effective to alleviate one or more symptoms of the disease in the patient or population being treated to induce regression of such symptoms or to inhibit the development of such symptoms to any clinically measurable degree. The amount of therapeutic agent effective to alleviate any particular symptom of the disease (also known as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the present disclosure (for example, treatment methods or articles of manufacture) may not be effective in alleviating the symptoms of each disease of interest, they shall reduce the symptoms of the disease of interest in a statistically significant number of patients, as determined according to any statistical testing methods known in the art, such as Student t-test, chi-square test, Mann and Whitney's U test, Kruskal-Wallis test (H test), Jonckheere-Terpstra test and Wilcoxon test.

"Conservative modification" or "conservative replacement or substitution" refers to replacement of amino acids in a protein with other amino acids having similar characteristics (e.g., charge, side-chain size, hydrophobicity/hydrophilicity, or backbone conformation and rigidity), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th ed.)). In addition, the replacement of amino acids with similar structure or function is unlikely to disrupt the biological activity. Exemplary conservative substitutions are stated in the Table 2 "Exemplary amino acid conservative substitutions" below.

**Table 2. Exemplary amino acid conservative substitutions**

| Original residue | Conservative substitution |
|---|---|
| Ala (A) | Gly; Ser |
| Arg (R) | Lys; His |
| Asn (N) | Gln; His; Asp |
| Asp (D) | Glu; Asn |
| Cys (C) | Ser; Ala; Val |
| Gln (Q) | Asn; Glu |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu |

"Effective amount" or "effective dosage" refers to the amount of a drug, a compound or a pharmaceutical composition necessary to obtain any one or more beneficial or desired therapeutic results. For preventive use, the beneficial or desired results include elimination or reduction of risk, reduction of severity or delay of the onset of a disorder, including the biochemistry, histology and/or behavioral symptoms of the disorder, complications thereof and intermediate pathological phenotypes that appear during the progression of the disorder. For therapeutic applications, the beneficial or desired results include clinical results, such as reducing the incidence of various disorders related to the target antigen of the present disclosure or alleviating one or more symptoms of the disorder, reducing the dosage of other agents required to treat the disorder, enhancing the therapeutic effect of another agent, and/or delaying the progression of disorders of the patient related to the target antigen of the present disclosure.

"Exogenous" refers to substances produced outside organisms, cells or human bodies according to circumstances. "Endogenous" refers to substances produced inside cells, organisms or human bodies according to circumstances.

"Homology" refers to sequence similarity between two polynucleotide sequences or between two polypeptides. When positions in two compared sequences are occupied by identical bases or amino acid monomer subunits, e.g., if the position of each of two DNA molecules is occupied by adenine, the molecules are homologous at that position. The homology percentage between two sequences is a function of the number of matching or homologous positions shared by the two sequences divided by the number of positions compared × 100%. For example, in the optimal alignment of sequences, if 6 out of 10 positions of two sequences are matched or homologous, the two sequences are 60% homologous, and if 95 out of 100 positions of two sequences are matched or homologous, the two sequences are 95% homologous. Generally, two sequences, when aligned, are compared to give the maximum percent homology. For example, the comparison may be made by the BLAST algorithm, wherein the parameters of the algorithm are selected to give the maximum match between the reference sequences over the entire length of each sequence. The following references relate to the BLAST algorithm often used for sequence analysis: the BLAST algorithms: Altschul, S.F. et al., (1990) J. Mol. Biol., 215: 403-410; Gish, W., et al., (1993) Nature Genet., 3: 266-272; Madden, T.L. et al., (1996) Meth. Enzymol., 266: 131-141; Altschul, S.F. et al., (1997) Nucleic Acids Res., 25: 3389-3402; and Zhang, J. et al., (1997) Genome Res., 7: 649-656. Other conventional BLAST algorithms, such as one provided by NCBI BLAST, are also well known to those skilled in the art.

As used herein, the expressions "cell", "cell line", and "cell culture" are used interchangeably, and all such designations include their progenies. Therefore, the words "transformant" and "transformed cell" include primary test cells and cultures derived therefrom, regardless of the number of transfers. It should also be understood that all progenies may not be precisely identical in DNA content due to deliberate or unintentional mutations. Mutant progeny with identical function or biological activity as screened in the original transformed cells is included. When referring to different designations, they will become clear through the context. "Optional" or "optionally" means that the event or circumstance subsequently described may, but not necessarily, occur, and that the description includes instances where the event or circumstance occurs or does not occur.

There is no limitation for a TSLP-related disease in the present disclosure, as long as it is a disease related to TSLP. For example, a therapeutic response induced by the antibody of the present disclosure can be achieved by binding to human TSLP, then blocking the binding of TSLP to its receptor, or killing cells overexpressing TSLP; or inhibiting the growth of cells overexpressing TSLP.

The details of one or more embodiments of the present disclosure are set forth in the specification above. Although any methods and materials similar or identical to those described herein can be used in the practice or testing of the present disclosure, the preferred methods and materials are described below. Other features, objects and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

### Examples

The present disclosure is further described below with reference to the examples, which, however, are not intended to limit the scope of the present disclosure.

Experimental methods without specific conditions indicated in the examples or test examples of the present disclosure are generally conducted according to conventional conditions, or according to conditions recommended by the manufacturer of the starting materials or commercial products. See Sambrook et al., Molecular Cloning - A Laboratory Manual, Cold Spring Harbor Laboratory Press; Current Protocols in Molecular Biology, Ausubel et al., Greene Publishing Association, Wiley Interscience, NY. Reagents without specific origins indicated are commercially available conventional reagents.

### Part I. Antibody

### Example 1-1. Expression of TSLP and TSLP Receptor

The sequences encoding His-tagged human TSLP, His-tagged cynomolgus monkey TSLP, human IgG1-Fc-tagged human TSLP, human IgG1-Fc-tagged cynomolgus monkey TSLP, human TSLP receptor extracellular region were separately cloned into a phr vector to construct an expression plasmid, which was then used to transfect HEK293E. The specific transfection steps were as follows: the day before the transfection, HEK293E cells were seeded into a Freestyle expression medium (containing 1% FBS) at 0.8×10⁶ cells/mL and placed on a constant temperature shaker (120 rpm) at 37 °C for culture for 24 h. 24 h later, the transfection plasmid and transfection reagent PEI were sterilized with a 0.22 µm filter, and then the transfection plasmid was adjusted to a concentration of 100 µg/100 mL of cells, with the mass ratio of PEI (1 mg/mL) to the plasmid being 3:1. Taking the transfection of 200 mL of HEK293E cells as an example, 10 mL of Opti-MEM and 200 µg of plasmid were mixed uniformly and left to stand for 5 minutes (min); another 10 mL of Opti-MEM and 600 µg of PEI were mixed uniformly and left to stand for 5 min. The plasmid and PEI were mixed uniformly and left to stand for 15 min. The mixture of the plasmid and PEI was added slowly to 200 mL of HEK293E cells, which were then cultured in a shaker at 37 °C with 8% CO₂ at 120 rpm. On day 3 of transfection, 10% volume of feed medium was added. On day 6 of transfection, the samples were collected and centrifuged at 4500 rpm for 10 min, the cell supernatants were collected and filtered, and the recombinant TSLP and TSLP receptor protein supernatants were purified as described in Example 1-2. The purified proteins can be used in the following experiments of the examples. The related sequences are as shown below.
1. Amino acid sequence of his-tagged human TSLP antigen (huTSLP-his) 
MFPFALLYVLSVSFRKIFILQLVGLVLTYDFTNCDFEKIKAAYLSTISKDLITYMSGTKST
EFNNTVSCSNRPHCLTEIQSLTFNPTAGCASLAKEMFAMKTKAALAIWCPGYSETQINA
TQAMKKARKSKVTTNKCLEQVSQLQGLWRRFNRPLLKQQGSSDYKDDDDKHHHHHH
(SEQ ID NO: 1)
 Note: the underlined part indicates a signal peptide sequence; and the italicized part indicates a Flag-His6-tag.
2. Amino acid sequence of Fc-tagged human TSLP antigen (huTSLP-Fc) 
MFPFALLYVLSVSFRKIFILQLVGLVLTYDFTNCDFEKIKAAYLSTISKDLITYMSGTKST
EFNNTVSCSNRPHCLTEIQSLTFNPTAGCASLAKEMFAMKTKAALAIWCPGYSETQINA
TQAMKKARKSKVTTNKCLEQVSQLQGLWRRFNRPLLKQQDIEGRMDEPKSSDKTHTC
PPCPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGK (SEQ ID NO: 2)
 Note: the underlined part indicates a signal peptide sequence; and the italicized part indicates a linker-human Fc-tag.
3. Amino acid sequence of His-tagged cynomolgus monkey TSLP antigen (cynoTSLP-His) 
METDTLLLWVLLLWVPGSTGYDFTNCDFQKIEADYLRTISKDLITYMSGTKSTDFNNT
VSCSNRPHCLTEIQSLTFNPTPRCASLAKEMFARKTKATLALWCPGYSETQINATQAMK
KARKSKVTTNKCLEQVSQLLGLWRRFIRTLLKQQGSSDYKDDDDKHHHHHH
(SEQ ID NO: 3)
 Note: the underlined part indicates a signal peptide sequence; and the italicized part indicates a flag-His6-tag.
4. Amino acid sequence of Fc-tagged cynomolgus monkey TSLP antigen (cynoTSLP-Fc) 
METDTLLLWVLLLWVPGSTGYDFTNCDFQKIEADYLRTISKDLITYMSGTKSTDFNNT
VSCSNRPHCLTEIQSLTFNPTPRCASLAKEMFARKTKATLALWCPGYSETQINATQAMK
KARKSKVTTNKCLEQVSQLLGLWRRFIRTLLKQQDIEGRMDEPKSSDKTHTCPPCPAPE
LLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 4)
 Note: the underlined part indicates a signal peptide sequence; and the italicized part indicates a linker-human Fc-tag.
5. Amino acid sequence of Fc-tagged human TSLP receptor extracellular region (human TSLPR-Fc-ECD) 
GAAEGVQIQIIYFNLETVQVTWNASKYSRTNLTFHYRFNGDEAYDQCTNYLLQEGHTS
GCLLDAEQRDDILYFSIRNGTHPVFTASRWMVYYLKPSSPKHVRFSWHQDAVTVTCSD
LSYGDLLYEVQYRSPFDTEWQSKQENTCNVTIEGLDAEKCYSFWVRVKAMEDVYGPD
TYPSDWSEVTCWQRGEIRDACAETPTPPKPKLSKDIEGRMDEPKSSDKTHTCPPCPAPEL
LGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 5)
 Note: the underlined part indicates the extracellular region of human TSLPR, and the italicized part indicates a linker-human Fc-tag.

### Example 1-2. Purification of TSLP and TSLP Receptor (TSLPR) Recombinant Proteins

### 2.1 Purification of His-tagged TSLP recombinant proteins of various species

The cell expression supernatant was centrifuged at a high speed to remove impurities and filtered. A nickel column was equilibrated with a PBS solution and washed with 10 column volumes of PBS. The filtered supernatant was loaded on the column. The column was washed with a PBS solution containing 30 mM imidazole until A₂₈₀ reading dropped to baseline. The target protein was eluted with a PBS solution containing 300 mM imidazole, and the elution peak was collected. The eluate was concentrated and buffer-exchanged to PBS, identified by LC-MS, and subpackaged for later use if it was determined to be correct. Then, His-tagged human TSLP and His-tagged cynomolgus monkey TSLP were obtained.

### 2.2 Purification of human Fc-tagged TSLP recombinant proteins of various species and human Fc-tagged human TSLP receptor extracellular region recombinant proteins

The cell expression supernatant was centrifuged at a high speed to remove impurities, and the recombinant antibody expression supernatant was purified using a Protein A column. The column was washed with PBS until A₂₈₀ reading dropped to baseline. The target protein was eluted with 100 mM acetic acid (pH 3.5) and neutralized with 1 M Tris-HCl, pH 8.0. The eluate was concentrated and buffer-exchanged to PBS. The obtained protein was subjected to electrophoresis, identified by LC-MS, and subpackaged for later use if it was determined to be correct.

### Example 1-3. Construction and Identification of Cell Lines of Recombinant TSLP Receptor and IL-7Rα Receptor

To screen for antibodies that can block the binding of TSLP to the TSLP receptor, CHO-K1 and BaF3 cell strains expressing both human TSLP receptor and human IL-7Rα (TSLPR/IL-7Rα) were constructed. The target gene TSLPR/IL-7Rα was packaged by lentivirus and cloned into a target cell strain to form a stable high-expression cell strain. Human TSLPR and human IL-7Rα genes were first cloned into pCDH-CMV-MCS-EF1-puro and pCDH-CMV-MCS-EF1-Neo (SBI, CD500B-1) plasmids, respectively, then human TSLPR was cloned into CHO-K1 and BaF3 cell strains by lentivirus infection, and selectively cultured for three weeks under screening pressure of 10 µg/mL puromycin (Gibco, US). A second round of infection was performed on this basis, and the human IL-7Rα gene was cloned into the cell strains and screened with 1 mg/mL G418 (Gibco, US) and 10 µg/mL puromycin for two to three weeks. Finally, CHO-K1 and BaF3 monoclonal cell strains highly expressing both TSLPR and IL-7Rα were selected by a flow cytometry sorting method.

### Example 1-4. Preparation and Screening of Anti-Human TSLP Monoclonal Antibodies

The anti-human TSLP monoclonal antibodies are produced by immunizing mice, and the mice are laboratory SJL white mice, female, 6-8 weeks of age (Beijing Vital River Laboratory Animal Technology Co., Ltd., animal production license number: SCXK(Beijing)2012-0001). Housing environment: SPF. The purchased mice were housed in a laboratory environment for 1 week, in 12/12 hour light/dark cycles, at a temperature of 20-25 °C with humidity at 40-60%. The acclimatized mice were immunized with recombinant proteins huTSLP-Fc (25 µg), huTSLP-his (12.5 µg), and cynoTSLP-his (12.5 µg) as well as TiterMax, Alum or CpG adjuvant. After 4-5 immunizations, the mice in which the antibody titer in serum was high and was reaching a plateau were selected and sacrificed, from which spleen cells were taken for fusion with myeloma cells. Spleen lymphocytes and myeloma cells, Sp2/0 cells (ATCC^{®} CRL-8287^{™}), were fused by following an optimized PEG-mediated fusion procedure to obtain hybridoma cells.

Primary screening was performed by ELISA binding assays on human and cynomolgus monkey TSLP, assays on the blocking of the binding of human TSLP to its receptor TSLPR, and assays on the inhibition of TSLP-induced proliferation of BaF3 cells. After the hybridoma cells were transferred to a 24-well plate, the supernatants were rescreened. The selected positive clones were subjected to two rounds of subcloning to obtain hybridoma clones, which were used for antibody production and purified by an affinity method.

Monoclonal hybridoma cell strains No. 3, No. 119, No. 179, and No. 199 with good activity were obtained by screening, and hybridoma cells in the logarithmic growth phase were collected. RNA was extracted using NucleoZol (MN), and reverse transcription was performed (PrimeScript^{™} Reverse Transcriptase, Takara, cat # 2680A). The cDNA obtained by reverse transcription was amplified by PCR using mouse Ig-Primer Set (Novagen, TB326 Rev.B 0503) and sent for sequencing by a sequencing company. The murine anti-TSLP antibodies mab3, mab119, mab179, and mab199 were obtained by sequencing. Their variable region amino acid sequences are as follows (the underlined part denotes complementarity determining region sequences):
Murine heavy chain variable region sequence of mab3 (SEQ ID NO: 6): 
EVQLQQSGPVLVKPGASVKMSCKASGYTFTDDYMNWVKQSHGKSLEWIGIISPYNGGTSYN
QKFKGKATLTVDKSSSTAYMELNSLTSEDSAVYYCAREDYDYDGYAMDHWGQGTSVTVSS
Murine light chain variable region sequence of mab3 (SEQ ID NO: 7): 
QIVLSQSPAILSASPGEKVTMTCRASSSVSYMHWYQQKPGSSPKPWIYATSNLASGVPARFSGS
GSGTSYSLTISRVEAEDAATYYCQQWSSNRTFGGGTKLEIK
Murine heavy chain variable region sequence of mab119 (SEQ ID NO: 8): 
QAYLQQSGAELVRPGASVKMSCKASGFAFTTYNMHWVKHTPGQGLEWIGAIYPGNGETSYN
QKFKDRATLTVDKSSRTAYMQLSSLTSEDSAVYFCAREDDYGEGYFDVWGAGTTVTVSS
Murine light chain variable region sequence of mab119 (SEQ ID NO: 9): 
DIVLTQSPASLAVSLGQRATISCRASESVDNSGLSFMHWYQQKPGQPPRLLLYRASNLGSGIPA
RFSGSGSGTDFTLTLNPVETDDVATYYCQQINTDPLTFGAGTKLELK
Murine heavy chain variable region sequence of mab179 (SEQ ID NO: 10): 
QVQLQQSGAELVRPGTSVKVSCKASGYAFTNYLIEWVKQRPGQGLEWIGVIDPGNGDTNYN
ENFKGKATLTADKSSSTAYIELSRLTSEDSAVYFCAREDNTGTAFDYWGQGTTLTVSS
Murine light chain variable region sequence of mab179 (SEQ ID NO: 11): 
SIVMTQTPKFLLVSAGDRVTISCKASQSVSSDVTWYQQKPGQSPKLLIYYVSNHYTGVPDRFT
GSGYGTDFTFTISSVQAEDLAVYFCQQHHRFPLTFGAGTKLELK
Murine heavy chain variable region sequence of mab199 (SEQ ID NO: 12): 
QVQLQQSGPQLVRPGASVKISCKASGYSFTTYWMHWVKQRPGQGLEWIGMIDPSDSETTLI
QKFKDKATLTVDKSSSTAYMQLSSPTSEDSAVYYCARTLDGYYDYWGQGTTLTVSS
Murine light chain variable region sequence ofmab199 (SEQ ID NO: 13):

DIQMTQSPASLSASVGETVTITCRASENIYSYLAWYQQKQGKSPQLLVYFAKTLAEGVPSRFSG
SVSGTQFSLKINSLQPEDFGSYYCQHHYGTPWTFGGGTKLEIK

The amino acid sequences of the CDR regions obtained according to the Kabat numbering scheme are shown in Table 3 below:

**Table 3. CDR sequences of heavy and light chains of antibodies derived from hybridoma clones**

| **Antibody** | **Heavy chain** | | **Light chain** | |
|---|---|---|---|---|
| mab3 | HCDR1 | DDYMN SEQ ID NO: 14 | LCDR1 | RASSSVSYMH SEQ ID NO: 17 |
| | HCDR2 | IISPYNGGTSYNQKFKG | LCDR2 | ATSNLAS |
| | | SEQ ID NO: 15 | | SEQ ID NO: 18 |
| | HCDR3 | EDYDYDGYAMDH SEQ ID NO: 16 | LCDR3 | QQWSSNRT SEQ ID NO: 19 |
| mab119 | HCDR1 | TYNMH SEQ ID NO: 20 | LCDR1 | RASESVDNSGLSFMH SEQ ID NO: 23 |
| | HCDR2 | AIYPGNGETSYNQKFKD SEQ ID NO: 21 | LCDR2 | RASNLGS SEQ ID NO: 24 |
| | HCDR3 | EDDYGEGYFDV SEQ ID NO: 22 | LCDR3 | QQINTDPLT SEQ ID NO: 25 |
| mab179 | HCDR1 | NYLIE SEQ ID NO: 26 | LCDR1 | KASQSVSSDVT SEQ ID NO: 29 |
| | HCDR2 | VIDPGNGDTNYNENFKG SEQ ID NO: 27 | LCDR2 | YVSNHYT SEQ ID NO: 30 |
| | HCDR3 | EDNTGTAFDY SEQ ID NO: 28 | LCDR3 | QQHHRFPLT SEQ ID NO: 31 |
| mab199 | HCDR1 | TYWMH SEQ ID NO: 32 | LCDR1 | RASENIYSYLA SEQ ID NO: 35 |
| | HCDR2 | MIDPSDSETTLIQKFKD SEQ ID NO: 33 | LCDR2 | FAKTLAE SEQ ID NO: 36 |
| | HCDR3 | TLDGYYDY SEQ ID NO: 34 | LCDR3 | QHHYGTPWT SEQ ID NO: 37 |

The light and heavy chain variable regions of the murine antibodies described above were linked to the light and heavy chain constant regions of the human antibody (the kappa constant region set forth in SEQ ID NO: 134 and the IgG1-YTE constant region set forth in SEQ ID NO: 133) to form chimeric antibodies. The chimeric antibody corresponding to mab3 clone was named Ch3, the chimeric antibody corresponding to mab119 clone was named Ch119, the chimeric antibody corresponding to mab179 clone was named Ch179, and the chimeric antibody corresponding to mab199 was named Ch199.

### Example 1-5. Humanization Design of Anti-Human TSLP Monoclonal Antibodies

To reduce the immunogenicity of the murine antibodies, the selected antibodies with excellent *in vitro* and *in vivo* activity, mab3, mab119, mab179, and mab199, were humanized. Humanization of the murine monoclonal antibodies was performed according to the method described in many publications in the art. Briefly, the human antibody constant domains were used in place of parent (murine antibody) constant domains, and human species antibody sequences were selected, based on the homology of the murine and human antibodies, for CDR grafting. Then, based on the three-dimensional structures of the murine antibodies, the constant regions of the murine antibodies were replaced by human constant regions by back mutation of the amino acid residues of VL and VH to obtain final humanized molecules.

### 5.1 Selection and back mutation of human FR regions of mab3

### (1) Selection and back mutation of human FR regions

For mab3, the humanized VH template was IGHV1-3^{∗}01 + IGHJ6^{∗}01, and the humanized VL template was IGKV3-20 + IGKJ4*01. The CDRs of mab3 were grafted onto the humanized templates. The variable region sequences obtained after grafting are as follows:
hu3 VL-CDR grafted: 
EIVLTQSPATLSLSPGERATLSCRASSSVSYMHWYQQKPGQAPRLLIYATSNLASGIPARF
SGSGSGTDFTLTISRLEPEDFAVYYCQQWSSNRTFGGGTKVEIK (SEQ ID NO: 38)
hu3 VH-CDR grafted:

EVQLVQSGAEVKKPGASVKVSCKASGYTFTDDYMNWVRQAPGQRLEWMGIISPYNG
GTSYNQKFKGRVTITRDTSASTAYMELSSLRSEDTAVYYCAREDYDYDGYAMDHWGQ
GTTVTVSS (SEQ ID NO: 42)

The back mutation design for the humanized antibodies of mab3 is shown in Table 4 below:

**Table 4. Back mutations of humanized antibodies of mab3**

| **hu3 VL** | | **hu3VH** | |
|---|---|---|---|
| hu3VL1 | Grafted | hu3VH1 | Grafted |
| hu3VL2 | L46P, F71Y | hu3VH2 | I69L, R71V, T73K |
| hu3VL3 | L46P, L47W, I58V, F71Y | hu3VH3 | R38K, M48I, V67A, I69L, R71V, T73K |
| hu3VL4 | L46P, L47W, I58V, D70S, F71Y | | |

Note: grafted denotes that the murine antibody CDRs are grafted onto the human species FR region sequences, and L46P denotes that L at position 46 is back mutated to P according to the Kabat numbering scheme.

The variable region sequences of the humanized antibodies of mab3 obtained after grafting are as follows:
hu3VL1 (hu3 VL-CDR grafted) (SEQ ID NO: 38): 
EIVLTQSPATLSLSPGERATLSCRASSSVSYMHWYQQKPGQAPRLLIYATSNLASGIPARF
SGSGSGTDFTLTISRLEPEDFAVYYCQQWSSNRTFGGGTKVEIK
hu3VL2 (SEQ ID NO: 39): 
EIVLTQSPATLSLSPGERATLSCRASSSVSYMHWYQQKPGQAPRPLIYATSNLASGIPARF
SGSGSGTDYTLTISRLEPEDFAVYYCQQWSSNRTFGGGTKVEIK
hu3VL3 (SEQ ID NO: 40): 
EIVLTQSPATLSLSPGERATLSCRASSSVSYMHWYQQKPGQAPRPWIYATSNLASGVPA
RFSGSGSGTDYTLTISRLEPEDFAVYYCQQWSSNRTFGGGTKVEIK
hu3VL4 (SEQ ID NO: 41): 
EIVLTQSPATLSLSPGERATLSCRASSSVSYMHWYQQKPGQAPRPWIYATSNLASGVPA
RFSGSGSGTSYTLTISRLEPEDFAVYYCQQWSSNRTFGGGTKVEIK
hu3VH1 (hu3 VH-CDR Grafted) (SEQ ID NO: 42): 
EVQLVQSGAEVKKPGASVKVSCKASGYTFTDDYMNWVRQAPGQRLEWMGIISPYNG
GTSYNQKFKGRVTITRDTSASTAYMELSSLRSEDTAVYYCAREDYDYDGYAMDHWGQ
GTTVTVSS
hu3VH2 (SEQ ID NO: 43): 
EVQLVQSGAEVKKPGASVKVSCKASGYTFTDDYMNWVRQAPGQRLEWMGIISPYNG
GTSYNQKFKGRVTLTVDKSASTAYMELSSLRSEDTAVYYCAREDYDYDGYAMDHWG
QGTTVTVSS
hu3VH3 (SEQ ID NO: 44):

EVQLVQSGAEVKKPGASVKVSCKASGYTFTDDYMNWVKQAPGQRLEWIGIISPYNGG
TSYNQKFKGRATLTVDKSASTAYMELSSLRSEDTAVYYCAREDYDYDGYAMDHWGQ
GTTVTVSS

Note: the single underlined parts indicate the CDR regions, and the double underlined parts indicate the back mutation positions.

The light and heavy chain variable regions described above were combined with the human species light chain and heavy constant region sequences, respectively, to form final complete light and heavy chain sequences, and then the antibodies with the full-length sequences were obtained. Illustratively, the heavy chain constant region of the humanized antibodies of mab3 of the present disclosure was the IgG1-YTE constant region set forth in SEQ ID NO: 133, the light chain constant region was the kappa chain constant region set forth in SEQ ID NO: 134, and they may be replaced by other constant regions known in the art.

The sequences of the heavy and light chain variable regions of the humanized antibodies of mab3 obtained are shown in Table 5 below:

**Table 5. Heavy and light chain variable region sequences of humanized antibodies of mab3**

| Antibody | VH (SEQ ID NO) | VL (SEQ ID NO) |
|---|---|---|
| hu3-01 | 42 | 39 |
| hu3-02 | 42 | 40 |
| hu3-03 | 42 | 41 |
| hu3-04 | 43 | 38 |
| hu3-05 | 43 | 39 |
| hu3-06 | 43 | 40 |
| hu3-07 | 43 | 41 |
| hu3-08 | 44 | 39 |
| hu3-09 | 44 | 40 |
| hu3-10 | 44 | 41 |

The binding activity of the humanized antibodies of mab3 against human TSLP was assayed by an ELISA method. The results showed that the humanized antibodies of mab3 had good binding ability to human TSLP.

### (2) Point mutations of hu3 antibody

After the assay, it was found that there were hot spots on the MDH sequence of HCDR3 and the NTR sequence of LCDR3 of the humanized antibodies of mab3, and therefore, the corresponding hot spot positions were subjected to mutations. The CDR sequences of the humanized antibodies of mab3 obtained by the mutation are shown in Table 6 below:

**Table 6. HCDR3 and LCDR3 sequences after mutation**

| | |
|---|---|
| hu3 HCDR3-H110Y | EDYDYDGYAMD**Y** SEQ ID NO: 45 |
| hu3 LCDR3-N93D | QQWSS**D**RT SEQ ID NO: 46 |

| | |
|---|---|
| Note: the locations of the mutation positions in Table 6 are numbered in the natural order according to the variable region sequences. | |

Therefore, the CDR sequences of the humanized antibodies of mab3 are shown in Table 7 below:

**Table 7. CDRs of humanized antibodies of mab3 after mutation**

| **Heavy chain** | | **Light chain** | |
|---|---|---|---|
| HCDR1 | DDYMN SEQ ID NO: 14 | LCDR1 | RASSSVSYMH SEQ ID NO: 17 |
| HCDR2 | IISPYNGGTSYNQKFKG SEQ ID NO: 15 | LCDR2 | ATSNLAS SEQ ID NO: 18 |
| HCDR3 (general formula) | EDYDYDGYAMDX₁ SEQ ID NO: 47 | LCDR3 (general formula 1) | QQWSSX₂RT SEQ ID NO: 48 |

X₁ is selected from the group consisting of H and Y, and X₂ is selected from the group consisting of N and D.

Illustratively, the CDRs and the heavy and light chain variable regions of the humanized antibodies hu3-11 obtained by the mutation are shown in Table 8 below:

**Table 8. CDR regions of hu3-11**

| **Heavy chain** | | **Light chain** | |
|---|---|---|---|
| HCDR1 | DDYMN SEQ ID NO: 14 | LCDR1 | RASSSVSYMH SEQ ID NO: 17 |
| HCDR2 | IISPYNGGTSYNQKFKG SEQ ID NO: 15 | LCDR2 | ATSNLAS SEQ ID NO: 18 |
| HCDR3-H11 0Y | EDYDYDGYAMDY SEQ ID NO: 45 | LCDR3-N 93D | QQWSSDRT SEQ ID NO: 46 |

The amino acid sequence of the light chain variable region of hu3-11 (hu3VL4-N93D) is as follows:

EIVLTQSPATLSLSPGERATLSCRASSSVSYMHWYQQKPGQAPRPWIYATSNLASGVPA
RFSGSGSGTSYTLTISRLEPEDFAVYYCQQWSSDRTFGGGTKVEIK (SEQ ID NO: 49)

The amino acid sequence of the heavy chain variable region of hu3-11 (hu3VH2-H110Y) is as follows:

EVQLVQSGAEVKKPGASVKVSCKASGYTFTDDYMNWVRQAPGQRLEWMGIISPYNG
GTSYNQKFKGRVTLTVDKSASTAYMELSSLRSEDTAVYYCAREDYDYDGYAMDYWG
QGTTVTVSS (SEQ ID NO: 50)

The light and heavy chain variable regions after the hot spot mutation were combined with the human species light chain and heavy constant region sequences, respectively, to form final complete light and heavy chain sequences, and then the antibodies with the full-length sequences were obtained.

The binding activity of the antibodies obtained by the mutation against human TSLP was assayed by an ELISA method. The results showed that hu3-11 still had high affinity for human TSLP, suggesting that hot spot mutations on HCDR3 and LCDR3 of the humanized antibodies of mab3 do not affect the activity of the antibody.

### (3) Affinity maturation of antibody hu3-11

Affinity maturation was performed on the hu3-11 molecule. The process of affinity maturation is as follows:
Construction of yeast library: degenerate primers were designed, the designed mutant amino acids were introduced into scFv mutant libraries of the antibody hu3-11 by PCR, with the size of each library being about 10⁹, and the constructed yeast libraries were verified for library diversity by sequencing.

In the first round of screening, about 5×10¹⁰ cells from the hu3-11-scFv mutant libraries were incubated with biotinylated TSLP-Fc protein (1-10 µg/mL) in 50 mL of buffer containing 0.1% bovine serum albumin (BSA)-phosphate buffer (PBSA) for 1 h at room temperature. Then, the mixture was washed with 0.1% PBSA to remove unbound antibody fragments. Then, 100 µL of streptavidin microbeads (Milenvi Biotec, Auburn, CA) were added to the hu3-11-scFv antibody mutant libraries bound biotinylated TSLP-Fc and loaded onto the AutoMACS system for sorting. The cells of the antibody libraries with high affinity for TSLP-Fc were collected and induced at 250 rpm and 20 °C for 18 h. The obtained enriched library was subjected to a second round of screening for biotinylated recombinant TSLP-Fc protein.

For the third and fourth rounds of screening, library cells from the previous round were incubated with a biotinylated recombinant TSLP-Fc protein (0.1-1 µg/mL) and 10 µg/mL murine anti-cMyc (9E10, sigma) antibody in 0.1% PBSA at room temperature for 1 hour (h), and the mixture was washed with 0.1% PBSA to remove unbound antibody fragments. Goat anti-mouse-Alexa488 (A-11001, Life technologies) and Streptavidin-PE (S-866, Life technologies) were added and incubated at 4 °C for 1 h, and the mixture was washed with 0.1% PBSA to remove unbound antibody fragments. Finally, the high-affinity antibodies were selected by FACS.

The hu3-11-scFv mutant libraries were subjected to 2 rounds of MACS screening and 2 rounds of FACS screening using a biotinylated TSLP-Fc antigen. About 400 yeast monoclonals were selected for culture and expression induction, and the binding of the yeast monoclonals to the TSLP-Fc antigen was assayed by FACS. The yeast monoclonals with high affinity were selected for sequencing verification, and the sequenced clones were subjected to alignment and analysis to remove redundant sequences. After that, non-redundant sequences were converted into full-length antibodies for expression in mammalian cells.

The light chain variable region sequences obtained by affinity maturation are as follows:
hu3VL5 (SEQ ID NO: 51): 
EIVLTQSPATLSLSPGERATLSCRASSSVSYMHWYQQKPGQAPRPWIYATSNLASGVPA
RFSGSGSGTSYTLTISRLEPEDFAVYYCQQSDNVRGFGGGTKVEIK
hu3VL6 (SEQ ID NO: 52):

EIVLTQSPATLSLSPGERATLSCRASSSVSYMHWYQQKPGQAPRPWIYATSNLASGVPA
RFSGSGSGTSYTLTISRLEPEDFAVYYCQQSDSGREFGGGTKVEIK

Note: the single underlined parts indicate the CDR regions, and the double underlined parts indicate the back mutation positions.

The obtained light chain variable regions were recombined with the heavy chain variable regions of the humanized antibodies of mab3 to obtain novel humanized antibodies of mab3. Illustratively, huVL5 and huVL6 were each combined with hu3VH2-H110Y to obtain novel antibody molecules hu3-12 and hu3-13. The results are shown in table 9 below:

**Table 9. Antibodies obtained by affinity maturation**

| Antibody | hu3VH | hu3VL |
|---|---|---|
| hu3-12 | hu3VH2-H110Y | hu3VL5 |
| hu3-13 | hu3VH2-H110Y | hu3VL6 |

The CDR sequences of the humanized antibodies of mab obtained by affinity maturation are shown in Table 10 below:

**Table 10. CDRs of humanized antibodies of mab3 obtained by affinity maturation**

| **Antibody** | **Heavy chain** | | **Light chain** | |
|---|---|---|---|---|
| hu3-12 | HCDR1 | DDYMN SEQ ID NO: 14 | LCDR1 | RASSSVSYMH SEQ ID NO: 17 |
| | HCDR2 | IISPYNGGTSYNQKFKG SEQ ID NO: 15 | LCDR2 | ATSNLAS SEQ ID NO: 18 |
| | HCDR3-H110Y | EDYDYDGYAMDY SEQ ID NO: 45 | LCDR3-V1 | QQSDNVRG SEQ ID NO: 53 |
| hu3-13 | HCDR1 | DDYMN SEQ ID NO: 14 | LCDR1 | RASSSVSYMH SEQ ID NO: 17 |
| | HCDR2 | IISPYNGGTSYNQKFKG SEQ ID NO: 15 | LCDR2 | ATSNLAS SEQ ID NO: 18 |
| | HCDR3-H110Y | EDYDYDGYAMDY SEQ ID NO: 45 | LCDR3-V2 | QQSDSGRE SEQ ID NO: 54 |

The binding activity of the obtained novel humanized antibodies of mab3 to human TSLP was assayed by ELISA. The results showed that hu3-12 and hu3-13 still had strong binding ability to human TSLP. It suggests that certain amino acid changes in LCDR3 do not affect the activity of the series of hu3 antibodies.

In summary, the CDRs of the humanized antibodies of mab3 have the sequences shown in Table 11 below:

**Table 11. CDR sequences of general formulas of humanized antibodies of mab3**

| **Heavy chain** | | **Light chain** | |
|---|---|---|---|
| HCDR1 | DDYMN SEQ ID NO: 14 | LCDR1 | RASSSVSYMH SEQ ID NO: 17 |
| HCDR2 | IISPYNGGTSYNQKFKG | LCDR2 | ATSNLAS |
| | SEQ ID NO: 15 | | SEQ ID NO: 18 |
| HCDR3 (general formula) | EDYDYDGYAMDX₁ SEQ ID NO: 47 | LCDR3 (general formula 2) | QQSDX₃X₄RX₅ SEQ ID NO: 55 |

X₁ is H or Y, X₃ is N or S, X₄ is V or G, and X₅ is G or E.

The combinations of antibody heavy and light chain variable regions of humanized antibodies of mab3 after the hot spot mutation and affinity maturation are shown in Table 12 below:

**Table 12. Sequences of affinity-matured antibodies**

| Antibody | VH (SEQ ID NO) | VL (SEQ ID NO) |
|---|---|---|
| hu3-11 | 50 | 49 |
| hu3-12 | 50 | 51 |
| hu3-13 | 50 | 52 |

### 5.2 Selection and back mutation of human FR regions of mab119

### (1) Selection and back mutation of human FR regions

For mab119, IGHV1-69*02 + HJ6*01 was taken as the template for the VH, and IGKV4-1^{∗}01 + IGKJ2^{∗}01 and IGKV3-11*01 + IGKJ2*01 were taken as the templates for VL. The CDR regions of the murine antibody were grafted onto the selected humanized templates, and the FR regions were back mutated to obtain different light and heavy chain variable regions. The variable region sequences obtained by CDR grafting are as follows:
hu119VL (Grafted, IGKV4-1^{∗}01) (SEQ ID NO: 56): 
DIVMTQSPDSLAVSLGERATINCRASESVDNSGLSFMHWYQQKPGQPPKLLIYRASNLG
SGVPDRFSGSGSGTDFTLTISSLQAEDVAVYYCQQINTDPLTFGQGTKLEIK
hu119VL4 (Grafted, IGKV3-11*01) (SEQ ID NO: 59): 
EIVLTQSPATLSLSPGERATLSCRASESVDNSGLSFMHWYQQKPGQAPRLLIYRASNLGS
GIPARFSGSGSGTDFTLTISSLEPEDFAVYYCQQINTDPLTFGQGTKLEIK
hu119VH (Grafted, IGHV1-69*02) (SEQ ID NO: 62):

EVQLVQSGAEVKKPGSSVKVSCKASGGTFSTYNMHWVRQAPGQGLEWMGAIYPGNG
ETSYNQKFKDRVTITADKSTSTAYMELSSLRSEDTAVYYCAREDDYGEGYFDVWGQGT
TVTVSS

The back mutations of the humanized antibodies of mab119 are shown in Table 13 below:

**Table 13. Back mutations of mab119**

| **hu119VL** | | **hu119VH** | |
|---|---|---|---|
| hu119VL1 | Grafted (IGKV4-1*01) | hu119VH1 | Grafted (IGHV1-69*02) |
| hu119VL2 | Grafted (IGKV4-1*01)+M4L | hu119VH2 | G27F, I69L, A71V |
| hu119VL3 | Grafted (IGKV4-1*01)+I48L, V58I | hu119VH3 | G27F, M48I, V67A, I69L, A71V |
| hu119VL4 | Grafted (IGKV3-11*01) | hu119VH4 | G27F, R38K, Q39H, M48I, V67A, I69L, A71V |
| hu119VL5 | Grafted (IGKV3-11*01) +A43P, I48L | hu119VH5 | M48I, V67A, I69L, A71V |
| hu119VL6 | Grafted (IGKV3-11*01)+E1D, A43P, I48L | hu119VH6 | V2A, G27F, M48I, V67A, I69L, A71V |
| | | hu119VH7 | M48I, V67A, I69L, A71V, S76R |
| | | hu119VH8 | V2A, G27F, M48I, V67A, I69L, A71V, S76R |

| | | | |
|---|---|---|---|
| Note: for example, M4L denotes that M at position 4 is back mutated to L according to the Kabat numbering scheme. Grafted denotes that the murine antibody CDRs are grafted onto the human species FR region sequences. | | | |

The specific variable region sequences of the humanized antibodies of mab 119 are as follows:
hu119VL1 (Grafted (IGKV4-1^{∗}01)) (SEQ ID NO: 56): 
DIVMTQSPDSLAVSLGERATINCRASESVDNSGLSFMHWYQQKPGQPPKLLIYRASNLG
SGVPDRFSGSGSGTDFTLTISSLQAEDVAVYYCQQINTDPLTFGQGTKLEIK
hu119VL2 (SEQ ID NO: 57): DIVLTQSPDSLAVSLGERATINCRASESVDNSGLSFMHWYQQKPGQPPKLLIYRASNLG SGVPDRFSGSGSGTDFTLTISSLQAEDVAVYYCQQINTDPLTFGQGTKLEIK
hu119VL3 (SEQ ID NO: 58): 
DIVMTQSPDSLAVSLGERATINCRASESVDNSGLSFMHWYQQKPGQPPKLLLYRASNL
GSGIPDRFSGSGSGTDFTLTISSLQAEDVAVYYCQQINTDPLTFGQGTKLEIK
hu119VL4 (Grafted, IGKV3-11*01) (SEQ ID NO: 59): 
EIVLTQSPATLSLSPGERATLSCRASESVDNSGLSFMHWYQQKPGQAPRLLIYRASNLGS
GIPARFSGSGSGTDFTLTISSLEPEDFAVYYCQQINTDPLTFGQGTKLEIK
hu119VL5 (SEQ ID NO: 60): 
EIVLTQSPATLSLSPGERATLSCRASESVDNSGLSFMHWYQQKPGQPPRLLLYRASNLGS
GIPARFSGSGSGTDFTLTISSLEPEDFAVYYCQQINTDPLTFGQGTKLEIK
hu119VL6 (SEQ ID NO: 61): 
DIVLTQSPATLSLSPGERATLSCRASESVDNSGLSFMHWYQQKPGQPPRLLLYRASNLG
SGIPARFSGSGSGTDFTLTISSLEPEDFAVYYCQQINTDPLTFGQGTKLEIK
hu119VH1 (Grafted) (SEQ ID NO: 62): 
EVQLVQSGAEVKKPGSSVKVSCKASGGTFSTYNMHWVRQAPGQGLEWMGAIYPGNG
ETSYNQKFKDRVTITADKSTSTAYMELSSLRSEDTAVYYCAREDDYGEGYFDVWGQGT
TVTVSS
hu119VH2 (SEQ ID NO: 63): 
EVQLVQSGAEVKKPGSSVKVSCKASGFTFSTYNMHWVRQAPGQGLEWMGAIYPGNG
ETSYNQKFKDRVTLTVDKSTSTAYMELSSLRSEDTAVYYCAREDDYGEGYFDVWGQG
TTVTVSS
hu119VH3 (SEQ ID NO: 64): 
EVQLVQSGAEVKKPGSSVKVSCKASGFTFSTYNMHWVKHAPGQGLEWIGAIYPGNGE
TSYNQKFKDRATLTVDKSTSTAYMELSSLRSEDTAVYYCAREDDYGEGYFDVWGQGT
TVTVSS
hu119VH4 (SEQ ID NO: 65): 
EVQLVQSGAEVKKPGSSVKVSCKASGFTFSTYNMHWVKHAPGQGLEWIGAIYPGNGE
TSYNQKFKDRATLTVDKSTSTAYMELSSLRSEDTAVYYCAREDDYGEGYFDVWGQGT
TVTVSS
hu119VH5 (SEQ ID NO: 66): EVQLVQSGAEVKKPGSSVKVSCKASGGTFSTYNMHWVRQAPGQGLEWIGAIYPGNGE 
TSYNQKFKDRATLTVDKSTSTAYMELSSLRSEDTAVYYCAREDDYGEGYFDVWGQGT
TVTVSS
hu119VH6 (SEQ ID NO: 67): 
EAQLVQSGAEVKKPGSSVKVSCKASGFTFSTYNMHWVRQAPGQGLEWIGAIYPGNGE
TSYNQKFKDRATLTVDKSTSTAYMELSSLRSEDTAVYYCAREDDYGEGYFDVWGQGT
TVTVSS
hu119VH7 (SEQ ID NO: 68): 
EVQLVQSGAEVKKPGSSVKVSCKASGGTFSTYNMHWVRQAPGQGLEWIGAIYPGNGE
TSYNQKFKDRATLTVDKSTRTAYMELSSLRSEDTAVYYCAREDDYGEGYFDVWGQGT
TVTVSS
hu119VH8 (SEQ ID NO: 69):

EAQLVQSGAEVKKPGSSVKVSCKASGFTFSTYNMHWVRQAPGQGLEWIGAIYPGNGE
TSYNQKFKDRATLTVDKSTRTAYMELSSLRSEDTAVYYCAREDDYGEGYFDVWGQGT
TVTVSS

Note: the single underlined parts indicate the variable regions, and the double underlined parts indicate the back mutations.

The light and heavy chain variable regions described above were combined with the human species light and heavy chain constant region sequences, respectively, to form final complete light and heavy chain sequences, and then the antibodies with the full-length sequences were obtained. Illustratively, the heavy chain constant region of the humanized antibodies of mab119 of the present disclosure was the IgG1-YTE constant region set forth in SEQ ID NO: 133, the light chain constant region was the kappa chain constant region set forth in SEQ ID NO: 134, and they may be replaced by other constant regions known in the art.

The heavy and light chain variable regions of the humanized antibodies of mab119 are shown in Table 14.

**Table 14. Heavy and light chain variable regions of humanized antibodies of mab119**

| Antibody | VH (SEQ ID NO) | VL (SEQ ID NO) |
|---|---|---|
| hu119-01 | 62 | 56 |
| hu119-02 | 63 | 56 |
| hu119-03 | 64 | 56 |
| hu119-04 | 65 | 56 |
| hu119-05 | 62 | 57 |
| hu119-06 | 63 | 57 |
| hu119-07 | 64 | 57 |
| hu119-08 | 65 | 57 |
| hu119-09 | 62 | 58 |
| hu119-10 | 63 | 58 |
| hu119-11 | 64 | 58 |
| hu119-12 | 65 | 58 |
| hu119-13 | 64 | 59 |
| hu119-14 | 66 | 59 |
| hu119-15 | 67 | 59 |
| hu119-16 | 68 | 59 |
| hu119-17 | 69 | 59 |
| hu119-18 | 64 | 60 |
| hu119-19 | 66 | 60 |
| hu119-20 | 67 | 60 |
| hu119-21 | 68 | 60 |
| hu119-22 | 69 | 60 |
| hu119-23 | 64 | 61 |
| hu119-24 | 66 | 61 |
| hu119-25 | 67 | 61 |
| hu119-26 | 68 | 61 |
| hu119-27 | 69 | 61 |

The binding activity of the humanized antibodies against human TSLP was assayed by an ELISA method. The results showed that the humanized antibodies of mab119 could specifically bind to human TSLP.

### (2) Mutation of hu119

After the assay, it was found that there were hot spots on the DNS sequence of LCDR1 of the humanized antibodies of mab 119, and therefore, the corresponding positions were mutated to N31S or N31Q. The LCDR1 sequences obtained by the mutation are shown in Table 15:

**Table 15. LCDR1 of humanized antibodies of mab 119 after point mutation**

| | |
|---|---|
| hu119 LCDR1-N31S | RASESVDSSGLSFMH SEQ ID NO: 70 |
| hu119 LCDR1-N31Q | RASESVDQSGLSFMH SEQ ID NO: 71 |

| | |
|---|---|
| Note: the locations of the mutation positions in Table 15 are numbered in the natural order. Illustratively, the hu119VL2 and hu119VL6 mutant sequences obtained by the mutation are as follows: | |

hu119VL2-N31S (SEQ ID NO: 72) 
DIVLTQSPDSLAVSLGERATINCRASESVDSSGLSFMHWYQQKPGQPPKLLIYRASNLG
SGVPDRFSGSGSGTDFTLTISSLQAEDVAVYYCQQINTDPLTFGQGTKLEIK
hu119VL2-N31Q (SEQ ID NO: 73) 
DIVLTQSPDSLAVSLGERATINCRASESVDQSGLSFMHWYQQKPGQPPKLLIYRASNLG
SGVPDRFSGSGSGTDFTLTISSLQAEDVAVYYCQQINTDPLTFGQGTKLEIK
hu119VL6-N31S (SEQ ID NO: 74) 
DIVLTQSPATLSLSPGERATLSCRASESVDSSGLSFMHWYQQKPGQPPRLLLYRASNLGS
GIPARFSGSGSGTDFTLTISSLEPEDFAVYYCQQINTDPLTFGQGTKLEIK
hu119VL6-N31Q (SEQ ID NO: 75)

DIVLTQSPATLSLSPGERATLSCRASESVDQSGLSFMHWYQQKPGQPPRLLLYRASNLG
SGIPARFSGSGSGTDFTLTISSLEPEDFAVYYCQQINTDPLTFGQGTKLEIK

Note: the single underlined parts indicate the variable regions, and the double underlined parts indicate the back mutations.

The obtained hu119VL2 and hu119VL6 mutants were combined with hu119VH to obtain novel humanized hu119 antibodies. Illustratively, hu119VL2-N31S and hu119VL2-N31Q were each combined with hu119VH3 to obtain the antibodies hu119-28 and hu119-29; and hu119VL3-N31S was combined with hu119VH8 to obtain the antibody hu119-30. Exemplary combinations of the variable regions of the antibodies after the mutation are shown in Table 16:

**Table 16. Combinations of variable regions of humanized antibodies after hot spot mutation**

| | hu119VH | hu119VL |
|---|---|---|
| hu119-28 | hu119VH3 | hu119VL2-N31S |
| hu119-29 | hu119VH3 | hu119VL2-N31Q |
| hu119-30 | hu119VH8 | hu119VL6-N31S |

The affinity of the antibody obtained after the mutation for human TSLP was assayed by an ELISA method. The results showed that the antibodies hu119-28 and hu119-29 still had high affinity for human TSLP, suggesting that the N31S and N31Q mutations of LCDR2 do not affect the activity of the anti-TSLP antibodies.

In summary, the CDRs of the humanized antibodies of mab119 have the sequences shown in Table 17:

**Table 17. CDRs of humanized antibodies ofmab119**

| | | | |
|---|---|---|---|
| HCDR1 | TYNMH SEQ ID NO: 20 | LCDR1-g eneral formula | RASESVDX₆SGLSFMH SEQ ID NO: 76 |
| HCDR2 | AIYPGNGETSYNQKFKD SEQ ID NO: 21 | LCDR2 | RASNLGS SEQ ID NO: 24 |
| HCDR3 | EDDYGEGYFDV SEQ ID NO: 22 | LCDR3 | QQINTDPLT SEQ ID NO: 25 |

X₆ is selected from the group consisting of N, S, and Q.

### 5.3 Selection and back mutation of humanized FR regions of mab179

### (1) humanization template selection and back mutation of murine antibody of mab 179

For mab179, IGHV1-69*02 + IGHJ6*01 was taken as the template for the VH, and IGKV4-1*01 + IGKJ2*01 or IGKV2-29*02 + IGKJ2*01 was taken as the template for VL. The CDR regions of the murine antibody were grafted onto the selected humanization templates, and the FR regions were back mutated to obtain light and heavy chain variable regions with different sequences, with the results shown in Table 18. The humanized variable region sequences and back mutations are as follows:
hu179VL1 (Grafted (IGKV4-1^{∗}01)) (SEQ ID NO: 77) DIVMTQSPDSLAVSLGERATINCKASQSVSSDVTWYQQKPGQPPKLLIYYVSNHYTGV PDRFSGSGSGTDFTLTISSLQAEDVAVYYCQQHHRFPLTFGQGTKLEIK
hu179VL5 (Grafted (IGKV2-29*02)) (SEQ ID NO: 81) 
DIVMTQTPLSLSVTPGQPASISCKASQSVSSDVTWYLQKPGQSPQLLIYYVSNHYTGVP
DRFSGSGSGTDFTLKISRVEAEDVGVYYCQQHHRFPLTFGQGTKLEIK
hu179VH1 (Grafted) (SEQ ID NO: 85)

EVQLVQSGAEVKKPGSSVKVSCKASGGTFSNYLIEWVRQAPGQGLEWMGVIDPGNGD
TNYNENFKGRVTITADKSTSTAYMELSSLRSEDTAVYYCAREDNTGTAFDYWGQGTTV
TVSS

**Table 18. Humanization templates and back mutations of mab 179**

| **hu179VL1VL** | | **hu179VH** | |
|---|---|---|---|
| hu179VL1 | Grafted (IGKV4-1*01) | hu179VH1 | Grafted (IGHV1-69*02) |
| hu179VL2 | Grafted (IGKV4-1*01) P43S | hu179VH2 | G27Y, I69L |
| hu179VL3 | Grafted (IGKV4-1*01) P43S, L73F | hu179VH3 | G27Y, M48I, V67A, I69L, M80I |
| hu179VL4 | Grafted (IGKV4-1*01) D1S, P43S | hu179VH4 | G27Y, R38K, M48I, R66K, V67A, I69L, M80I, S82bR |
| hu179VL5 | Grafted (IGKV2-29*02) | hu179VH5 | G27Y, T28A, M48I, V67A, I69L |
| hu179VL6 | Grafted (IGKV2-29*02) D1S | | |
| hu179VL7 | Grafted (IGKV2-29*02) D1S, L73F | | |
| hu179VL8 | Grafted (IGKV2-29*02) D1S, S67Y | | |

| | | | |
|---|---|---|---|
| Note: for example, P43S denotes that P at position 43 is back mutated to S according to the Kabat numbering scheme. Grafted denotes that the murine antibody CDRs are grafted onto the human species FR region sequences. | | | |

The variable regions of the humanized antibodies of mab 179 are shown below:
hu179VL1 (Grafted (IGKV4-1*01)) (SEQ ID NO: 77): 
DIVMTQSPDSLAVSLGERATINCKASQSVSSDVTWYQQKPGQPPKLLIYYVSNHYTGV
PDRFSGSGSGTDFTLTISSLQAEDVAVYYCQQHHRFPLTFGQGTKLEIK
hu179VL2 (SEQ ID NO: 78): 
DIVMTQSPDSLAVSLGERATINCKASQSVSSDVTWYQQKPGQSPKLLIYYVSNHYTGV
PDRFSGSGSGTDFTLTISSLQAEDVAVYYCQQHHRFPLTFGQGTKLEIK
hu179 VL3 (SEQ ID NO: 79): 
DIVMTQSPDSLAVSLGERATINCKASQSVSSDVTWYQQKPGQSPKLLIYYVSNHYTGV
PDRFSGSGSGTDFTFTISSLQAEDVAVYYCQQHHRFPLTFGQGTKLEIK
hu179 VL4 (SEQ ID NO: 80): 
SIVMTQSPDSLAVSLGERATINCKASQSVSSDVTWYQQKPGQSPKLLIYYVSNHYTGVP
DRFSGSGSGTDFTLTISSLQAEDVAVYYCQQHHRFPLTFGQGTKLEIK
hu179VL5 (Grafted (IGKV2-29*02)) (SEQ ID NO: 81): 
DIVMTQTPLSLSVTPGQPASISCKASQSVSSDVTWYLQKPGQSPQLLIYYVSNHYTGVP
DRFSGSGSGTDFTLKISRVEAEDVGVYYCQQHHRFPLTFGQGTKLEIK
hu179VL6 (SEQ ID NO: 82): 
SIVMTQTPLSLSVTPGQPASISCKASQSVSSDVTWYLQKPGQSPQLLIYYVSNHYTGVP
DRFSGSGSGTDFTLKISRVEAEDVGVYYCQQHHRFPLTFGQGTKLEIK
hu179VL7 (SEQ ID NO: 83): 
SIVMTQTPLSLSVTPGQPASISCKASQSVSSDVTWYLQKPGQSPQLLIYYVSNHYTGVP
DRFSGSGSGTDFTFKISRVEAEDVGVYYCQQHHRFPLTFGQGTKLEIK
hu179VL8 (SEQ ID NO: 84): 
SIVMTQTPLSLSVTPGQPASISCKASQSVSSDVTWYLQKPGQSPQLLIYYVSNHYTGVP
DRFSGSGYGTDFTLKISRVEAEDVGVYYCQQHHRFPLTFGQGTKLEIK
hu179VH1 (Grafted) (SEQ ID NO: 85): 
EVQLVQSGAEVKKPGSSVKVSCKASGGTFSNYLIEWVRQAPGQGLEWMGVIDPGNGD
TNYNENFKGRVTITADKSTSTAYMELSSLRSEDTAVYYCAREDNTGTAFDYWGQGTTV
TVSS
hu179VH2 (SEQ ID NO: 86): EVQLVQSGAEVKKPGSSVKVSCKASGYTFSNYLIEWVRQAPGQGLEWMGVIDPGNGD 
TNYNENFKGRVTLTADKSTSTAYMELSSLRSEDTAVYYCAREDNTGTAFDYWGQGTT
VTVSS
hu179VH3 (SEQ ID NO: 87): 
EVQLVQSGAEVKKPGSSVKVSCKASGYTFSNYLIEWVRQAPGQGLEWIGVIDPGNGD
TNYNENFKGRATLTADKSTSTAYIELSSLRSEDTAVYYCAREDNTGTAFDYWGQGTTV
TVSS
hu179VH4 (SEQ ID NO: 88): 
EVQLVQSGAEVKKPGSSVKVSCKASGYTFSNYLIEWVKQAPGQGLEWIGVIDPGNGD
TNYNENFKGKATLTADKSTSTAYIELSRLRSEDTAVYYCAREDNTGTAFDYWGQGTTV
TVSS
hu179VH5 (SEQ ID NO: 89):

EVQLVQSGAEVKKPGSSVKVSCKASGYAFSNYLIEWVRQAPGQGLEWIGVIDPGNGD
TNYNENFKGRATLTADKSTSTAYMELSSLRSEDTAVYYCAREDNTGTAFDYWGQGTTV
TVSS

Note: the single underlined parts indicate the CDRs, and the double underlined parts indicate the back mutation positions.

The light and heavy chain variable regions described above were combined with the human species light and heavy chain constant region sequences, respectively, to form final complete light and heavy chain sequences, and then the antibodies with the full-length sequences were obtained. Illustratively, the heavy chain constant region of the humanized antibodies of mab 199 of the present disclosure was the IgG1-YTE constant region set forth in SEQ ID NO: 133, the light chain constant region was the kappa chain constant region set forth in SEQ ID NO: 134, and they may be replaced by other constant regions known in the art.

The sequences of the heavy and light chain variable regions of the humanized antibodies of mab 179 obtained are shown in Table 19 below:

**Table 19. Combinations of heavy and light chain variable regions of humanized antibodies of mab 179**

| Antibody | VH (SEQ ID NO) | VL (SEQ ID NO) |
|---|---|---|
| hu179-01 | 85 | 77 |
| hu179-02 | 85 | 78 |
| hu179-03 | 86 | 77 |
| hu179-04 | 86 | 78 |
| hu179-05 | 87 | 77 |
| hu179-06 | 87 | 78 |
| hu179-07 | 87 | 79 |
| hu179-08 | 87 | 81 |
| hu179-09 | 87 | 82 |
| hu179-10 | 87 | 83 |
| hu179-11 | 87 | 84 |
| hu179-12 | 88 | 77 |
| hu179-13 | 88 | 78 |
| hu179-14 | 89 | 79 |
| hu179-15 | 89 | 80 |
| hu179-16 | 89 | 81 |
| hu179-17 | 89 | 82 |
| hu179-18 | 89 | 83 |
| hu179-19 | 89 | 84 |

The affinity of the humanized antibodies of mab179 for human TSLP was assayed by an ELISA method. The results showed that the humanized antibodies of mab 179 had good affinity for human TSLP.

### (2) Mutation of hu179 antibody

After the assay, it was found that there were hot spots on the HCDR2 and LCDR2 sequences of the humanized antibodies of mab179, and therefore, the corresponding positions were mutated to eliminate the risk of molecular modification.

In one example, amino acid mutations were made to GNG of HCDR2 of hu179VH1. The sequences of hu179VH1 after the mutation are as follows:
hu179VH1-NSSQ (SEQ ID NO: 90): 
EVQLVQSGAEVKKPGSSVKVSCKASGGTFSNYLIEWVRQAPGQGLEWMGVIDPGQGD
TNYNENFKGRVTITADKSTSTAYMELSSLRSEDTAVYYCAREDNTGTAFDYWGQGTTV
TVSS
hu179VH1-NSSV (SEQ ID NO: 91): 
EVQLVQSGAEVKKPGSSVKVSCKASGGTFSNYLIEWVRQAPGQGLEWMGVIDPGVGD
TNYNENFKGRVTITADKSTSTAYMELSSLRSEDTAVYYCAREDNTGTAFDYWGQGTTV
TVSS
hu179VH1-G56V (SEQ ID NO: 92):

EVQLVQSGAEVKKPGSSVKVSCKASGGTFSNYLIEWVRQAPGQGLEWMGVIDPGNVD
TNYNENFKGRVTITADKSTSTAYMELSSLRSEDTAVYYCAREDNTGTAFDYWGQGTTV
TVSS

Note: the single underlined parts indicate the CDRs, and the double underlined parts indicate the back mutation positions.

The HCDR2 sequences of the humanized antibodies of mab179 obtained by the mutation are shown in Table 20:

**Table 20. HCDR2 mutants of humanized antibodies of mab 179**

| | |
|---|---|
| hu179 HCDR2-N55Q | VIDPGQGDTNYNENFKG SEQ ID NO: 93 |
| hu179 HCDR2-N55V | VIDPGVGDTNYNENFKG SEQ ID NO: 94 |
| hu179 HCDR2-G56V | VIDPGNVDTNYNENFKG SEQ ID NO: 95 |

Note: the locations of the mutation positions in Table 20 are numbered in the natural order. The CDR regions of the humanized antibodies of mab179 after the mutation can be obtained from above. The results are shown in Table 21:

**Table 21. CDRs of humanized antibodies of mab 179 after mutation**

| | | | |
|---|---|---|---|
| HCDR1 | NYLIE SEQ ID NO: 26 | LCDR1 | KASQSVSSDVT SEQ ID NO: 29 |
| HCDR2 (general formula) | VIDPGX₇X₈DTNYNENFKG SEQ ID NO: 96 | LCDR2 | YVSNHYT SEQ ID NO: 30 |
| HCDR3 | EDNTGTAFDY SEQ ID NO: 28 | LCDR3 | QQHHRFPLT SEQ ID NO: 31 |

X₇ is selected from the group consisting of N, Q, and V, and Xs is selected from the group consisting of G and V

The hu179VH1 mutants obtained by the mutation were combined with humanized hu179VL to obtain novel humanized antibodies of mab179. Exemplary antibodies obtained by combining the hu179VH1 mutants with hu179VL2 are shown in Table 22:

**Table 22. Combination of antibody variable regions after the mutation**

| Variable region | hu179VH1-N55Q | hu179VH1-NSSV | hu179VH1-G56V |
|---|---|---|---|
| hu179VL2 | hu 179-20 | hu179-21 | hu 179-22 |

The affinity of the antibodies obtained after the mutation for human TSLP was assayed by an ELISA method. The result showed that the antibodies obtained after the HCDR2 mutation still maintained high affinity for human TSLP. This suggests that the point mutations N55Q, N55V, and G56V of HCDR2 of the humanized antibodies of mab179 do not substantially affect the affinity activity of the antibody for TSLP.

According to the same method, the point mutations N55Q, N55V, and G56V (numbered in the natural order) were made on each of hu179VH2, hu179VH3, hu179VH4, and hu179VH5, and the heavy and light chain variable regions obtained by the mutations were recombined to obtain novel humanized antibodies of mab179. Illustratively, the sequence of the hu179VH3 after the mutation is shown below:
hu179VH3-NSSV (SEQ ID NO: 97):

EVQLVQSGAEVKKPGSSVKVSCKASGYTFSNYLIEWVRQAPGQGLEWIGVIDPGVGD
TNYNENFKGRATLTADKSTSTAYIELSSLRSEDTAVYYCAREDNTGTAFDYWGQGTTV
TVSS

Note: the single underlined parts indicate the CDRs, and the double underlined parts indicate the back mutation positions.

In some other examples, amino acid mutations were made to LCDR2 of the humanized antibodies of mab179. Illustratively, the sequences of hu179VL2 after the mutation are as follows:
hu179VL2-Y50E (SEQ ID NO: 98): 
DIVMTQSPDSLAVSLGERATINCKASQSVSSDVTWYQQKPGQSPKLLIYEVSNHYTGVP
DRFSGSGSGTDFTLTISSLQAEDVAVYYCQQHHRFPLTFGQGTKLEIK
hu179VL2-S52D (SEQ ID NO: 99): 
DIVMTQSPDSLAVSLGERATINCKASQSVSSDVTWYQQKPGQSPKLLIYYVDNHYTGV
PDRFSGSGSGTDFTLTISSLQAEDVAVYYCQQHHRFPLTFGQGTKLEIK
hu179VL2-S52E (SEQ ID NO: 100): 
DIVMTQSPDSLAVSLGERATINCKASQSVSSDVTWYQQKPGQSPKLLIYYVENHYTGV
PDRFSGSGSGTDFTLTISSLQAEDVAVYYCQQHHRFPLTFGQGTKLEIK
hu179VL2-N53Q (SEQ ID NO: 101): 
DIVMTQSPDSLAVSLGERATINCKASQSVSSDVTWYQQKPGQSPKLLIYYVSQHYTGV
PDRFSGSGSGTDFTLTISSLQAEDVAVYYCQQHHRFPLTFGQGTKLEIK
hu179VL2-N53D (SEQ ID NO: 102): 
DIVMTQSPDSLAVSLGERATINCKASQSVSSDVTWYQQKPGQSPKLLIYYVSDHYTGV
PDRFSGSGSGTDFTLTISSLQAEDVAVYYCQQHHRFPLTFGQGTKLEIK
hu179VL2-N53E (SEQ ID NO: 103): 
DIVMTQSPDSLAVSLGERATINCKASQSVSSDVTWYQQKPGQSPKLLIYYVSEHYTGVP
DRFSGSGSGTDFTLTISSLQAEDVAVYYCQQHHRFPLTFGQGTKLEIK
hu179VL2-H54Y (SEQ ID NO: 104): 
DIVMTQSPDSLAVSLGERATINCKASQSVSSDVTWYQQKPGQSPKLLIYYVSNYYTGV
PDRFSGSGSGTDFTLTISSLQAEDVAVYYCQQHHRFPLTFGQGTKLEIK
hu179VL2-H54D (SEQ ID NO: 105): 
DIVMTQSPDSLAVSLGERATINCKASQSVSSDVTWYQQKPGQSPKLLIYYVSNDYTGV
PDRFSGSGSGTDFTLTISSLQAEDVAVYYCQQHHRFPLTFGQGTKLEIK
hu179VL2-H54E (SEQ ID NO: 106): 
DIVMTQSPDSLAVSLGERATINCKASQSVSSDVTWYQQKPGQSPKLLIYYVSNEYTGVP
DRFSGSGSGTDFTLTISSLQAEDVAVYYCQQHHRFPLTFGQGTKLEIK
hu179VL2-Y55E (SEQ ID NO: 107):

DIVMTQSPDSLAVSLGERATINCKASQSVSSDVTWYQQKPGQSPKLLIYYVSNHETGVP
DRFSGSGSGTDFTLTISSLQAEDVAVYYCQQHHRFPLTFGQGTKLEIK

Note: the single underlined parts indicate the CDRs, and the double underlined parts indicate the back mutation positions.

The LCDR2 sequences of the humanized antibodies of mab179 obtained by the mutation are shown in Table 23:

**Table 23. LCDR2 mutants of the humanized antibodies of mab 179**

| Mutant | Sequence |
|---|---|
| hu179 LCDR2-Y50E | EVSNHYT SEQ ID NO: 108 |
| hu179 LCDR2-S52D | YVDNHYT SEQ ID NO: 109 |
| hu179 LCDR2-S52E | YVENHYT SEQ ID NO: 110 |
| hu179LCDR2-N53Q | YVSQHYT SEQ ID NO: 111 |
| hu179 LCDR2-N53D | YVSDHYT SEQ ID NO: 112 |
| hu179 LCDR2-N53E | YVSEHYT SEQ ID NO: 113 |
| hu179 LCDR2-H54Y | YVSNYYT SEQ ID NO: 114 |
| hu179 LCDR2-H54D | YVSNDYT SEQ ID NO: 115 |
| hu179 LCDR2-H54E | YVSNEYT SEQ ID NO: 116 |
| hu179 LCDR2-Y55E | YVSNHET SEQ ID NO: 117 |

| | |
|---|---|
| Note: the double underlined parts indicate the back mutation positions. | |

It can be seen from the above that the LCDR2 of the humanized antibodies of mab179 has the general formula of X₉VX₁₀X₁₁X₁₂X₁₃T (SEQ ID NO: 118), wherein X₉ is selected from the group consisting of Y and E; X₁₀ is selected from the group consisting of S, D, and E; X₁₁ is selected from the group consisting of N, Q, D, and E; X₁₂ is selected from the group consisting of H, Y, D, and E; and X₁₃ is selected from the group consisting of E and Y The CDR regions of the humanized antibodies of mab179 are shown in Table 24 below:

**Table 24. CDRs of humanized antibodies of mab 179**

| | | | |
|---|---|---|---|
| HCDR1 | NYLIE SEQ ID NO: 26 | LCDR1 | KASQSVSSDVT SEQ ID NO: 29 |
| HCDR2 | VIDPGX₇X₈DTNYNENFKG SEQ ID NO: 96 | LCDR2 | X₉VX₁₀X₁₁X₁₂X₁₃T SEQ ID NO: 118 |
| HCDR3 | EDNTGTAFDY SEQ ID NO: 28 | LCDR3 | QQHHRFPLT SEQ ID NO: 31 |

X₇ is selected from the group consisting of N, Q, and V; X₈ is selected from the group consisting of G and V; X₉ is selected from the group consisting of Y and E; X₁₀ is selected from the group consisting of S, D, and E; X₁₁ is selected from the group consisting of N, Q, D, and E; X₁₂ is selected from the group consisting of H, Y, D, and E; and X₁₃ is selected from the group consisting of E and Y

The hu179VL2 mutants obtained by the mutation were combined with the humanized hu179 heavy chain variable regions to obtain novel humanized antibodies of mab179. Exemplary hu179VL2 mutants were combined with hu179VH1 and hu179VH3. The CDRs and combinations of the heavy and light chain variable regions of the obtained humanized antibodies of mab 179 are shown in Table 25 below:

**Table 25. CDR sequences of humanized antibodies of mab 179 after LCDR2 mutation**

| | | | |
|---|---|---|---|
| HCDR1 | NYLIE SEQ ID NO: 26 | LCDR1 | KASQSVSSDVT SEQ ID NO: 29 |
| HCDR2 | VIDPGNGDTNYNENFKG SEQ ID NO: 27 | LCDR2 | X₅VX₆X₇X₈X₉T SEQ ID NO: 118 |
| HCDR3 | EDNTGTAFDY SEQ ID NO: 28 | LCDR3 | QQHHRFPLT SEQ ID NO: 31 |

X₅ is selected from the group consisting of Y and E; X₆ is selected from the group consisting of S, D, and E; X₇ is selected from the group consisting of N, Q, D, and E; X₈ is selected from the group consisting of H, Y, D, and E; and X₉ is selected from the group consisting of E and Y The sequences of the heavy and light chain variable regions of the humanized antibodies of mab179 obtained are shown in Table 26 below:

**Table 26. Combinations of heavy and light chain variable regions of humanized antibodies of mab 179 after LCDR2 mutation**

| Antibody | VH (SEQ ID NO) | VL (SEQ ID NO) |
|---|---|---|
| hu179-23 | 85 | 102 |
| hu179-24 | 85 | 104 |
| hu179-25 | 87 | 98 |
| hu179-26 | 87 | 99 |
| hu179-27 | 87 | 100 |
| hu179-28 | 87 | 101 |
| hu179-29 | 87 | 103 |
| hu179-30 | 87 | 105 |
| hu179-31 | 87 | 106 |
| hu179-32 | 87 | 107 |

The affinity of the humanized antibodies of mab179 obtained after the LCDR2 mutation for human TSLP was assayed by an ELISA method. The results showed that the antibodies obtained after the mutation of hot spot positions of LCDR2 still had good affinity for human TSLP. This suggests that the mutation of hot spot positions of LCDR2 also does not affect the binding activity of the humanized antibodies of mab 179.

According to the same method, the mutation N53Q, N53D, N53S, H54Y, Y50E, S52D, S52E, N53E, H54D, H54E or Y55E was made to LCDR2 of hu179VL3, hu179VL4, hu179VL5, hu179VL6, hu179VL7, and hu179VL8. The light chain variable regions after the mutation were combined with the heavy chain variable regions to form novel mab humanized antibodies. In one embodiment, the sequence of hu179VL8 after the mutation is shown below:
hu179VL8-N53E (SEQ ID NO: 119):

SIVMTQTPLSLSVTPGQPASISCKASQSVSSDVTWYLQKPGQSPQLLIYYVSEHYTGVP
DRFSGSGYGTDFTLKISRVEAEDVGVYYCQQHHRFPLTFGQGTKLEIK

The hu179VL8-N53E obtained by the mutation was combined with hu179VH3-NSSV to obtain the novel antibody molecule hu179-33. The CDR sequences of the molecule are shown in Table 27 below:

**Table 27. CDR regions of antibody hu179-33**

| | | | |
|---|---|---|---|
| HCDR1 | NYLIE SEQ ID NO: 26 | LCDR1 | KASQSVSSDVT SEQ ID NO: 29 |
| HCDR2-N55 V | VIDPGVGDTNYNENFKG SEQ ID NO: 94 | LCDR2-N5 3E | YVSEHYT SEQ ID NO: 113 |
| HCDR3 | EDNTGTAFDY SEQ ID NO: 28 | LCDR3 | QQHHRFPLT SEQ ID NO: 31 |

The binding activity of the antibodies obtained after the mutation against human TSLP was assayed by Biacore. The binding activity of exemplary antibodies is shown in Table 28 below:

**Table 28. Affinity of hu179-33 for human TSLP**

| Antibody | Affinity KD (M) for huTSLP |
|---|---|
| AMG157 | 8.12E-12 |
| hu179-33 | 9.03E-13 |

In the table, AMG157 (tezepelumab, anti-TSLP antibody) was a positive control. The results showed that the hu179-33 antibody had high specific binding activity against human TSLP. This suggests that the point mutations of hot spot positions on both HCDR2 and LCDR2 does not affect the affinity of the humanized antibodies of mab 179 for human TSLP. Thus, it can be seen that in the humanized antibody molecules of mab179, the mutation N55Q, N55V or G56V made on HCDR2 and the mutation N53Q, N53D, N53S, H54Y, Y50E, S52D, S52E, N53E, H54D, H54E or Y55E made in LCDR2 do not affect the binding of the antibodies to human TSLP, i.e., do not affect the activity of the anti-TSLP antibodies.

### 5.4 Selection and back mutation of human FR regions of antibody mab199

For mab199, IGHV1-46*01 + HJ6*01 was taken as the template for the VH, and IGKV1-39*01 + IGKJ4*01 was taken as the template for the VL. The CDR regions of the murine antibody were grafted onto the selected humanization templates, and the FR regions were back mutated to obtain light and heavy chain variable regions with different sequences. The back mutations are shown in Table 29.

**Table 29. Back mutation design for mab199**

| **hu199VL** | | **hu199VH** | |
|---|---|---|---|
| hu199VL1 | Grafted | hu199VH1 | Grafted |
| hu199VL2 | I48V | hu199VH2 | R71V, T73K, V78A |
| hu199VL3 | A43S, K45Q, I48V, D70Q | hu199VH3 | M69L, R71V, T73K, V78A |
| hu199VL4 | G66V | hu199VH4 | M48I, V67A, M69L, R71V, T73K, V78A |
| hu199VL5 | I48V, G66V | hu199VH5 | R38K, M48I, R66K, V67A, M69L, R71V, T73K, V78A |
| hu199VL6 | A43S, K45Q, I48V, G66V, D70Q | hu199VH6 | R38K, R66K, R71V, T73K, V78A |
| | | hu199VH7 | R38K, R67K, M69L, R71V, T73K, V78A |

| | | | |
|---|---|---|---|
| Note: for example, I48V denotes that I at position 48 is back mutated to V according to the Kabat numbering scheme. Grafted denotes that the murine antibody CDRs are grafted onto the human species FR region sequences. | | | |

The variable regions of the humanized antibodies of mab 199 are shown below:
hu199VL1 (Grafted) (SEQ ID NO: 120): 
DIQMTQSPSSLSASVGDRVTITCRASENIYSYLAWYQQKPGKAPKLLIYFAKTLAEGVP
SRFSGSGSGTDFTLTISSLQPEDFATYYCQHHYGTPWTFGGGTKVEIK
hu199VL2 (SEQ ID NO: 121): 
DIQMTQSPSSLSASVGDRVTITCRASENIYSYLAWYQQKPGKAPKLLVYFAKTLAEGVP
SRFSGSGSGTDFTLTISSLQPEDFATYYCQHHYGTPWTFGGGTKVEIK
hu199VL3 (SEQ ID NO: 122): 
DIQMTQSPSSLSASVGDRVTITCRASENIYSYLAWYQQKPGKSPQLLVYFAKTLAEGVP
SRFSGSGSGTQFTLTISSLQPEDFATYYCQHHYGTPWTFGGGTKVEIK
hu199VL4 (SEQ ID NO: 123): 
DIQMTQSPSSLSASVGDRVTITCRASENIYSYLAWYQQKPGKAPKLLIYFAKTLAEGVP
SRFSGSVSGTDFTLTISSLQPEDFATYYCQHHYGTPWTFGGGTKVEIK
hu199VL5 (SEQ ID NO: 124): 
DIQMTQSPSSLSASVGDRVTITCRASENIYSYLAWYQQKPGKAPKLLVYFAKTLAEGVP
SRFSGSVSGTDFTLTISSLQPEDFATYYCQHHYGTPWTFGGGTKVEIK
hu199VL6 (SEQ ID NO: 125): 
DIQMTQSPSSLSASVGDRVTITCRASENIYSYLAWYQQKPGKSPQLLVYFAKTLAEGVP
SRFSGSVSGTQFTLTISSLQPEDFATYYCQHHYGTPWTFGGGTKVEIK
hu199VH1 (Grafted) (SEQ ID NO: 126): 
EVQLVQSGAEVKKPGASVKVSCKASGYTFTTYWMHWVRQAPGQGLEWMGMIDPSD
SETTLIQKFKDRVTMTRDTSTSTVYMELSSLRSEDTAVYYCARTLDGYYDYWGQGTTV
TVSS
hu199VH2 (SEQ ID NO: 127): 
EVQLVQSGAEVKKPGASVKVSCKASGYTFTTYWMHWVRQAPGQGLEWMGMIDPSD
SETTLIQKFKDRVTMTVDKSTSTAYMELSSLRSEDTAVYYCARTLDGYYDYWGQGTTV
TVSS
hu199VH3 (SEQ ID NO: 128): 
EVQLVQSGAEVKKPGASVKVSCKASGYTFTTYWMHWVRQAPGQGLEWMGMIDPSD
SETTLIQKFKDRVTLTVDKSTSTAYMELSSLRSEDTAVYYCARTLDGYYDYWGQGTTV
TVSS
hu199VH4 (SEQ ID NO: 129): 
EVQLVQSGAEVKKPGASVKVSCKASGYTFTTYWMHWVRQAPGQGLEWIGMIDPSDS
ETTLIQKFKDRATLTVDKSTSTAYMELSSLRSEDTAVYYCARTLDGYYDYWGQGTTVT
VSS
hu199VH5 (SEQ ID NO: 130): 
EVQLVQSGAEVKKPGASVKVSCKASGYTFTTYWMHWVKQAPGQGLEWIGMIDPSDS
ETTLIQKFKDKATLTVDKSTSTAYMELSSLRSEDTAVYYCARTLDGYYDYWGQGTTVT
VSS
hu199VH6 (SEQ ID NO: 131): 
EVQLVQSGAEVKKPGASVKVSCKASGYTFTTYWMHWVKQAPGQGLEWMGMIDPSD
SETTLIQKFKDKVTMTVDKSTSTAYMELSSLRSEDTAVYYCARTLDGYYDYWGQGTT
 VTVSS
hu199VH7 (SEQ ID NO: 132):

EVQLVQSGAEVKKPGASVKVSCKASGYTFTTYWMHWVKQAPGQGLEWMGMIDPSD
SETTLIQKFKDKVTLTVDKSTSTAYMELSSLRSEDTAVYYCARTLDGYYDYWGQGTTV
TVSS

Note: the single underlined parts indicate the CDRs, and the double underlined parts indicate the back mutation positions.

The light and heavy chain variable regions described above were combined with the human species light and heavy chain constant region sequences, respectively, to form final complete light and heavy chain sequences, and then the antibodies with the full-length sequences were obtained. In the present disclosure, unless otherwise explicitly stated, the light chain constant region of humanized antibodies of mab199 is a constant region set forth in SEQ ID NO: 134, and the heavy chain constant region is a constant region set forth in SEQ ID NO: 133.

The obtained humanized antibodies of mab199 are shown in Table 30 below:

**Table 30. Sequences of heavy and light chain variable regions of humanized antibodies of mab 199**

| Antibody | VH (SEQ ID NO) | VL (SEQ ID NO) |
|---|---|---|
| hu199-01 | 127 | 120 |
| hu199-02 | 127 | 121 |
| hu199-03 | 127 | 122 |
| hu199-04 | 127 | 123 |
| hu199-05 | 127 | 124 |
| hu199-06 | 127 | 125 |
| hu199-07 | 128 | 120 |
| hu199-08 | 128 | 121 |
| hu199-09 | 128 | 122 |
| hu199-10 | 128 | 123 |
| hu199-11 | 128 | 124 |
| hu199-12 | 128 | 125 |
| hu199-13 | 129 | 120 |
| hu199-14 | 129 | 121 |
| hu199-15 | 129 | 122 |
| hu199-16 | 129 | 123 |
| hu199-17 | 129 | 124 |
| hu199-18 | 129 | 125 |
| hu199-19 | 130 | 120 |
| hu199-20 | 130 | 121 |
| hu199-21 | 130 | 122 |
| hu199-22 | 130 | 123 |
| hu199-23 | 130 | 124 |
| hu199-24 | 130 | 125 |
| hu199-25 | 131 | 120 |
| hu199-26 | 131 | 121 |
| hu199-27 | 131 | 122 |
| hu199-28 | 131 | 123 |
| hu199-29 | 131 | 124 |
| hu199-30 | 131 | 125 |
| hu199-31 | 132 | 120 |
| hu199-32 | 132 | 121 |
| hu199-33 | 132 | 122 |
| hu199-34 | 132 | 123 |
| hu199-35 | 132 | 124 |
| hu199-36 | 132 | 125 |

The blocking activity of the humanized antibodies of mab 199 blocking the binding of TSLP to the TSLP receptor was assayed by an ELISA method. The assay results are shown in Table 31 below:

**Table 31. Blocking activity of humanized antibodies of mab 199 against binding of TSLP to TSLP receptor**

| Antibody | IC50 (nM) | Antibody | IC50 (nM) | Antibody | IC50 (nM) | Antibody | IC50 (nM) |
|---|---|---|---|---|---|---|---|
| hu199-01 | 0.1912 | hu199-10 | 0.3910 | hu199-19 1 | 0.6584 | hu199-28 | 0.4619 |
| hu 199-02 | 0.2193 | hu199-11 | 0.3648 | hu199-20 | 0.4001 | hu 199-29 | 0.5543 |
| hu199-03 | 0.2077 | hu199-12 | 0.3700 | hu 199-21 | 0.5353 | hu 199-30 | 0.3493 |
| hu199-04 | 0.4242 | hu199-13 | 0.2395 | hu 199-22 | 0.3449 | hu 199-31 | 0.3044 |
| hu199-05 | 0.4726 | hu199-14 | 0.3112 | hu199-23 | 0.3370 | hu199-32 | 0.2870 |
| hu 199-06 | 0.3806 | hu199-15 | 0.2866 | hu 199-24 | 0.4960 | hu 199-33 | 0.2055 |
| hu 199-07 | 0.2834 | hu199-16 | 0.7367 | hu 199-25 | 0.2460 | hu199-34 | 0.7107 |
| hu 199-08 | 0.2828 | hu199-17 | 0.6111 | hu 199-26 | 0.3651 | hu199-3 5 | 0.4849 |
| hu 199-09 | 0.2732 | hu199-18 | 0.4806 | hu 199-27 | 0.3544 | hu199-3 6 | 0.7273 |
| Ch199 | 0.4266 | | | | | | |

The results showed that the humanized antibodies of mab199 still had high blocking activity against the binding of human TSLP to the TSLP receptor.

### 5.5 Antibody constant regions

The heavy chain constant region of humanized and chimeric antibodies can be selected from the group consisting of constant regions of IgG1, IgG2, IgG4 and variants thereof. Illustratively, the constant region IgG1-YTE was used in the present disclosure, which has a sequence set forth in SEQ ID NO: 133. The light chain constant region can be selected from the group consisting of light chain constant regions of human κ and λ chains or variants thereof. Illustratively, the constant region of human κ chain was used in the present disclosure, which has a sequence set forth in SEQ ID NO: 134.
IgG1-YTE heavy chain constant region (SEQ ID NO: 133): 
ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPELL
GGPSVFLFPPKPKDTLYITREPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTL
 
PPSRDELTKNQVSLTCLVKGFYPSDIAVEWESNGQPENNYKTTPPVLDSDGSFFLYSKLT
VDKSRWQQGNVFSCSVMHEALHNHYTQKSLSLSPGK
 Note: the underlined parts indicate the designed M252Y, S254T, and T256E mutations.
κ light chain constant region (SEQ ID NO: 134):

The humanized heavy and light chain variable regions of the present disclosure were recombined with the constant regions to obtain full-length sequences of the heavy and light chains. Illustratively, the antibody sequences are shown below:
hu3-13 antibody heavy chain (SEQ ID NO: 135): 
EVQLVQSGAEVKKPGASVKVSCKASGYTFTDDYMNWVRQAPGQRLEWMGIISPYNG
GTSYNQKFKGRVTLTVDKSASTAYMELSSLRSEDTAVYYCAREDYDYDGYAMDYWG
QGTTVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCPAPE
LLGGPSVFLFPPKPKDTLYITREPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSRWQ
QGNVFSCSVMHEALHNHYTQKSLSLSPGK
hu3-13 antibody light chain (SEQ ID NO: 136): 
EIVLTQSPATLSLSPGERATLSCRASSSVSYMHWYQQKPGQAPRPWIYATSNLASGVPA
RFSGSGSGTSYTLTISRLEPEDFAVYYCQQSDSGREFGGGTKVEIKRTVAAPSVFIFPPSDE
QLKSGTASVVCLLNNFYPREAKVQWKVDNALQSGNSQESVTEQDSKDSTYSLSSTLTLSKAD
YEKHKVYACEVTHQGLSSPVTKSFNRGEC
hu119-30 antibody heavy chain (SEQ ID NO: 137): 
EAQLVQSGAEVKKPGSSVKVSCKASGFTFSTYNMHWVRQAPGQGLEWIGAIYPGNGE
TSYNQKFKDRATLTVDKSTRTAYMELSSLRSEDTAVYYCAREDDYGEGYFDVWGQGT
TVTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLGG
PSVFLFPPKPKDTLYITREPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQGN
 VFSCSVMHEALHNHYTQKSLSLSPGK
hu119-30 antibody light chain (SEQ ID NO: 138): 
DIVLTQSPATLSLSPGERATLSCRASESVDSSGLSFMHWYQQKPGQPPRLLLYRASNLGS
GIPARFSGSGSGTDFTLTISSLEPEDFAVYYCQQINTDPLTFGQGTKLEIKRTVAAPSVFIFP
PSDEQLKSGTASVVCLLNNFYPREAKVQWKVDNALQSGNSQESVTEQDSKDSTYSLSSTLTLS
KADYEKHKVYACEVTHQGLSSPVTKSFNRGEC
hu179-33 antibody heavy chain (SEQ ID NO: 139): 
EVQLVQSGAEVKKPGSSVKVSCKASGYTFSNYLIEWVRQAPGQGLEWIGVIDPGVGD
TNYNENFKGRATLTADKSTSTAYIELSSLRSEDTAVYYCAREDNTGTAFDYWGQGTTV
TVSSASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPELLGGPS
VFLFPPKPKDTLYITREPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFS
CSVMHEALHNHYTQKSLSLSPGK
hu179-33 antibody light chain (SEQ ID NO: 140): 
SIVMTQTPLSLSVTPGQPASISCKASQSVSSDVTWYLQKPGQSPQLLIYYVSEHYTGVP
DRFSGSGYGTDFTLKISRVEAEDVGVYYCQQHHRFPLTFGQGTKLEIKRTVAAPSVFIFP
PSDEQLKSGTASVVCLLNNFYPREAKVQWKVDNALQSGNSQESVTEQDSKDSTYSLSSTLTLS
KADYEKHKVYACEVTHQGLSSPVTKSFNRGEC
hu199-36 antibody heavy chain (SEQ ID NO: 141): 
EVQLVQSGAEVKKPGASVKVSCKASGYTFTTYWMHWVKQAPGQGLEWMGMIDPSD
SETTLIQKFKDKVTLTVDKSTSTAYMELSSLRSEDTAVYYCARTLDGYYDYWGQGTTV
TVSSASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPELLGGPS
VFLFPPKPKDTLYITREPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFS
CSVMHEALHNHYTQKSLSLSPGK
hu199-36 antibody light chain (SEQ ID NO: 142):

DIQMTQSPSSLSASVGDRVTITCRASENIYSYLAWYQQKPGKSPQLLVYFAKTLAEGVP

SRFSGSVSGTQFTLTISSLQPEDFATYYCQHHYGTPWTFGGGTKVEIKRTVAAPSVFIFPP
SDEQLKSGTASVVCLLNNFYPREAKVQWKVDNALQSGNSQESVTEQDSKDSTYSLSSTLTLS
KADYEKHKVYACEVTHQGLSSPVTKSFNRGEC

Note: the underlined parts indicate the CDRs, and the italicized parts indicate the constant regions.

In the present disclosure, AMG157 was used as a positive control, which has sequences set forth in SEQ ID NO: 143 and SEQ ID NO: 144.
Heavy chain sequence of AMG157 (SEQ ID NO: 143): 
QMQLVESGGGVVQPGRSLRLSCAASGFTFRTYGMHWVRQAPGKGLEWVAVIWYDGS
NKHYADSVKGRFTITRDNSKNTLNLQMNSLRAEDTAVYYCARAPQWELVHEAFDIWG
QGTMVTVSSASTKGPSVFPLAPCSRSTSESTAALGCLVKDYFPEPVTVSWNSGALTSGV
HTFPAVLQSSGLYSLSSVVTVPSSNFGTQTYTCNVDHKPSNTKVDKTVERKCCVECPPC
PAPPVAGPSVFLFPPKPKDTLMISRTPEVTCVVVDVSHEDPEVQFNWYVDGVEVHNAK
TKPREEQFNSTFRVVSVLTVVHQDWLNGKEYKCKVSNKGLPAPIEKTISKTKGQPREP
QVYTLPPSREEMTKNQVSLTCLVKGFYPSDIAVEWESNGQPENNYKTTPPMLDSDGSF
FLYSKLTVDKSRWQQGNVFSCSVMHEALHNHYTQKSLSLSPGK
Light chain sequence of AMG157 (SEQ ID NO: 144):

SYVLTQPPSVSVAPGQTARITCGGNNLGSKSVHWYQQKPGQAPVLVVYDDSDRPSWIP
ERFSGSNSGNTATLTISRGEAGDEADYYCQVWDSSSDHVVFGGGTKLTVLGQPKAAPS
VTLFPPSSEELQANKATLVCLISDFYPGAVTVAWKADSSPVKAGVETTTPSKQSNNKYA
ASSYLSLTPEQWKSHRSYSCQVTHEGSTVEKTVAPTECS

In addition, in the assay for antibody activity, the human TSLP receptor and human IL-7Rα were used in the present disclosure to construct cell lines, which have sequences shown below: Amino acid sequence of human TSLP receptor full-length sequence (SEQ ID NO: 145):

MGRLVLLWGAAVFLLGGWMALGQGGAAEGVQIQIIYFNLETVQVTWNASKYSRTNLT
FHYRFNGDEAYDQCTNYLLQEGHTSGCLLDAEQRDDILYFSIRNGTHPVFTASRWMVY
YLKPSSPKHVRFSWHQDAVTVTCSDLSYGDLLYEVQYRSPFDTEWQSKQENTCNVTIE
GLDAEKCYSFWVRVKAMEDVYGPDTYPSDWSEVTCWQRGEIRDACAETPTPPKPKLS
KFILISSLAILLMVSLLLLSLWKLWRVKKFLIPSVPDPKSIFPGLFEIHQGNFQEWITDTQ
NVAHLHKMAGAEQESGPEEPLVVQLAKTEAESPRMLDPQTEEKEASGGSLQLPHQPL
QGGDVVTIGGFTFVMNDRSYVAL

Note: the underlined part indicates a signal peptide.

Amino acid sequence of human IL-7Ra full-length sequence (Uniprot accession No.: P16871) (SEQ ID NO: 146):

Note: the underlined part indicates a signal peptide.

The antibodies of the present disclosure can be cloned, expressed and purified using conventional methods of gene cloning and recombinant expression.

### Part II. Antibody Activity Assay

### Test Example 1. Assay on Binding of Anti-TSLP Antibodies to Human TSLP by ELISA

Human TSLP-his (SEQ ID NO: 1) was diluted to 1 µg/mL with a PBS buffer (Shanghai BasalMedia, B320) at pH 7.4, and added to a 96-well plate (Coming, CLS3590-100EA) at 100 µg/well. The plate was incubated at 4 °C overnight. After the liquid was discarded, 5% skim milk (skim milk powder from Brightdairy) blocking solution diluted with PBS was added at 200 µL/well, and the plate was incubated in an incubator at 37 °C for 2 h for blocking. After the blocking, the blocking solution was discarded, and the plate was washed 3 times with a PBST buffer (PBS containing 0.1% (v/v) tween-20, pH 7.4). The test antibodies at different concentrations obtained by dilution with a sample diluent and the positive antibody AMG157 were added at 100 µL/well, and the plate was incubated in an incubator at 37 °C for 1 h. After the incubation, the plate was washed 3 times with PBST. An HRP-labeled goat anti-mouse secondary antibody (Jackson Immuno Research, 115-035-003) diluted with a sample diluent was added at 100 µL/well, and the plate was incubated at 37 °C for 1 h. After the plate was washed 6 times with PBST, a TMB chromogenic substrate (KPL, 52-00-03) was added at 50 µL/well, and the plate was incubated at room temperature for 10-15 min. Then, 1 M H₂SO₄ was added at 50 µL/well to stop the reaction. The absorbance at 450 nm was read with a NOVOStar microplate reader, and the EC₅₀ values for the binding of the TSLP antibodies to TSLP were calculated. The results are shown in Table 32 below.

**Table 32. Results for binding activity of antibodies against human TSLP**

| Antibody | EC50 (nM) | Antibody | EC50 (nM) | Antibody | EC50 (nM) |
|---|---|---|---|---|---|
| Ch3 | 0.4929 | hu119-14 | 0.345 | hu179-09 | 0.1573 |
| hu3-01 | 0.8494 | hu119-15 | 0.3497 | hu179-10 | 0.19 |
| hu3-02 | 0.6285 | hu119-16 | 0.366 | hu179-11 | 0.1369 |
| hu3-03 | 0.5545 | hu119-17 | 0.3515 | hu179-12 | 0.1437 |
| hu3-04 | 0.4353 | hu119-18 | 0.3455 | hu179-13 | 0.2011 |
| hu3-05 | 0.5168 | hu119-19 | 0.3533 | hu179-14 | 0.2053 |
| hu3-06 | 0.594 | hu119-20 | 0.3412 | hu179-15 | 0.2035 |
| hu3-07 | 0.3853 | hu119-21 | 0.3987 | hu179-16 | 0.2287 |
| hu3-08 | 0.4687 | hu119-22 | 0.351 | hu179-17 | 0.218 |
| hu3-09 | 0.4941 | hu119-23 | 0.3404 | hu179-18 | 0.2458 |
| hu3-10 | 0.3879 | hu119-24 | 0.3446 | hu179-19 | 0.1616 |
| hu3-12 | 0.1519 | hu119-25 | 0.3575 | hu179-20 | 0.7077 |
| hu3-13 | 0.1477 | hu119-26 | 0.3782 | hu179-21 | 0.9784 |
| Ch119 | 0.851 | hu119-27 | 0.3347 | hu179-22 | 0.7519 |
| hu119-01 | 0.107 | hu119-28 | 0.2648 | hu179-23 | 0.997 |
| hu119-02 | 0.1938 | hu119-29 | 0.2729 | hu179-24 | 0.6358 |
| hu119-03 | 0.1593 | hu119-28 | 0.2648 | hu179-25 | 0.1313 |
| hu119-04 | 0.1881 | hu119-29 | 0.2729 | hu179-26 | 0.2006 |
| hu119-05 | 0.1445 | Ch179 | 0.2023 | hu179-27 | 0.1799 |
| hu119-06 | 0.2206 | hu179-01 | 0.1248 | hu179-28 | 0.0906 |
| hu119-07 | 0.2132 | hu179-02 | 0.1697 | hu179-29 | 0.2041 |
| hu119-08 | 0.2015 | hu179-03 | 0.138 | hu179-30 | 0.246 |
| hu119-09 | 0.1492 | hu179-04 | 0.1886 | hu179-31 | 0.2012 |
| hu119-10 | 0.2329 | hu179-05 | 0.1416 | hu179-32 | 0.145 |
| hu119-11 | 0.174 | hu179-06 | 0.2188 | Ch199 | 0.5157 |
| hu119-12 | 0.2034 | hu179-07 | 0.4478 | AMG157 | 0.7219 |
| hu119-13 | 0.3438 | hu179-08 | 0.1615 | | |

The results showed that the antibodies of the present disclosure had good binding activity against human TSLP.

### Test Example 2. Assay on Affinity of Anti-TSLP Humanized Antibodies for TSLP of Different Species by Biacore

The affinity of the test humanized TSLP antibodies for human and monkey TSLP was determined using a Biacore T200 (GE) instrument.

The test molecules were affinity-captured on a Protein A biosensor chip (Cat. # 29127556, GE), and then antigens (huTSLP-his, cynoTSLP-his, prepared in example 1-1) flowed through the surface of the chip. The reaction signals were detected in real time using a Biacore T200 instrument to obtain association and dissociation curves. After dissociation was completed for each cycle, the biosensor chip was washed with a glycine-hydrochloric acid regeneration solution (pH 1.5, Cat # BR-1003-54, GE). Data were fitted using BIAevaluation version 4.1, GE software with a (1:1) Langmuir model to obtain affinity values. The results are shown in Table 33 below.

**Table 33. Affinity of anti-TSLP antibodies for TSLP of different species**

| Antibody | Affinity for huTSLP KD (M) | Affinity for cynoTSLP KD (M) |
|---|---|---|
| AMG157 | 8.12E-12 | 9.22E-12 |
| hu179-33 | 9.03E-13 | 3.04E-11 |
| hu3-13 | 1.0E-12 | 3.40E-10 |
| hu119-30 | 5.0E-12 | 1.95E-09 |
| hu199-3 6 | 10.5E-12 | 1.72E-11 |

The results showed that the anti-TSLP antibodies of the present disclosure had high affinity for human TSLP and could also bind to cynomolgus monkey TSLP.

### Test Example 3. Assay on Blocking of Binding of TSLP to TSLP Receptor by Anti-TSLP Antibodies Based on ELISA

The TSLP receptor has two subunits, TSLPR and IL-7R. TSLPR is a TSLP-specific receptor, and IL-7R is a shared receptor of TSLP and IL-7. TSLP binds to TSLPR first, and then to IL-7R. This test example was performed to identify whether TSLP antibodies could block the binding of TSLP to the extracellular region of the recombinantly expressed TSLPR receptor protein.

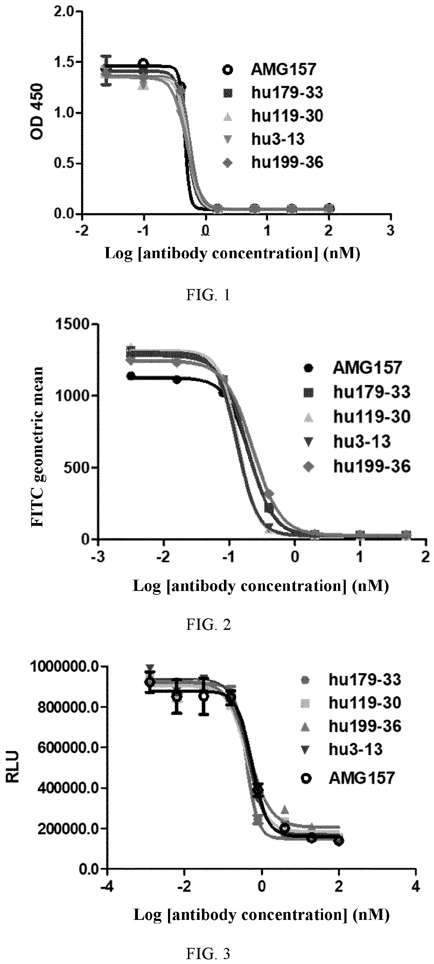

An ELISA plate was coated with human-TSLPR-Fc-ECD (2 µg/mL, SEQ ID NO: 5) and incubated at 4 °C overnight. After the liquid was discarded, 5% skim milk blocking solution diluted with PBS was added at 200 µL/well, and the plate was incubated in an incubator at 37 °C for 2 h for blocking. After the blocking, the blocking solution was discarded, and the plate was washed 3 times with a PBST buffer (PBS containing 0.05% (v/v) tween-20, pH 7.4). A biotin-labeled huTSLP-Fc antigen was prepared at 3 nM, and the test antibody was diluted in a gradient from 200 nM. After the antigen and antibody were mixed uniformly at a ratio of 1:1, the mixture was left to stand at 37 °C for 15 min and added to the plate at 100 µL/well, and the plate was left to stand at 37 °C for 1 h. After the plate was washed 3 times with PBST, streptavidin-peroxidase polymer diluted with a sample diluent at a dilution of 1:4000 was added at 100 µL/well, and the plate was incubated at 37 °C for 1 h. After the plate was washed 5 times with PBST, a TMB chromogenic substrate (KPL, 52-00-03) was added at 100 µL/well, and the plate was incubated at room temperature for 3-10 min. Then, 1 M H₂SO₄ was added at 100 µL/well to stop the reaction. The absorbance at 450 nm was read with a NOVOStar microplate reader, and IC₅₀ values for the blocking of the binding of TSLP to TSLPR by the TSLP antibodies were calculated. The results are shown in Table 34 and FIG. 1.

**Table 34. Results for blocking activity of antibodies**

| Antibody | hu179-33 | hu119-30 | hu3-13 | hu199-36 |
|---|---|---|---|---|
| IC50 (nM) | 0.5038 | 0.5192 | 0.4975 | 0.5693 |

The results showed that the antibodies of the present disclosure could strongly inhibit the binding of TSLP to its receptor TSLPR.

### Test Example 4. Assay on Blocking of Binding of TSLP to TSLP Receptor by TSLP Antibodies Based on FACS

This test example was performed to identify anti-TSLP antibodies that can each block the binding of TSLP to TSLPR/IL-7R receptor on the surface of the CHOK1 cell line.

The specific process was as follows: CHOK1-TSLPR/II,-7R was incubated with DME/F12 containing 10% FBS, 1 mg/mL G418, and 10 µg/mL puromicine, and the CHOK1-TSLPR/IL-7R cells in good condition were centrifuged (1000 rpm, 5 min), washed once with 2% FBS in PBS and counted. After the cells were adjusted to a concentration of 1× 10⁶ cells/mL, 50 µL of cell suspension was added to a round-bottom 96-well plate. The test antibody was serially diluted with PBS containing 2% BSA at an initial concentration of 20 nM at a 1:4 dilution ratio to obtain 8 gradients. A biotin-labeled TSLP-Fc antigen was prepared at 2 nM. After the antigen and the antibody were mixed uniformly at a ratio of 1:1, the mixture was left to stand at 37 °C for 15 min and added, at 50 µL/well, to the 96-well plate into which the cells seeded. The plate was incubated at 4 °C for 1 h. After the incubation, the plate was centrifuged at 4 °C (800 g, 5 min), and the supernatant was discarded. The cells were centrifuged and washed with 200 µL of precooled PBS, which was repeated twice. A PE-SA secondary antibody diluted at a 1:1000 dilution ratio was added, and the plate was incubated at 4 °C in the dark for 40 min. The plate was centrifuged at 4 °C (800 g, 5 min), the supernatant was discarded, and 200 µL of precooled PBS was added for resuspending the cells. The cells were centrifuged and washed at 4 °C, which was repeated three times. 100 µL of PBS was added, and the plate was loaded on a machine for reading. The IC₅₀ values for the blocking of the binding of TSLP to TSLPR/IL-7R by the TSLP antibodies were calculated according to fluorescence signal values. The results are shown in Table 35.

**Table 35. Results for blocking of TSLPR on the cell surface by antibodies**

| Antibody | AMG157 | hu179-33 | hu119-30 | hu3-13 | hu199-36 |
|---|---|---|---|---|---|
| IC50 (nM) | 0.2068 | 0.1867 | 0.1368 | 0.1325 | 0.2270 |

The results showed that the antibodies of the present disclosure could strongly block the binding of TSLP to TSLPR/IL-7R on the cell surface.

### Test Example 5. Inhibition of TSLP-Induced Chemokine Production by Anti-TSLP Antibodies

TSLP can induce primary myeloid dendritic cells (mDCs) to mature and secrete chemokines, thymus activation regulated chemokine (TARC) and osteoprotegerin (OPG), thereby further mediating innate and adaptive immune inflammatory responses. This test example was performed to verify that the obtained antibody can block the TSLP from inducing mDCs to generate chemokines, thereby blocking the occurrence of innate and adaptive inflammatory responses.

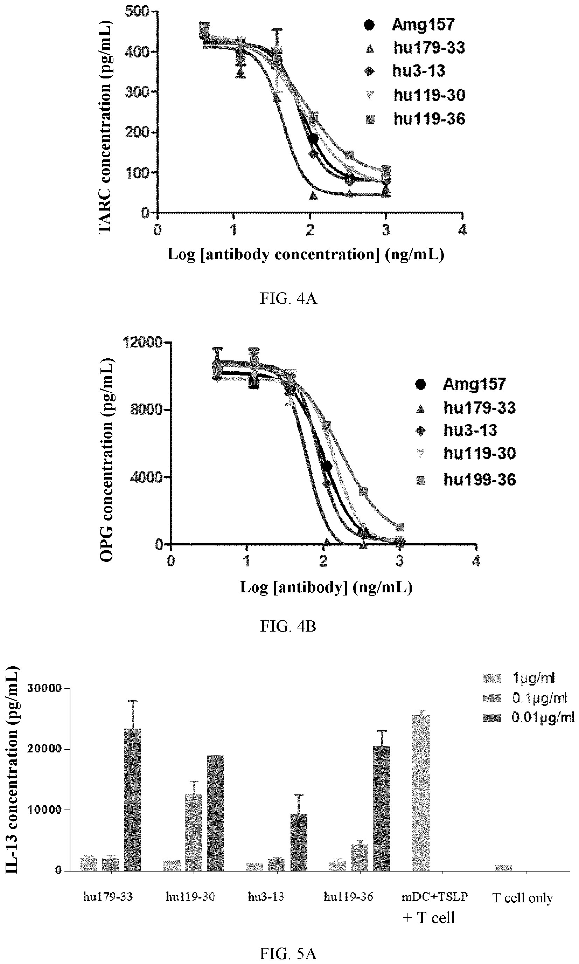

Primary myeloid mDCs were isolated from human peripheral blood mononuclear cells (PBMCs) and purified by magnetic bead sorting (CD1c (BDCA-1) + dendritic cell isolation kit, Miltenyi Biotec), and the obtained mDCs were seeded into a 96-well cell culture plate. The antibody samples obtained by gradient dilution and human TSLP (huTSLP-his, final concentration: 50 ng/mL) were incubated for about 45 min (37 °C) in advance, then added to the culture wells containing mDCs, respectively, for *in vitro* stimulation of the mDCs, and the plate was incubated in an incubator for 48 h. The cell culture supernatant was collected, diluted properly, and detected for the content of chemokines by an ELISA method. TARC was detected using human CCL17/TARC Quantikine ELISA Kit (R&D); and the OPG content was determined using human CCL22/MDC Quantikine ELISA Kit (R&D). The results are shown in FIGs. 4A-4B.

The results showed that antibodies of the present disclosure all could significantly inhibit TSLP-induced production of the chemokines TARC and OPG, suggesting that the antibodies of the present disclosure can block the occurrence of innate and adaptive inflammatory responses.

### Test Example 6. Blocking of Native TSLP-Induced Proliferation of BaF3-TLSPR/IL-7R Cells by Anti-TSLP Antibodies

BaF3-hTSLPR/hIL-7R cells can proliferate under stimulation of native TSLP, and the stimulation effect of TSLP on BaF3-hTSLPR/hIL-7R cells is reduced after the antibody binds to the native TSLP.

NHLF cells (Beina Bio, BNCC340764) and HLF1 cells (Beina Bio, BNCC337730) were cultured. When the cells grew to convergence of 80%, the supernatant was discarded, and human lung fibroblasts NHLF (Beina Bio, BNCC340764) and HLF1 (Beina Bio, BNCC337730) were stimulated with 10 ng/mL IL1-β (Sino Biological, GMP-10139-HNAE), 20 ng/mL IL13 (R&D, 213-ILB-005), and 20 ng/mL TNF-α (PEPROTECH 300-01A) for 72 h to induce the production of native TSLP. After the stimulation, the cell supernatant was collected and centrifuged at 4500 rpm for 5 min to remove cell debris. The supernatant was collected, concentrated to about 10-fold concentration by a concentration column, and filtered for later use.

BaF3-hTSLPR/hIL-17R cells were cultured in RPMI1640 (10 ng/mL mIL3, R&D 213-II,B-005) containing 10% FBS, adjusted to a density of 1 × 10⁴ cells/mL, and cultured in an incubator at 37 °C with 5% CO₂ to the logarithmic phase. The cells were collected and centrifuged at 800 rpm/min for 5 min, and the supernatant was discarded. The cells were washed three times with PBS to remove cytokines that stimulate the proliferation of the cells from the medium. The cells were resuspended in an RPMI1640 medium containing 4% FBS, seeded into a 96-well plate at 4000 cells/50 µL/well, and cultured in an incubator for 2 h. The test antibody was diluted with native TSLP at an initial concentration of 100 nM at a 10-fold dilution ratio to obtain 3 gradients of 100 nM, 10 nM, and 1 nM. The antibody/antigen mixture obtained by the dilution was added to the cells at 50 µL/well to make final antibody concentrations of 50 nM, 5 nM, and 0.5 nM, and the mixture was cultured in an incubator at 37 °C with 5% CO₂ for 72 h. Then, 30 µL of Cell Titer-Glo (Promega) was added to each well, and the plate was incubated at room temperature in the dark for 10 min and detected using the Luminescence program of Cytation5 cell imager. The results are shown in Table 36 below.

**Table 36. Results for inhibition of proliferation of BaF3-TLSPR/IL-7R cells by anti-TSLP antibodies**

| Antibody | AMG157 | hu179-33 | hu3-13 | hu119-30 |
|---|---|---|---|---|
| IC50 (nM) | 3.379 | 0.02279 | 0.2888 | 1.533 |

The results showed that the antibodies of the present disclosure could significantly inhibit the activity of native TSLP from stimulating the proliferation of BaF3, particularly the antibody hu179-33, the activity of which was 100 times or more of AMG157.

### Test Example 7. Assay on Inhibition of TSLP-Induced Proliferation of BaF3 Cells Overexpressing TSLPR/IL7R by Anti-TSLP Antibodies

TSLP can bind to TSLPR/IL-7R on the surface of BaF3, thereby promoting the proliferation of BaF3. This test example was performed to identify that the antibodies of the present disclosure can block the activity of TSLP to induce the proliferation of BaF3.

The specific process was as follows: BaF3 cells overexpressing TSLPR/IL-7R were cultured in RPMI1640 containing 10% FBS and 2 ng/mL rhIL3 (LiankeBio, Catalog No. 96-AF-300-03-20) in an incubator at 37 °C with 5% CO₂ to a cell density of no more than 1 × 10⁶ cells/mL. In the assay for the antibodies, the cells in the logarithmic growth phase were washed three times with PBS, centrifuged at 800 rpm for 5 min, and adjusted to a cell density of 8000 cells/well/90 µL with RPMI1640 (2% FBS, recombinant human TSLP-Fc: 40 ng/mL). 10 µL of the test antibody diluted in a gradient was added to a 96-well plate, and the mixture was cultured for 2 days. 30 µL of cell titer was added to the mixture to be mixed uniformly for detection, and then the IC₅₀ values were calculated according to the readings. The results are shown in Table 37 and FIG. 3.

**Table 37. Inhibition of proliferative activity of BaF3 cells by antibodies**

| Antibody | AMG157 | hu179-33 | hu119-30 | hu3-13 | hu199-36 |
|---|---|---|---|---|---|
| IC50 (nM) | 0.5730 | 0.4092 | 0.4305 | 0.4436 | 0.4769 |

The results showed that the antibodies of the present disclosure all had a strong ability to inhibit TSLP-mediated proliferation of BaF3 cells.

### Test Example 8. Blocking of TSLP-Induced Differentiation of Native CD4⁺ T Cells into Th2 Cells by Humanized Anti-TSLP Antibodies

TSLP can induce primary myeloid mDC cells to mature, and the mature mDC cells highly express the OX40 ligand. The OX40 ligand can bind to OX40 on the surface of native **CD4⁺** T cells, thereby causing native **CD4⁺** T cells to differentiate into Th2 cells, which produce immune response-related factors such as IL-4/IL-5/IL-13, resulting in the Th2 inflammatory response in the body. This test example was performed to identify that the antibodies of the present disclosure can block TSLP-induced differentiation of Th2 cells.

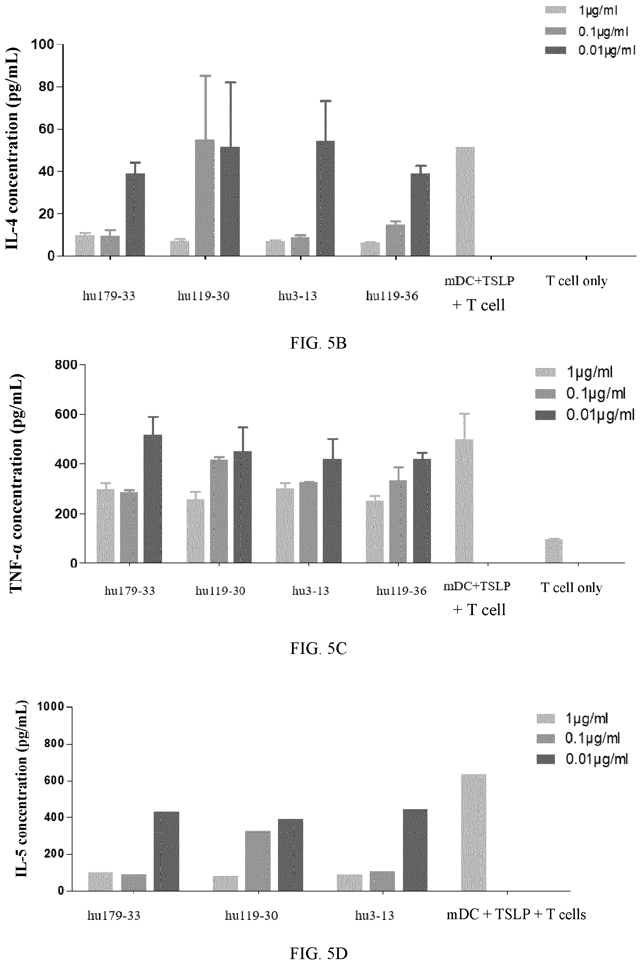

Primary myeloid DCs were isolated from human peripheral blood mononuclear cells (PBMCs) and purified by magnetic bead sorting (CD1c (BDCA-1) + dendritic cell isolation kit, Miltenyi Biotec), and the obtained mDCs were seeded into a 96-well cell culture plate. The antibody samples obtained by gradient dilution and the recombinantly expressed human TSLP (huTSLP-his, final concentration: 50 ng/mL) were incubated for about 45 min (37 °C) in advance, then added to the culture wells containing mDCs, respectively, and the plate was incubated at 37 °C for 24 h. Mature mDCs by the stimulation were collected and washed twice with PBS. CD4⁺ CD45RA⁺ native T cells were extracted from PBMCs by magnetic bead isolation (Myltenyi, Biotec). The isolated native T cells and mature mDCs were mixed at a ratio of 5:1 and seeded into a 96-well cell culture plate and co-cultured for 6 days. The cells were collected, seeded into a 96-well plate in which anti-CD3 (10 µg/mL) was pre-coated, and anti-CD28 (1 µg/mL) was added to re-stimulate differentiated T-cells. The mixture was cultured for 24 h, and finally, the cell culture supernatant was collected. The supernatant was detected for Th2-related cytokines secreted by the cells by ELISA. IL-4 and IL-5 cytokines were detected using the ELISA kit from R&D, and TNF-α and IL-13 were detected using the ELISA kit from NeoBioscience. The results are shown in FIGs. 5A-5D. No antibody was added to the mDC + TSLP + T cell group and T cell group.

The results showed that the antibody of the present disclosure could significantly inhibit the production of Th2 cytokines IL-4, IL-5, IL-13, and TNF-α, suggesting that the antibodies of the present disclosure can block TSLP-induced differentiation of Th2 cells.

### Part III. Formulation

### The equipment used in the formulation preparation and determination and the calculation method for results are shown below:

### SEC molecular exclusion chromatography:

This is a method for analyzing the separation of a solute by the relative relationship between the pore size of the gel pores and the size of the polymer sample molecule coil.

SEC monomer content percentage (SEC%) = A monomer / A total × 100% (A monomer represents the peak area of the main peak monomer in the sample, and A total represents the sum of all peak areas).
Instrument for SEC determination: Agilent 1260; column: Waters, XBrige BEH200Å SEC (300 ×7.8 mm, 3.5 µm)

### CE (NR) capillary gel electrophoresis:

This is a method of moving the gel into a capillary as a supporting medium for electrophoresis and performing separation according to the molecular weight of the sample under a certain voltage.

Non-reduced CE purity percentage (CE-(NR)%) = A main peak / A total × 100% (A main peak represents the peak area of the main peak in the sample, and A total represents the sum of all peak areas).
Instrument for CE determination: Beckman model plus800

### iCIEF imaged capillary isoelectric focusing electrophoresis:

This is a technique for separation according to the difference of isoelectric points (pI) of proteins.

iCIEF neutral peak content percentage (iCIEF%) = neutral peak area / total area × 100% (total area represents the sum of areas of acidic, neutral and basic peaks).

Manufacturer of instrument for the iCIEF determination: simple protein, model: muarice.

### IEC ion exchange chromatography:

This is a technique for sequentially eluting the protein in the order of weak to strong affinity of the charged proteins for the stationary ion groups by increasing the concentration of salt ions in the mobile phase, taking the ion exchange resin as a stationary phase.

IEC neutral peak content percentage = neutral peak area / total area × 100% (total area represents the sum of areas of acidic, neutral and basic peaks).

Manufacturer of instrument for IEC determination: Agilent, model: 1260 Bio.

### Osmotic pressure determination:

The freezing point method is used for determining the osmotic pressure. The freezing point of a solution is determined by using a high-sensitivity temperature-sensing element on the basis of the proportional relation between the freezing point depression value and the molar concentration of the solution, and then converted into the osmotic pressure through electric quantity. Manufacturer of instrument: Loser, model: OM815.

### Protein concentration determination:

Instrument for protein concentration determination: ultraviolet-visible spectrophotometer, model: Nano Drop oneC.

### Example 2-1. Screening of Buffer Systems and pH Values for Formulations

A formulation containing 100 mg/mL hu179-33 antibody and 0.1 mg/mL polysorbate 80 (PS80) was prepared using the following different buffers. The antibody was exchanged into each of the following buffers by ultrafiltration.
1. 20 mM succinic acid-sodium succinate (SA), pH 5.0;
2. 20 mM SA, pH 5.5;
3. 20 mM citric acid-sodium citrate (CA), pH 5.0;
4. 20 mM CA, pH 5.5;
5. 20 mM histidine-hydrochloride (His-HCl), pH 5.5;
6. 20 mM His-HCl, pH 6.0;
7. 20 mM His-HCl, pH 6.5;
8. 20 mM histidine-acetate (His-AA), pH 5.5;
9. 20 mM His-AA, pH 6.0;
10. 20 mM His-AA, pH 6.5;
11. 20 mM phosphate (PB), pH 7.0;
12. 20 mM PB, pH 7.5; and
13. 20 mM tris(hydroxymethyl)aminomethane-hydrochloride (TRIS), pH 7.5.

Each of the formulations was filtered and filled into vials, which were stoppered and capped. The samples were taken for forced degradation experiments of high-temperature stability (40 °C), shaking (25 °C, 300 rpm), and 5 freeze-thaw cycles (FT5C) from -35 °C to 2-8 °C. The stability of the antibody in each of the buffer systems was investigated in terms of appearance, SEC monomer, non-reduced CE monomer (CE (NR)), and iCIEF neutral peaks. The results are shown in Table 38 below.

**Table 38. Stability results for buffer and pH screening**

| Buffer and pH | Storage condition | Appearance | SEC% | ΔSEC% | CE (NR)% | ΔCE (NR)% | iCIEF % | ΔiCIEF% |
|---|---|---|---|---|---|---|---|---|
| 20 mM SA 5.0 | 0 h | Clear and transparent | 98.67 | / | 94.71 | / | 58.02 | / |
| | FT5C | Clear and transparent | 97.74 | 0.93 | 94.73 | -0.02 | 58.2 | -0.18 |
| | Shaking D7 | Clear and transparent | 97.77 | 0.9 | 94.24 | 0.47 | 57.32 | 0.7 |
| | 40°C M1 | Clear and transparent | 92.39 | 6.28 | 85.73 | 8.98 | 30.03 | 27.99 |
| 20 mM SA 5.5 | 0 h | Clear and transparent | 98.72 | / | 94.61 | / | 58.41 | / |
| | FT5C | Clear and transparent | 97.74 | 0.98 | 94.43 | 0.18 | 58.39 | 0.02 |
| | Shaking D7 | Clear and transparent | 97.77 | 0.95 | 94.21 | 0.4 | 57.29 | 1.12 |
| | 40°C M1 | Clear and transparent | 93.17 | 5.55 | 86.17 | 8.44 | 33.98 | 24.43 |
| 20 mM CA 5.0 | 0 h | Opalescent | 98.7 | / | 94.8 | / | 57.6 | / |
| | FT5C | Opalescent | N/A | N/A | N/A | N/A | N/A | N/A |
| | Shaking D7 | Fine particles +++ | N/A | N/A | N/A | N/A | N/A | N/A |
| | 40°C M1 | N/A | N/A | N/A | N/A | N/A | N/A | N/A |
| 20 mM CA 5.5 | 0 h | Opalescent | 98.68 | | 94.68 | | 58.6 | |
| | FT5C | Opalescent | 97.85 | 0.83 | 94.55 | 0.13 | 57.78 | 0.82 |
| | Shaking D7 | Opalescent | 97.84 | 0.84 | 94.06 | 0.62 | 57.71 | 0.89 |
| | 40°C M1 | Fine particles ++ | 93.32 | 5.36 | 87.66 | 7.02 | 28.6 | 30 |
| 20 mM His-HCl 5.5 | 0 h | Clear and transparent | 98.75 | / | 94.81 | / | 57.64 | / |
| | FT5C | Clear and transparent | 97.82 | 0.93 | 94.95 | -0.14 | 58.51 | -0.87 |
| | Shaking D7 | Clear and transparent | 97.87 | 0.88 | 94.16 | 0.65 | 58 | -0.36 |
| | 40°C M1 | Clear and transparent | 94.21 | 4.54 | 87.38 | 7.43 | 37.07 | 20.57 |
| 20 mM His-HCl 6.0 | 0 h | Clear and transparent | 98.71 | / | 94.79 | / | 57.64 | / |
| | FT5C | Clear and transparent | 97.71 | 1 | 94.69 | 0.1 | 57.86 | -0.22 |
| | Shaking D7 | Clear and transparent | 97.81 | 0.9 | 94.77 | 0.02 | 58.01 | -0.37 |
| | 40°C M1 | Clear and transparent | 94.08 | 4.63 | 88.86 | 5.93 | 39.06 | 18.58 |
| 20 mM His-HCl 6.5 | 0 h | Clear and transparent | 98 | / | 94.81 | / | 57.47 | / |
| | FT5C | Clear and transparent | 97.68 | 0.32 | 94.67 | 0.14 | 57.51 | -0.04 |
| | Shaking D7 | Clear and transparent | 97.9 | 0.1 | 94.71 | 0.1 | 58.33 | -0.86 |
| | 40°C M1 | Suspended particles + | 93.63 | 4.37 | 88.76 | 6.05 | 36.12 | 21.35 |
| 20 mM His-AA 5.5 | 0 h | Clear and transparent | 98.67 | / | 94.71 | / | 57.8 | / |
| | FT5C | Clear and transparent | 97.82 | 0.85 | 94.5 | 0.21 | 58.28 | -0.48 |
| | Shaking D7 | Clear and transparent | 97.84 | 0.83 | 94.43 | 0.28 | 57.81 | -0.01 |
| | 40°C M1 | Clear and transparent | 93.96 | 4.71 | 88.68 | 6.03 | 34.98 | 22.82 |
| 20 mM His-AA 6.0 | 0 h | Clear and transparent | 97.91 | / | 94.74 | / | 57.37 | / |
| | FT5C | Clear and transparent | 97.91 | 0 | 94.56 | 0.18 | 57.29 | 0.08 |
| | Shaking D7 | Clear and transparent | 97.76 | 0.15 | 94.66 | 0.08 | 57.83 | -0.46 |
| | 40°C M1 | Clear and transparent | 94.13 | 3.78 | 90.09 | 4.65 | 36.61 | 20.76 |
| 20 mM His-AA 6.5 | 0 h | Clear and transparent | 98.33 | / | 94.85 | / | 57.51 | / |
| | FT5C | Clear and | 97.46 | 0.87 | 94.61 | 0.24 | 57.46 | 0.05 |
| | | transparent | | | | | | |
| | Shaking D7 | Clear and transparent | 97.95 | 0.38 | 94.76 | 0.09 | 57.36 | 0.15 |
| | 40°C M1 | Clear and transparent | 93.61 | 4.72 | 88.92 | 5.93 | 36.03 | 21.48 |
| 20 mM PB7.0 | 0 h | Opalescent | 98.5 | / | 94.6 | / | 58.4 | / |
| | FT5C | Opalescent | N/A | N/A | N/A | N/A | N/A | N/A |
| | Shaking D7 | Turbid | N/A | N/A | N/A | N/A | N/A | N/A |
| | 40°C M1 | N/A | N/A | N/A | N/A | N/A | N/A | N/A |
| 20 mM PB7.5 | 0 h | Opalescent | 98.49 | / | 94.55 | / | 58.3 | / |
| | FT5C | Opalescent | 97.23 | 1.26 | 94.49 | 0.06 | 57.29 | 1.01 |
| | Shaking D7 | Opalescent | 97.07 | 1.42 | 94.06 | 0.49 | 56.27 | 2.03 |
| | 40°C M1 | Opalescent | 89.62 | 8.87 | 80.01 | 14.54 | 18.29 | 40.01 |
| 20 mM TRIS7.5 | 0 h | Opalescent | 98.1 | / | 94.6 | / | 58.7 | / |
| | FT5C | Opalescent | N/A | N/A | N/A | N/A | N/A | N/A |
| | Shaking D7 | Flocculent particles +++ | N/A | N/A | N/A | N/A | N/A | N/A |
| | 40°C M1 | N/A | N/A | N/A | N/A | N/A | N/A | N/A |

| | | | | | | | | |
|---|---|---|---|---|---|---|---|---|
| Note: in the table, "D" denotes day, for example, D3 denotes 3 days, "M" denotes month, for example, M1 denotes 1 month; "0 h" denotes the start of the experiment; "Δ" denotes the amount of change, "FT5C" denotes 5 freeze-thaw cycles, and "N/A" denotes not detected; and the same applies below. | | | | | | | | |

The results were analyzed as follows:
The appearance showed that the antibody appeared to be opalescent at the start of the experiment and after repeated freeze-thaw cycles in the CA pH 5.0 and CA pH 5.5 buffer systems, PB pH 7.0 and PB pH7.5 buffer systems, and TRIS pH7.5 buffer system. After shaking for 7 days, the appearance did not improve or particles or turbidity appeared, suggesting that the antibody has poor stability in the above buffer systems. However, the formulation still had clear and transparent appearance under the forced degradation conditions of repeated freeze-thaw cycles, shaking, and storage at 40 °C for 1 month in SA (pH 5.0 and pH 5.5), His-HCl (pH 5.5, pH 6.0, and pH 6.5), and His-AA (pH 5.5, pH 6.0, and pH 6.5) buffer systems; and the formulation showed suspended particles only in the His-HCl pH 6.5 buffer system after storage at 40 °C for 1 month, suggesting that the antibody was stable in the SA, His-HCl (pH 5.0-6.0), and His-AA (pH 5.0-6.5) buffer systems.

Data for SEC and non-reduced CE showed that: under the conditions of repeated freeze-thaw cycles and shaking for 7 days, the SEC monomer, CE (NR) monomer, and iCIEF monomer of all formulas showed no significant change; however, under the condition of 40 °C M1, the SEC monomer was reduced by about 6.28% and the CE (NR) monomer was reduced by 8.98% in the SA pH 5.0 buffer system; the SEC monomer was reduced by about 5.55%, and the CE (NR) monomer was reduced by 8.44% in the SA pH 5.5 buffer system; and the SEC monomer was reduced by about 3.78%-4.72%, and the CE (NR) monomer was reduced by about 4.65%-7.43% in His-HCl and His-AA buffer systems, with the reduction range less than that of the SA pH 5.0 and SA pH 5.5 buffer systems. This suggests that the antibody has superior stability in the His-HCl and His-AA systems than that in the SA buffer systems.

In summary, the preferred buffer systems for the anti-TSLP antibody are the His-AA and His-HCl systems, followed by the SA systems, with the pH of 5.0-6.5, preferably 5.5-6.5.

### Example 2-2. Sugar Type Screening

Formulations containing sucrose, trehalose, sorbitol, and mannitol, respectively, and 20 mM His-AA pH 6.0 buffer, 0.4 mg/mL PS80, and 100 mg/mL hu179-33 antibody were prepared. Each of the formulations was filtered and filled into vials, which were stoppered and capped. The samples were subjected to forced degradation experiments of high-temperature stability (40 °C), shaking (25 °C, 300 rpm), and 5 freeze-thaw cycles (-35 °C to 2-8 °C), and the effect of different sugars on the stability of the formulations was investigated. The results are shown in Table 39 below.

**Table 39. Results for sugar type screening**

| Sugar type | Storage condition | SEC% | ΔSEC% | CE (NR)% | ΔCE (NR)% | iCIEF% | Δ iCIEF% |
|---|---|---|---|---|---|---|---|
| 70 mg/mL sucrose | D0 | 98.3 | / | 94.1 | / | 57.2 | / |
| | FT5C | 98.3 | 0 | 94 | 0.1 | 58.2 | -1 |
| | Shaking D7 | 98.1 | 0.2 | 93.9 | 0.2 | 60.1 | -2.9 |
| | 40°C D17 | 95.6 | 2.7 | 92.4 | 1.7 | 46.6 | 10.6 |
| 70 mg/mL trehalose | D0 | 98.3 | / | 94.2 | / | 59.3 | / |
| | FT5C | 98.4 | -0.1 | 94.2 | 0 | 59.1 | 0.2 |
| | Shaking D7 | 98.1 | 0.2 | 93.7 | 0.5 | 59.8 | -0.5 |
| | 40°C D17 | 95.6 | 2.7 | 92.3 | 1.9 | 46.2 | 12.9 |
| 50 mg/mL sorbitol | D0 | 98.3 | / | 94.1 | / | 58.9 | / |
| | FT5C | 98.4 | -0.1 | 94.1 | 0 | 58.6 | 0.3 |
| | Shaking D7 | 98.1 | 0.2 | 93.7 | 0.4 | 60.1 | -1.2 |
| | 40°C D17 | 95.8 | 2.5 | 92.4 | 1.7 | 47.3 | 11.6 |
| 50 mg/mL mannitol | D0 | 98.3 | / | 94.1 | / | 59.2 | / |
| | FT5C | 98.3 | 0 | 94 | 0.1 | 58.9 | 0.3 |
| | Shaking D7 | 98.1 | 0.2 | 93.6 | 0.5 | 59.9 | -0.7 |
| | 40°C D17 | 95.8 | 2.5 | 92.1 | 2 | 47 | 12.2 |

| | | | | | | | |
|---|---|---|---|---|---|---|---|
| Note: the content of trehalose in the table is measured as the content of α,α-trehalose dihydrate. The results showed that there was no significant difference in the effect of sugar type and concentration on the stability of each of the formulations, that is, sucrose, trehalose, sorbitol and mannitol all could act as stabilizers for the TSLP antibody formulations. | | | | | | | |

### Example 2-3. Surfactant Type and Concentration Screening

Formulations containing 20 mM histidine-acetate buffer pH 6.0, 70 mg/mL sucrose, 100 mg/mL hu179-33 antibody, and different polysorbates were prepared. The specific formulas are shown in Table 40 below:

**Table 40. Polysorbate types and concentrations**

| Polysorbate | Buffer | Antibody concentration |
|---|---|---|
| PS80 0.2 mg/mL | 20 mM His-AA | 100 mg/mL |
| PS80 0.4 mg/mL | pH 6.0 + 70 | |
| PS80 0.6 mg/mL | mg/mL sucrose | |
| PS20 0.2 mg/mL | | |
| PS20 0.6 mg/mL | | |

Each of the formulations was filtered and filled into vials, which were stoppered and capped. The samples were taken for forced degradation experiments of high-temperature stability (40 °C), shaking (25 °C, 300 rpm), and 5 freeze-thaw cycles (-35 °C to 2-8 °C), and the effect of PS80 and PS20 on the stability of the formulations was investigated. The results are shown in Table 41 below.

**Table 41. Results for polysorbate type screening**

| PS type and concentration | Storage condition | SEC% | ΔSEC% | CE (NR)% | ΔCE (NR)% | iCIEF% | ΔiCIEF% |
|---|---|---|---|---|---|---|---|
| 0.2 mg/mL | D0 | 98.3 | / | 94.2 | / | 58.8 | / |
| | FT5C | 98.4 | -0.1 | 93.8 | 0.4 | 59 | -0.2 |
| PS80 | Shaking D7 | 98.2 | 0.1 | 93.6 | 0.6 | 60.5 | -1.7 |
| | 40°C D17 | 95.9 | 2.4 | 92.4 | 1.8 | 46.1 | 12.7 |
| 0.4 mg/mL | D0 | 98.3 | / | 94.1 | / | 58.2 | / |
| | FT5C | 98.4 | -0.1 | 93.7 | 0.4 | 59.6 | -1.4 |
| PS80 | Shaking D7 | 98.2 | 0.1 | 93.6 | 0.5 | 59.7 | -1.5 |
| | 40°C D17 | 95.7 | 2.6 | 92.1 | 2 | 46.6 | 11.6 |
| 0.6 mg/mL | D0 | 98.3 | / | 94 | / | 58.9 | / |
| | FT5C | 98.4 | -0.1 | 93.7 | 0.3 | 59.3 | -0.4 |
| PS80 | Shaking D7 | 98.2 | 0.1 | 94 | 0 | 60.2 | -1.3 |
| | 40°C D17 | 95.4 | 2.9 | 92.2 | 1.8 | 45.5 | 13.4 |
| 0.2 mg/mL | D0 | 98.4 | / | 94 | / | 58.6 | / |
| | FT5C | 98.4 | 0 | 93.6 | 0.4 | 59.4 | -0.8 |
| PS20 | Shaking D7 | 98.2 | 0.2 | 94.2 | -0.2 | 59.7 | -1.1 |
| | 40°C D17 | 96.2 | 2.2 | 92.5 | 1.5 | 47.9 | 10.7 |
| 0.6 mg/mL | D0 | 98.3 | / | 93.8 | / | 58.6 | / |
| PS20 | FT5C | 98.4 | -0.1 | 93.7 | 0.1 | 58.4 | 0.2 |
| | Shaking D7 | 98.3 | 0 | 93.4 | 0.4 | 60.6 | -2 |
| | 40°C D17 | 96.1 | 2.2 | 92.4 | 1.4 | 47.6 | 11 |

The experimental results showed that different types of polysorbates could be used for the antibody formulations of the present disclosure; and there was no difference in the effect of different concentrations of polysorbate 20 or 80 on the stability of the TSLP antibody formulations.

### Example 2-4. Stability Study of formulations

A formulation containing 20 mM His-AA pH 6.0, 100 mg/mL hu179-33 antibody, 70 mg/mL sucrose, and 0.8 mg/mL PS80 was prepared. The formulation was subjected to sterile filtration and filled into vials. The samples were subjected to forced degradation experiments of high-temperature stability (40 °C), shaking (25 °C, 300 rpm), and 5 freeze-thaw cycles (-35 °C to 2-8 °C), and the stability of the formulation in terms of SEC, non-reduced CE, and iCIEF was investigated. The results are shown in Table 42 below.

**Table 42. Results for formulation stability**

| Storage condition | SEC% | CE (NR)% | iCIEF% |
|---|---|---|---|
| 0 h | 98.2 | 94.8 | 59.9 |
| FT5C | 97.8 | 94.7 | 59.3 |
| Shaking D10 | 97.4 | 94.7 | 60.8 |
| 40°C M1 | 93.6 | 88.7 | 39.3 |

The results showed that there was no significant change in the results for all test items under the conditions of freeze-thaw and shaking; and the changes in SEC, CE, and iCIEF were within the normal range of stability after storage for 1 month at 40 °C, and thus the antibody formulation containing 0.8 mg/mL PS80 was very stable.

To investigate the long-term stability of the formulation, the formulation containing 20 mM His-AA pH 6.0, 100 mg/mL hu179-33 antibody, 70 mg/mL sucrose, and 0.8 mg/mL PS80 were stored at 4 °C for 3 months and the stability of the formulation was tested. The results are shown in Table 43 below.

**Table 43. Results for 4 °C stability test**

| Time | Appearance | SEC monomer % | IEC neutral peak % | CE (NR)% |
|---|---|---|---|---|
| 0 h | Clear and transparent | 98.7 | 59.8 | 96.4 |
| 4°C M3 | Clear and transparent | 97.8 | 58.8 | 97.0 |

The results showed that: after the formulation described above was stored at 4 °C for 3 months, there was almost no change in all test indexes, suggesting that the formulation has good stability.

### Examples 2-5. Stabilizer Screening

Formulations containing 20 mM histidine-acetate buffer, pH 6.0, 0.8 mg/mL PS80, 100 mg/mL hu179-33 antibody, and different stabilizers were prepared. The specific formulas are as follows:
1. 30 mg/mL sucrose and 30 mM histidine (His);
2. 30 mg/mL sucrose and 30 mM tryptophan (Trp);
3. 30 mg/mL sucrose and 30 mM methionine (Met);
4. 70 mg/mL sucrose and 10 mM EDTA; and
5. 70 mg/mL sucrose, 20 mM methionine, and 10 mM EDTA.

Each of the formulations was filtered and filled into vials, which were stoppered and capped. The samples were taken for a high-temperature stability (40 °C) study, and the effect of different stabilizers on the stability of the antibody formulations was investigated. The specific results are shown in Table 44 below.

**Table 44. Experimental results for different stabilizers**

| Stabilizer | Storage condition | Appearance | SEC% | CE (NR)% | iCIEF% |
|---|---|---|---|---|---|
| 30 mg/mL sucrose + 30 mM His | D0 | Clear and transparent | 98.3 | 94.87 | 60.74 |
| | 40°C M1 | Clear and transparent | 92.9 | 90.1 | 38.2 |
| 30 mg/mL sucrose + 30 mM Trp | D0 | Clear and transparent | 98.3 | 94.68 | 62.82 |
| | 40°C M1 | Clear and transparent | 94.1 | 89.9 | 42.4 |
| 30 mg/mL sucrose + 30 mM Met | D0 | Clear and transparent | 98.2 | 96.14 | 61.41 |
| | 40°C M1 | Clear and transparent | 94.5 | 90.7 | 41.8 |
| 70 mg/mL sucrose + 10 mM EDTA | D0 | Clear and transparent | 98.3 | 94.63 | 61.16 |
| | 40°C M1 | Slightly opalescent | 94.7 | 92 | 43.4 |
| 70 mg/mL sucrose + 10 mM EDTA + 20 mM Met | D0 | Clear and transparent | 98.2 | 97.2 | 61.68 |
| | 40°C M1 | Slightly opalescent | 95.6 | 93.6 | 45.5 |

The experimental results showed that histidine, tryptophan, methionine, and EDTA and a mixture of the two could also inhibit the aggregation and degradation of the antibody, but did not have a significant effect on the stability of the antibody. Thus, the formulations of the present disclosure may also selectively incorporate the ingredients described above.

### Example 2-6. EDTA Concentration Screening

Formulations containing 20 mM histidine-acetate buffer pH 6.0, 70 mg/mL sucrose, 0.8 mg/mL PS80, 100 mg/mL hu179-33 antibody, and different concentrations of EDTA were prepared. The specific formulas are shown in Table 45 below.

**Table 45. Formulas for EDTA concentration screening**

| EDTA concentration | Other ingredients | Antibody concentration |
|---|---|---|
| 0.37 mM | 20 mM histidine-acetate buffer pH 6.0, 70 mg/mL sucrose, and 0.8 mg/mL PS80 | 100 mg/mL |
| 1 mM | | |
| 2 mM | | |
| 5 mM | | |
| 10 mM | | |

Each of the formulations was filtered and filled into vials, which were stoppered and capped. The samples were taken for a high-temperature stability (40 °C) study, and the appearance, SEC, non-reduced CE and iCIEF of the formulations were investigated. The results are shown in Table 46 below.

**Table 46. Results for EDTA concentration screening**

| EDTA | Storage condition | Appearance | SEC% | CE (NR)% | iCIEF |
|---|---|---|---|---|---|
| 0 mM | D0 | Clear and transparent | 98.4 | 97.2 | 62.4 |
| | 40°CM1 | Clear and transparent | 93.4 | 91.9 | 42.1 |
| 0.37 mM | D0 | Clear and transparent | 98.1 | 97.2 | 61.5 |
| | 40°CM1 | Clear and transparent | 94.5 | 92.2 | 44.4 |
| 1 mM | D0 | Clear and transparent | 98.1 | 97.2 | 62.2 |
| | 40°CM1 | Clear and transparent | 94.7 | 92.1 | 43.2 |
| 2 mM | D0 | Clear and transparent | 98.2 | 97.1 | 61.2 |
| | 40°CM1 | Clear and transparent | 94.7 | 92.5 | 43.9 |
| 5 mM | D0 | Clear and transparent | 98.1 | 97.2 | 61.1 |
| | 40°CM1 | Clear and transparent | 94.9 | 92.9 | 43.5 |
| 10 mM | D0 | Slightly opalescent | 98.1 | 97.2 | 61.4 |
| | 40°CM1 | Slightly opalescent | 95.1 | 93.1 | 43.8 |

The experimental results showed that 0.37-10 mM EDTA could also inhibit the aggregation and degradation of the antibody, but did not have a significant effect on the stability of the antibody. Thus, the formulations of the present disclosure may also selectively incorporate the EDTA ingredient.

### Example 2-7. Preparation of high-concentration formulations

A formulation containing 20 mM His-AA pH 6.0, 150 mg/mL hu179-33 antibody, 70 mg/mL sucrose, and 0.4 mg/mL PS80 was prepared, subjected to sterile filtration, and filled into vials. The samples were subjected to forced degradation experiments of high-temperature stability (40 °C), shaking (25 °C, 300 rpm), and 5 freeze-thaw cycles (-35 °C to 2-8 °C), and the stability of the formulation was investigated. The experimental results are shown in Table 47 below.

**Table 47. High-concentration formulation stability**

| Concentration | Storage condition | SEC% | CE (NR)% | iCIEF% |
|---|---|---|---|---|
| 150 mg/mL | D0 | 98.2 | 95.1 | 60.0 |
| | FT5C | 98.1 | 93.7 | 58.5 |
| | Shaking D7 | 97.9 | 94.1 | 60.4 |
| | 40°C M1 | 93.0 | 89.6 | 38.7 |

The experimental results showed that the formulation containing the antibody at a concentration of 150 mg/mL still had good stability.

Although specific embodiments of the present disclosure have been described above, it will be understood by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A pharmaceutical composition, wherein the pharmaceutical composition comprises an anti-TSLP antibody and a buffer, wherein the buffer is
a histidine salt buffer or a succinate buffer; preferably a histidine-acetate buffer or a histidine-hydrochloride buffer.

2. The pharmaceutical composition according to claim 1, wherein the anti-TSLP antibody comprises a heavy chain variable region and a light chain variable region, wherein:
i) the heavy chain variable region comprises a HCDR1, a HCDR2, and a HCDR3 set forth in SEQ ID NO: 26, SEQ ID NO: 94, and SEQ ID NO: 28, respectively; and
the light chain variable region comprises a LCDR1, a LCDR2, and a LCDR3 set forth in SEQ ID NO: 29, SEQ ID NO: 113, and SEQ ID NO: 31, respectively;
ii) the heavy chain variable region comprises a HCDR1, a HCDR2, and a HCDR3 set forth in SEQ ID NO: 20, SEQ ID NO: 21, and SEQ ID NO: 22, respectively; and
the light chain variable region comprises a LCDR1, a LCDR2, and a LCDR3 set forth in SEQ ID NO: 70, SEQ ID NO: 24, and SEQ ID NO: 25, respectively;
iii) the heavy chain variable region comprises a HCDR1, a HCDR2, and a HCDR3 set forth in SEQ ID NO: 14, SEQ ID NO: 15, and SEQ ID NO: 45, respectively; and
the light chain variable region comprises a LCDR1, a LCDR2, and a LCDR3 set forth in SEQ ID NO: 17, SEQ ID NO: 18, and SEQ ID NO: 54, respectively; or
iv) the heavy chain variable region comprises a HCDR1, a HCDR2, and a HCDR3 set forth in SEQ ID NO: 32, SEQ ID NO: 33, and SEQ ID NO: 34, respectively; and
the light chain variable region comprises a LCDR1, a LCDR2, and a LCDR3 set forth in SEQ ID NO: 35, SEQ ID NO: 36, and SEQ ID NO: 37, respectively;
preferably, the anti-TSLP antibody comprises a heavy chain variable region and a light chain variable region as shown in any one of the following:
a) a heavy chain variable region sequence set forth in SEQ ID NO: 97 and a light chain variable region set forth in SEQ ID NO: 119;
b) a heavy chain variable region sequence set forth in SEQ ID NO: 69 and a light chain variable region set forth in SEQ ID NO: 74;
c) a heavy chain variable region sequence set forth in SEQ ID NO: 50 and a light chain variable region set forth in SEQ ID NO: 52; or
(d) a heavy chain variable region sequence set forth in SEQ ID NO: 132 and a light chain variable region set forth in SEQ ID NO: 125;
and more preferably, the anti-TSLP antibody comprises:
a) a heavy chain set forth in SEQ ID NO: 139 and a light chain set forth in SEQ ID NO: 140;
b) a heavy chain set forth in SEQ ID NO: 137 and a light chain set forth in SEQ ID NO: 138;
c) a heavy chain set forth in SEQ ID NO: 135 and a light chain set forth in SEQ ID NO: 136; or
d) a heavy chain set forth in SEQ ID NO: 141 and a light chain set forth in SEQ ID NO: 142.

3. The pharmaceutical composition according to claim 1 or 2, wherein the buffer is at a concentration of 5-50 mM, preferably 10-30 mM, and more preferably about 20 mM.

4. The pharmaceutical composition according to any one of claims 1-3, wherein the anti-TSLP antibody is at a concentration of 1-150 mg/mL, preferably 100-150 mg/mL, and more preferably about 100 mg/mL.

5. The pharmaceutical composition according to any one of claims 1-4, wherein the pharmaceutical composition further comprises a surfactant, wherein the surfactant is preferably a polysorbate, more preferably polysorbate 80 or polysorbate 20, and most preferably polysorbate 80.

6. The pharmaceutical composition according to claim 5, wherein the surfactant is at a concentration of 0.01-1.0 mg/mL, preferably 0.1-0.8 mg/mL, and more preferably about 0.8 mg/mL.

7. The pharmaceutical composition according to any one of claims 1-6, wherein the pharmaceutical composition further comprises a stabilizer, wherein the stabilizer is selected from the group consisting of one or more of a sugar, an amino acid, and EDTA;
preferably, the sugar is selected from the group consisting of one or more of trehalose, sucrose, sorbitol, and mannitol; and/or
the amino acid is selected from the group consisting of one or more of histidine, tryptophan, and methionine;
and more preferably, the stabilizer is sucrose.

8. The pharmaceutical composition according to claim 7, wherein the stabilizer is a sugar at a concentration of 30-100 mg/mL, preferably 50-70 mg/mL, and more preferably about 70 mg/mL.

9. The pharmaceutical composition according to claim 8, wherein the stabilizer further comprises an amino acid at a concentration of 5-50 mM, preferably 10-30 mM, and more preferably 30 mM.

10. The pharmaceutical composition according to claim 8 or 9, wherein the stabilizer further comprises EDTA at a concentration of 0.1-10 mM, preferably 0.37-10 mM.

11. The pharmaceutical composition according to any one of claims 7-10, wherein the stabilizer is:
i) 30-70 mg/mL sugar and 10-30 mM amino acid;
ii) 30-70 mg/mL sugar and 0.37-10 mM EDTA; or
iii) 30-70 mg/mL sugar, 10-30 mM amino acid, and 0.37-10 mM EDTA;
wherein the sugar is selected from the group consisting of one or more of trehalose, sucrose, sorbitol, and mannitol; and/or
the amino acid is selected from the group consisting of one or more of histidine, tryptophan, and methionine;
preferably, the stabilizer is:
i) 30 mg/mL sucrose and 30 mM histidine, tryptophan or methionine;
ii) 70 mg/mL sucrose and 0.37-10 mM EDTA; or
iii) 70 mg/mL sucrose, 20 mM methionine, and 0.37-10 mM EDTA.

12. The pharmaceutical composition according to any one of claims 1-11, wherein the pharmaceutical composition has a pH of 5.0-6.5, preferably 5.5-6.5, and more preferably about 6.0-6.3.

13. The pharmaceutical composition according to any one of claims 1-12, wherein the pharmaceutical composition comprises the following components:
(a) 100-150 mg/mL anti-TSLP antibody; (b) 0.1-0.8 mg/mL polysorbate; (c) 10-30 mM histidine salt buffer; and (d) 50-70 mg/mL sugar; wherein the pharmaceutical composition has a pH of 5.5-6.5; or
the pharmaceutical composition comprises the following components:
(a) 100-150 mg/mL anti-TSLP antibody; (b) 0.1-0.8 mg/mL polysorbate; (c) 10-30 mM histidine salt buffer; and (d) 30-70 mg/mL sugar and 10-30 mM amino acid; wherein the pharmaceutical composition has a pH of 5.5-6.5; or
the pharmaceutical composition comprises the following components:
(a) 100-150 mg/mL anti-TSLP antibody; (b) 0.1-0.8 mg/mL polysorbate; (c) 10-30 mM histidine salt buffer; and (d) 30-70 mg/mL sugar and 0.37-10 mM EDTA; wherein the pharmaceutical composition has a pH of 5.5-6.5; or
the pharmaceutical composition comprises the following components:
(a) 100-150 mg/mL anti-TSLP antibody; (b) 0.1-0.8 mg/mL polysorbate; (c) 10-30 mM histidine salt buffer; and (d) 30-70 mg/mL sugar, 10-30 mM amino acid, and 0.37-10 mM EDTA; wherein the pharmaceutical composition has a pH of 5.5-6.5;
wherein the sugar is selected from the group consisting of one or more of trehalose, sucrose, sorbitol, and mannitol; the amino acid is selected from the group consisting of one or more of histidine, tryptophan, and methionine; and the polysorbate is selected from the group consisting of polysorbate 80 and polysorbate 20;
preferably, the pharmaceutical composition comprises the following components:
(a) about 100 mg/mL anti-TSLP antibody; (b) about 0.8 mg/mL polysorbate 80; (c) 20 mM histidine-acetate buffer; and (d) 70 mg/mL sucrose, wherein the pharmaceutical composition has a pH of 6.0-6.3.

14. A pharmaceutical composition, wherein the pharmaceutical composition comprises the following components:
(a) 100-150 mg/mL anti-TSLP antibody, wherein the anti-TSLP antibody comprises a heavy chain set forth in SEQ ID NO: 139 and a light chain set forth in SEQ ID NO: 140; (b) 0.1-0.8 mg/mL polysorbate 20 or 80; (c) 10-30 mM histidine-acetate buffer; and (d) 50-70 mg/mL trehalose, sucrose, sorbitol or mannitol; wherein the pharmaceutical composition has a pH of 5.5-6.5;
preferably, the pharmaceutical composition comprises the following components:
(a) about 100 mg/mL anti-TSLP antibody, wherein the anti-TSLP antibody comprises a heavy chain set forth in SEQ ID NO: 139 and a light chain set forth in SEQ ID NO: 140; (b) about 0.8 mg/mL polysorbate 80; (c) about 20 mM histidine-acetate buffer; and (d) about 70 mg/mL sucrose; wherein the pharmaceutical composition has a pH of about 6.0-6.3.

15. A pharmaceutical composition, wherein the pharmaceutical composition comprises the following components:
(a) 100-150 mg/mL anti-TSLP antibody, wherein the anti-TSLP antibody comprises a heavy chain set forth in SEQ ID NO: 139 and a light chain set forth in SEQ ID NO: 140;
(b) 0.1-0.8 mg/mL polysorbate;
(c) 10-30 mM histidine-acetate buffer; and
(d) 30-70 mg/mL sugar and 10-30 mM amino acid; or
the pharmaceutical composition comprises:
(a) 100-150 mg/mL anti-TSLP antibody, wherein the anti-TSLP antibody comprises a heavy chain set forth in SEQ ID NO: 139 and a light chain set forth in SEQ ID NO: 140;
(b) 0.1-0.8 mg/mL polysorbate;
(c) 10-30 mM histidine-acetate buffer; and
(d) 30-70 mg/mL sugar and 0.37-10 mM EDTA; or
the pharmaceutical composition comprises:
(a) 100-150 mg/mL anti-TSLP antibody, wherein the anti-TSLP antibody comprises a heavy chain set forth in SEQ ID NO: 139 and a light chain set forth in SEQ ID NO: 140;
(b) 0.1-0.8 mg/mL polysorbate;
(c) 10-30 mM histidine-acetate buffer; and
(d) 30-70 mg/mL sugar, 0.37-10 mM EDTA, and 10-30 mM amino acid;
wherein:
the sugar is selected from the group consisting of one or more of trehalose, sucrose, sorbitol, and mannitol;
the amino acid is selected from the group consisting of one or more of histidine, tryptophan, and methionine;
the polysorbate is selected from the group consisting of polysorbate 20 and polysorbate 80; and
the pharmaceutical composition has a pH of 5.5-6.5; preferably about 6.0-6.3.

16. A lyophilized formulation comprising an anti-TSLP antibody, wherein the lyophilized formulation is obtained by lyophilizing the pharmaceutical composition according to any one of claims 1-15.

17. A method for preparing the pharmaceutical composition according to any one of claims 1-15, wherein the method comprises the step of buffer-exchanging an anti-TSLP antibody stock solution.

18. An article of manufacture, wherein the article of manufacture comprises a container comprising the pharmaceutical composition according to any one of claims 1-15 or the lyophilized formulation according to claim 16.

19. A method for treating a disease or disorder, wherein the method comprises administering to a subject a therapeutically effective amount of the pharmaceutical composition according to any one of claims 1-15, or the lyophilized formulation according to claim 16, wherein the disease or disorder is selected from the group consisting of an allergic disease, cancer, and an immune disease;
wherein the allergic disease is selected from the group consisting of: asthma, idiopathic pulmonary fibrosis, atopic dermatitis, allergic conjunctivitis, allergic rhinitis, allergic sinusitis, urticaria, Netherton syndrome, eosinophilic esophagitis, food allergy, allergic diarrhea, eosinophilic gastroenteritis, allergic bronchopulmonary aspergillosis, allergic fungal sinusitis, and chronic pruritus;
the cancer is selected from the group consisting of: breast cancer, colon cancer, lung cancer, ovarian cancer, and prostate cancer; and
the immune disease is selected from the group consisting of: rheumatoid arthritis, chronic obstructive pulmonary disease, systemic sclerosis, multiple sclerosis, keloid, ulcerative colitis, nasal polyposis, chronic eosinophilic pneumonia, eosinophilic bronchitis, celiac disease, Churg-Strauss syndrome, eosinophilic myalgia syndrome, hypereosinophilic syndrome, eosinophilic granulomatosis with polyangiitis, inflammatory bowel disease, scleroderma, interstitial lung disease, fibrosis induced by chronic hepatitis B or C, radiation-induced fibrosis, and fibrosis induced by wound healing;
preferably, the disease or disorder is related to TSLP.
